# EUROPEAN PATENT SPECIFICATION

(11) **EP 2 104 736 B1**
(45) Date of publication and mention of the grant of the patent: **23.11.2011**
(21) Application number: 07871691.7
(22) Date of filing: 10.12.2007
(51) Int. Cl.: C12N 15/113, A61K 31/713

(54) **MIR-126 REGULATED GENES AND PATHWAYS AS TARGETS FOR THERAPEUTIC INTERVENTION**
MIR-126-REGULIERTE GENE UND PFADE ALS ZIELE FÜR THERAPEUTISCHE INTERVENTIONEN
GÈNES ET VOIES RÉGULÉS PAR MIR-126 COMME CIBLES D'INTERVENTION THÉRAPEUTIQUE

(30) Priority: 08.12.2006 US 869295 P; 22.05.2007 US 939563 P
(43) Date of publication of application: 30.09.2009
(73) Proprietor: Asuragen, INC., Austin, TX 78744 (US)
(72) Inventor: BADER, Andreas G., Austin, TX 78744 (US); BYROM, Mike, Austin, TX 78744 (US); JOHNSON, Charles D., Austin, TX 78744 (US); BROWN, David, Austin, TX 78744 (US)
(74) Representative: Sonn & Partner Patentanwälte
(86) International application number: PCT/US2007/087033
(87) International publication number: WO 2008/073921

(56) References cited:
- WO-A-2004/076622
- WO-A-2006/137941
- GARZON RAMIRO ET AL: "JicroRNA fingerprints during human megakaryocytopoiesis" PROCEEDINGS OF THE NATIONAL ACADEMY OF SCIENCES OF USA, NATIONAL ACADEMY OF SCIENCE, WASHINGTON, DC, vol. 103, no. 13, 28 March 2006 (2006-03-28), pages 5078-5083, XP002465978 ISSN: 0027-8424

## Description

### BACKGROUND OF THE INVENTION

### I. FIELD OF THE INVENTION

The present invention relates to the fields of molecular biology and medicine. More specifically, the invention relates to methods and compositions for the treatment of diseases or conditions that are affected by miR-126 microRNAs, microRNA expression, and genes and cellular pathways directly and indirectly modulated by such.

### II. BACKGROUND

In 2001, several groups used a cloning method to isolate and identify a large group of "microRNAs" (miRNAs) from *C*. *elegans, Drosophila,* and humans (Lagos-Quintana *et al.,* 2001; Lau *et al.,* 2001; Lee and Ambros, 2001). Several hundreds of miRNAs have been identified in plants and animals-including humans-which do not appear to have endogenous siRNAs. Thus, while similar to siRNAs, miRNAs are distinct.

miRNAs thus far observed have been approximately 21-22 nucleotides in length, and they arise from longer precursors, which are transcribed from non-protein-encoding genes (Carrington and Ambros, 2003). The precursors form structures that fold back on themselves in self-complementary regions; they are then processed by the nuclease Dicer (in animals) or DCL1 (in plants) to generate the short double-stranded miRNA. One of the miRNA strands is incorporated into a complex of proteins and miRNA called the RNA-induced silencing complex (RISC). The miRNA guides the RISC complex to a target mRNA, which is then cleaved or translationally silenced, depending on the degree of sequence complementarity of the miRNA to its target mRNA. Currently, it is believed that perfect or nearly perfect complementarity leads to mRNA degradation, as is most commonly observed in plants. In contrast, imperfect base pairing, as is primarily found in animals, leads to translational silencing. However, recent data suggest additional complexity (Bagga *et al.,* 2005; Lim *et al.,* 2005), and mechanisms of gene silencing by miRNAs remain under intense study.

Recent studies have shown that changes in the expression levels of numerous miRNAs are associated with various cancers (reviewed in Esquela-Kerscher and Slack, 2006; Calin and Croce, 2006). miRNAs have also been implicated in regulating cell growth and cell and tissue differentiation - cellular processes that are associated with the development of cancer.

The inventors previously demonstrated that hsa-miR-126 is involved with the regulation of numerous cell activities that represent intervention points for cancer therapy and for therapy of other diseases and disorders (U.S. Patent Applications serial number 11/141,707 filed May 31, 2005 and serial number 11/273,640 filed November 14, 2005). Hsa-miR-126 was found to be expressed at lower levels in colon, breast, thyroid, bladder, and lung tumor samples when compared with expression in normal adjacent tissues from the same patients. In contrast, the inventors found that hsa-miR-126 and hsa-miR-126* are expressed at higher levels in white blood cells from patients with acute myelogenous leukemia (AML) than in white blood cells from normal patients. Hsa-miR-126 affects the proliferation of numerous cell types, decreasing proliferation of human breast epithelial cells (MCF12A) and carcinoma cells (BT549), normal skin fibroblasts (TE353SK), prostate cancer cells (22Rv1), and lung cancer cells (A549, CRL-5826, HTB-57) and increasing the proliferation of human leukemia cells (Jurkat) and normal human T cells. Hsa-miR-126 also affects the percentage of HeLa cells in the G1 or G2/M phases of the cell cycle and affects the percentage of cells in apoptosis (programmed cell death) for a number of different cell types. Interestingly, when administered to lung cancer (A549, HTB-57) or cervical cancer (HeLa) cells prior to administration of the therapeutic compound (e.g., etoposide) hsa-miR-126 increased the capacity of the therapeutic compound to induce death in the cancer cells. In addition to these cancer-related effects, hsa-miR-126 was found to be expressed at higher levels in brain tissue from Alzheimer's patients than in corresponding tissues normal patients, but was found to be expressed at lower levels in brain tissues from multiple sclerosis patients. Also, hsa-miR-126 was expressed at lower levels in tissues from hypertrophic hearts (Gq-coupled receptor signaling pathway knockouts in mice). Hsa-miR-126* was found to be expressed at greater than two-fold lower levels in intestinal samples from Crohn's disease patients than in corresponding samples from normal patients. More recently, others have observed miR-126 to be down-regulated during megakaryocytopoiesis (cellular differentiation in the production of platelets) (Garzon *et al.,* 2006)

Bioinformatics analyses suggest that any given miRNA may bind to and alter the expression of up to several hundred different genes. In addition, a single gene may be regulated by several miRNAs. Thus, each miRNA may regulate a complex interaction among genes, gene pathways, and gene networks. Mis-regulation or alteration of these regulatory pathways and networks, involving miRNAs, are likely to contribute to the development of disorders and diseases such as cancer. Although bioinformatics tools are helpful in predicting miRNA binding targets, all have limitations. Because of the imperfect complementarity with their target binding sites, it is difficult to accurately predict the mRNA targets of miRNAs with bioinformatics tools alone. Furthermore, the complicated interactive regulatory networks among miRNAs and target genes make it difficult to accurately predict which genes will actually be mis-regulated in response to a given miRNA.

Correcting gene expression errors by manipulating miRNA expression or by repairing miRNA mis-regulation represent promising methods to repair genetic disorders and cure diseases like cancer. A current, disabling limitation of this approach is that, as mentioned above, the details of the regulatory pathways and networks that are affected by any given miRNA, including hsa-miR-126, remain largely unknown. This represents a significant limitation for treatment of cancers in which miR-126 may play a role. A need exists to identify the genes, genetic pathways, and genetic networks that are regulated by or that may regulate hsa-miR-126 expression.

Garzon et al. (PNAS 103 (13), 2006: 5078-5083) disclose upregulation of certain miRNAs in leukemic cell lines.

WO 2004/076622 and WO 2006/137941 disclose various miRNA molecules to modulate gene expression.

### SUMMARY OF THE INVENTION

The present invention relates to an isolated nucleic acid comprising a miR-126 nucleic acid sequence for use in combination with an isolated nucleic acid comprising a miR-34a, miR-124a, or miR-147 nucleic acid sequence in the treatment of cancer. Further aspects and embodiments of the invention are defined in the claims.

The present invention provides additional compositions and method by identifying genes that are direct targets for miR-126 regulation or that are indirect or downstream targets of regulation following the miR-126 -mediated modification of another gene(s) expression. Furthermore, the invention describes gene, disease, and/or physiologic pathways and networks that are influenced by miR-126 and its family members. Compositions of the invention are contemplated for administration to a subject having, suspected of having, or at risk of developing a metabolic, an immunologic, an infectious, a cardiovascular, a digestive, an endocrine, an ocular, a genitourinary, a blood, a musculoskeletal, a nervous system, a congenital, a respiratory, a skin, or a cancerous disease or condition.

A subject or patient may be selected for treatment based on expression and/or aberrant expression of one or more miRNA or mRNA. In a further aspect, a subject or patient may be selected for treatment based on aberrations in one or more biologic or physiologic pathway(s), including aberrant expression of one or more gene associated with a pathway, or the aberrant expression of one or more protein encoded by one or more gene associated with a pathway. In still a further aspect, a subject or patient may be selected based on aberrations in miRNA expression, or biologic and/or physiologic pathway(s). A subject may be assessed for sensitivity, resistance, and/or efficacy of a therapy or treatment regime based on the evaluation and/or analysis of miRNA or mRNA expression or lack thereof. A subject may be evaluated for amenability to certain therapy prior to, during, or after administration of one or therapy to a subject or patient. Typically, evaluation or assessment may be done by analysis of miRNA and/or mRNA, as well as combination of other assessment methods that include but are not limited to histology, immunohistochemistry, blood work, etc.

In some embodiments of the disclosure, an infectious disease or condition includes a bacterial, viral, parasite, or fungal infection. Many of these genes and pathways are associated with various cancers and other diseases. Cancerous conditions include, but are not limited to anaplastic large cell lymphoma, B-cell lymphoma, chronic lymphoblastic leukemia, multiple myeloma, testicular tumor, astrocytoma, acute myelogenous leukemia, breast carcinoma, bladder carcinoma, cervical carcinoma, colorectal carcinoma, endometrial carcinoma, esophageal squamous cell carcinoma, glioma, glioblastoma, gastric carcinoma, hepatocellular carcinoma, Hodgkin lymphoma, leukemia, lipoma, melanoma, mantle cell lymphoma, myxofibrosarcoma, multiple myeloma, neuroblastoma, non-Hodgkin lymphoma, lung carcinoma, non-small cell lung carcinoma, ovarian carcinoma, esophageal carcinoma, osteosarcoma, pancreatic carcinoma, prostate carcinoma, squamous cell carcinoma of the head and neck, thyroid carcinoma, urothelial carcinoma wherein the modulation of one or more gene is sufficient for a therapeutic response. Typically a cancerous condition is an aberrant hyperproliferative condition associated with the uncontrolled growth or inability to undergo cell death, including apoptosis.

The present invention provides methods and compositions for identifying genes that are direct targets for miR-126 regulation or that are downstream targets of regulation following the miR-126-mediated modification of upstream gene expression. Furthermore, the invention describes gene pathways and networks that are influenced by miR-126 expression in biological samples. Many of these genes and pathways are associated with various cancers and other diseases. The altered expression or function of miR-126 in cells would lead to changes in the expression of these key genes and contribute to the development of disease or other conditions. Introducing miR-126 (for diseases where the miRNA is down-regulated) or a miR-126 inhibitor (for diseases where the miRNA is up-regulated) into disease cells or tissues or subjects would result in a therapeutic response. The identities of key genes that are regulated directly or indirectly by miR-126 and the disease with which they are associated are provided herein. It is contemplated that a cell may be an epithelial, stromal, or mucosal cell. The cell can be, but is not limited to brain, a neuronal, a blood, an esophageal, a lung, a cardiovascular, a liver, a breast, a bone, a thyroid, a glandular, an adrenal, a pancreatic, a stomach, a intestinal, a kidney, a bladder, a prostate, a uterus, an ovarian, a testicular, a splenic, a skin, a smooth muscle, a cardiac muscle, or a striated muscle cell. In certain aspects, the cell, tissue, or target may not be defective in miRNA expression yet may still respond therapeutically to expression or over expression of a miRNA. miR-126 could be used as a therapeutic target for any of these diseases. In certain embodiments miR-126 can be used to modulate the activity of miR-126 in a subject, organ, tissue, or cell.

A cell, tissue, or subject may be a cancer cell, a cancerous tissue, harbor cancerous tissue, or be a subject or patient diagnosed or at risk of developing a disease or condition. It is contemplated that a cancer cell is a neuronal, glial, lung, liver, brain, breast, bladder, blood, leukemic, colon, endometrial, stomach, skin, ovarian, fat, bone, cervical, esophageal, pancreatic, prostate, kidney, testicular or thyroid cell. In still a further aspect cancer includes, but is not limited to anaplastic large cell lymphoma, B-cell lymphoma, chronic lymphoblastic leukemia, multiple myeloma, testicular tumor, astrocytoma, acute myelogenous leukemia, breast carcinoma, bladder carcinoma, cervical carcinoma, colorectal carcinoma, endometrial carcinoma, esophageal squamous cell carcinoma, glioma, glioblastoma, gastric carcinoma, hepatocellular carcinoma, Hodgkin lymphoma, leukemia, lipoma, melanoma, mantle cell lymphoma, myxofibrosarcoma, multiple myeloma, neuroblastoma, non-Hodgkin lymphoma, lung carcinoma, non-small cell lung carcinoma, ovarian carcinoma, esophageal carcinoma, osteosarcoma, pancreatic carcinoma, prostate carcinoma, squamous cell carcinoma of the head and neck, thyroid carcinoma, urothelial carcinoma.

Embodiments of the invention include methods of modulating gene expression, or biologic or physiologic pathways in a cell, a tissue, or a subject comprising administering to the cell, tissue, or subject an amount of an isolated nucleic acid or mimetic thereof comprising a miR-126 nucleic acid, mimetic, or inhibitor sequence in an amount sufficient to modulate the expression of a gene positively or negatively modulated by a miR-126 miRNA. A "miR-126 nucleic acid sequence" or "miR-126 inhibitor" includes the full length precursor of miR-126, or complement thereof or processed (i.e., mature) sequence of miR-126 and related sequences set forth herein, as well as 5, 6, 7, 8, 9, 10, 11, 12, 13, 14, 15, 16, 17, 18, 19, 20, 21, 22, 23, 24, 25, 26, 27, 28, 29 or more nucleotides of a precursor miRNA or its processed sequence, or complement thereof, including all ranges and integers there between. In certain embodiments, the miR-126 nucleic acid sequence or miR-126 inhibitor contains the full-length processed miRNA sequence or complement thereof and is referred to as the "miR-126 full-length processed nucleic acid sequence" or "miR-126 full-length processed inhibitor sequence." In still further aspects, the miR-126 nucleic acid comprises at least one 5, 6, 7, 8, 9, 10, 11, 12, 13, 14, 15, 16, 17, 18, 19, 20, 21, 22, 23, 24, 25, 50 nucleotide (including all ranges and integers there between) segment or complementary segment of a miR-126 that is at least 75, 80, 85, 90, 95, 98, 99 or 100% identical to SEQ ID NO: to SEQ ID NO:24. The general term miR-126 includes all members of the miR-126 family that share at least part of a mature miR-126 sequence. Mature miR-126 sequences include hsa-miR-126 UCGUACCGUGAGUAAUAAUGC (MIMAT0000445, SEQ ID NO:1); hsa-miR-126* CAUUAUUACUUUUGGUACGCG (MIMAT0000444, SEQ ID NO:2); bta-miR-126 CGUACCGUGAGUAAUAAUGCG (MIMAT0003540, SEQ ID NO:3); dre-miR-126 UCGUACCGUGAGUAAUAAUGC (MIMAT0001823, SEQ ID NO:4); dre-miR-126* CAUUAUUACUUUUGGUACGCG (MIMAT0003157, SEQ ID NO:5); fru-miR-126 UCGUACCGUGAGUAAUAAUGC (MIMAT0002957, SEQ ID NO:6); mmu-miR-126-5p CAUUAUUACUUUUGGUACGCG (MIMAT0000137, SEQ ID NO:7); mmu-miR-126-3p UCGUACCGUGAGUAAUAAUGC (MIMAT0000138, SEQ ID NO:8); gga-miR-126 UCGUACCGUGAGUAAUAAUGCGC (MIMAT0001169, SEQ ID NO:9); gga-miR-126* CAUUAUUACUUUUGGUACGCG (MIMAT0003723, SEQ ID NO:10); mo-miR-126 UCGUACCGUGAGUAAUAAUGC (MIMAT0000832, SEQ ID NO:11); mo-miR-126* CAUUAUUACUUUUGGUACGCG (MIMAT0000831, SEQ ID NO:12); tni-miR-126 UCGUACCGUGAGUAAUAAUGC (MIMAT0002958, SEQ ID NO:13); xtr-miR-126 UCGUACCGUGAGUAAUAAUGC (MIMAT0003588, SEQ ID NO:14); and/or xtr-miR-126* CAUUAUUACUUUUGGUACGCG (MIMAT0003587, SEQ ID NO:15) or a complement thereof. In certain aspects, a subset of these miRNAs will be used that include some but not all of the listed miR-126 family members. In one aspect, miR-126 sequences have a consensus sequence of YAYYRUKASUWWURRUR (SEQ ID NO:25). In certain aspects, a subset of these miRNAs will be used that include some but not all of the listed miR-126 family members. In certain embodiments the mature sequences of miR-126 comprises all or part of hsa-miR-126 (MIMAT0000445, SEQ ID NO:1), or hsa-miR-126* (MIMAT0000444, SEQ ID NO:2).

A "miR-126 nucleic acid sequence" includes all or a segment of the full length precursor of miR-126 family members. Stem-loop sequences of miR-126 family members include hsa-mir-126 CGCUGGCGACGGGACAUUAUUACUUUUGGUACGCGCUGUGACACUUCAAACUC GUACCGUGAGUAAUAAUGCGCCGUCCACGGCA (MI0000471, SEQ ID NO: 16); bta-mir-126 UGACGGGACAUUAUUACUUUUGGUACGCGCUGUGACACUUCAAACUCGUACCG UGAGUAAUAAUGCGCUGUCA (MI0004754, SEQ ID NO:17); dre-mir-126 GAGCCAUUUUAACUGCUUCACAGUCCAUUAUUACUUUUGGUACGCGCUAGGCC AGACUCAAACUCGUACCGUGAGUAAUAAUGCACUGUGGCAGUGGGUUU(M1000 1979, SEQ ID NO: 18); fru-mir-126 CGGCCCAUUAUUACUUUUGGUACGCGCUAUGCCACUCUCAACUCGUACCGUGA GUAAUAAUGC(MI0003273, SEQ ID NO:19); gga-mir-126 GCUGGUGACGGCCCAUUAUUACUUUUGGUACGCGCUGUGACACUUCAAACUCG UACCGUGAGUAAUAAUGCGCUGUGGUCAGCA(MI0001244, SEQ ID NO:20); mmu-mir-126 UGACAGCACAUUAUUACUUUUGGUACGCGCUGUGACACUUCAAACUCGUACCG UGAGUAAUAAUGCGCGGUCA (MI0000153, SEQ ID NO:21); mo-mir-126 UGACAGCACAUUAUUACUUUUGGUACGCGCUGUGACACUUCAAACUCGUACCG UGAGUAAUAAUGCGUGGUCA (MI0000898, SEQ ID NO:22); tni-mir-126 CGGCCCAUUAUUACUUUUGGUACGCGCUAUGCCACUCUCAACUCGUACCGUGA GUAAUAAUGC (MI0003274, SEQ ID NO:23); and/or xtr-mir-126 GGCUGUGCAUUAUUACUUUUGGUACGCGCUGUGUCACAUCAAACUCGUACCGU GAGUAAUAAUGCGCAG (MI0004827, SEQ ID NO:24) or a complement thereof.

In certain aspects, a miR-126 nucleic acid, or a segment or a mimetic thereof, will comprise 5, 6, 7, 8, 9, 10, 11, 12, 13, 14, 15, 16, 17, 18, 19, 20, 21, 22, 23, 24, 25, 26, 27, 28, 29 or more nucleotides of the precursor miRNA or its processed sequence, including all ranges and integers there between. In certain embodiments, the miR-126 nucleic acid sequence contains the full-length processed miRNA sequence and is referred to as the "miR-126 full-length processed nucleic acid sequence." In still further aspects, a miR-126 comprises at least one 5, 6, 7, 8, 9, 10, 11, 12, 13, 14, 15, 16, 17, 18, 19, 20, 21, 22, 23, 24, 25, 50 nucleotide (including all ranges and integers there between) segment of miR-126 that is at least 75, 80, 85, 90, 95, 98, 99 or 100% identical to SEQ ID NOs provided herein.

In specific embodiments, a miR-126 or miR-126 inhibitor containing nucleic acid is a hsa-miR-126 or hsa-miR-126 inhibitor, or a hsa-miR-126* or hsa-miR-126* inhibitor, or a variation thereof. In a further aspect, a miR-126 nucleic acid or miR-126 inhibitor can be administered with 1, 2, 3, 4, 5, 6, 7, 8, 9, 10 or more miRNAs or miRNA inhibitors. miRNAs or their complements can be administered concurrently, sequentially, or in an ordered progression. The present invention relates to the combination of miR-126 with a miR-34a, miR-124a or miR-147 nucleic acid. It is contemplated that a miR-126 or miR-126 inhibitor can be administered in combination with one or more of let-7, miR-15, miR-16, miR-20, miR-21, miR-26a, miR-34a, miR-143, miR-147, miR-188, miR-200, miR-215, miR-216, miR-292-3p, and/or miR-331. All or combinations of miRNAs or inhibitors thereof may be administered in a single formulation. Administration may be before, during or after a second therapy.

miR-126 nucleic acids or complement thereof may also include various heterologous nucleic acid sequences, i.e., those sequences not typically found operatively coupled with miR-126 in nature, such as promoters, enhancers, and the like. The miR-126 nucleic acid is a recombinant nucleic acid, and can be a ribonucleic acid or a deoxyribonucleic acid. The recombinant nucleic acid may comprise a miR-126 or miR-126 inhibitor expression cassette, *i.e.,* a nucleic acid segment that expresses a nucleic acid when introduce into an environment containing components for nucleic acid synthesis. In a further aspect, the expression cassette is comprised in a viral vector, or plasmid DNA vector or other therapeutic nucleic acid vector or delivery vehicle, including liposomes and the like. In a particular aspect, the miR-126 nucleic acid is a synthetic nucleic acid. Moreover, nucleic acids of the invention may be fully or partially synthetic. In certain aspects, viral vectors can be administered at 1x10², 1x10³, 1x10⁴ 1x10⁵, 1x10⁶, 1x10⁷, 1x10⁸, 1x10⁹, 1x10¹⁰, 1x10¹¹, 1x10¹², 1x10¹³, 1x10¹⁴ pfu or viral particle (vp).

In a particular aspect, the miR-126 nucleic acid or miR-126 inhibitor is a synthetic nucleic acid. Moreover, nucleic acids of the invention may be fully or partially synthetic. In still further aspects, a nucleic acid of the invention or a DNA encoding such a nucleic acid of the invention can be administered at 0.001, 0.01, 0.1, 1, 10, 20, 30, 40, 50, 100, 200, 400, 600, 800, 1000, 2000, to 4000 µg or mg, including all values and ranges there between. In yet a further aspect, nucleic acids of the invention, including synthetic nucleic acid, can be administered at 0.001, 0.01, 0.1, 1, 10, 20, 30, 40, 50, 100, to 200 µg or mg per kilogram (kg) of body weight. Each of the amounts described herein may be administered over a period of time, including 0.5, 1, 2, 3, 4, 5, 6, 7, 8, 9, 10, minutes, hours, days, weeks, months or years, including all values and ranges there between.

In certain embodiments, administration of the composition(s) can be enteral or parenteral. In certain aspects, enteral administration is oral. In further aspects, parenteral administration is intralesional, intravascular, intracranial, intrapleural, intratumoral, intraperitoneal, intramuscular, intralymphatic, intraglandular, subcutaneous, topical, intrabronchial, intratracheal, intranasal, inhaled, or instilled. Compositions of the invention may be administered regionally or locally and not necessarily directly into a lesion.

In certain aspects, the gene or genes modulated comprises 1, 2, 3, 4, 5, 6, 7, 8, 9, 10, 11, 12, 13, 14, 15, 20, 25, 30, 35, 40, 45, 50, 100, 150, 200 or more genes or combinations of genes identified in Tables 1, 3, 4, and/or 5. In still further aspects, the gene or genes modulated may exclude 1, 2, 3, 4, 5, 6, 7, 8, 9, 10, 11, 12, 13, 14, 15, 20, 25, 30, 35, 40, 45, 50, 100, 150, 175 or more genes or combinations of genes identified in Tables 1, 3, 4, and/or 5. Modulation includes modulating transcription, mRNA levels, mRNA translation, and/or protein levels in a cell, tissue, or organ. In certain aspects the expression of a gene or level of a gene product, such as mRNA or encoded protein, is down-regulated or up-regulated. In a particular aspect the gene modulated comprises or is selected from (and may even exclude) 1, 2, 3, 4, 5, 6, 7, 8, 9, 10, 11, 12, 13, 14, 15, 16, 17, 18, 19, 20, 21, 22, 23, 24, 25, 26. 27, 28, or all of the genes identified in Tables 1, 3, 4, and/or 5, or any combinations thereof. In certain embodiments a gene modulated or selected to be modulated is from Table 1. In further embodiments a gene modulated or selected to be modulated is from Table 3. In still further embodiments a gene modulated or selected to be modulated is from Table 4. In yet further embodiments a gene modulated or selected to be modulated is from Table 5. Embodiments of the invention may also include obtaining or assessing a gene expression profile or miRNA profile of a target cell prior to selecting the mode of treatment, e.g., administration of a miR-126 nucleic acid, inhibitor of miR-126, or mimetics thereof. In certain aspects of the invention one or more miRNA or miRNA inhibitor may modulate a single gene. In a further aspect, one or more genes in one or more genetic, cellular, or physiologic pathways can be modulated by one or more miRNAs or complements thereof, including miR-126 nucleic acids and miR-126 inhibitors in combination with other miRNAs.

miR-126 nucleic acids may also include various heterologous nucleic acid sequences, i.e., those sequences not typically found operatively coupled with miR-126 in nature, such as promoters, enhancers, and the like. The miR-126 nucleic acid is a recombinant nucleic acid, and can be a ribonucleic acid or a deoxyribonucleic acid. The recombinant nucleic acid may comprise a miR-126 expression cassette. In a further aspect, the expression cassette is comprised in a viral, or plasmid DNA vector or other therapeutic nucleic acid vector or delivery vehicle, including liposomes and the like. In a particular aspect, the miR-126 nucleic acid is a synthetic nucleic acid. Moreover, nucleic acids of the invention may be fully or partially synthetic.

Methods of modulating a cellular pathway comprising administering to the cell an amount of an isolated nucleic acid comprising a miR-126 nucleic acid sequence in an amount sufficient to modulate the expression, function, status, or state of a cellular pathway, in particular those pathways described in Table 2 or the pathways known to include one or more genes from Table 1, 3, 4, and/or 5 are disclosed. Modulation of a cellular pathway includes, but is not limited to modulating the expression of one or more gene. Modulation of a gene can include inhibiting the function of an endogenous miRNA or providing a functional miRNA to a cell, tissue, or subject. Modulation refers to the expression levels or activities of a gene or its related gene product or protein, e.g., the mRNA levels may be modulated or the translation of an mRNA may be modulated, etc. Modulation may increase or up regulate a gene or gene product or it may decrease or down regulate a gene or gene product.

Still a further embodiment includes methods of treating a patient with a pathological condition comprising one or more of step (a) administering to the patient an amount of an isolated nucleic acid comprising a miR-126 nucleic acid sequence in an amount sufficient to modulate the expression of a cellular pathway; and (b) administering a second therapy, wherein the modulation of the cellular pathway sensitizes the patient to the second therapy. A cellular pathway may include, but is not limited to one or more pathway described in Table 2 below or a pathway that is known to include one or more genes of Tables 1, 3, 4, and/or 5. A second therapy can include administration of a second miRNA or therapeutic nucleic acid, or may include various standard therapies, such as chemotherapy, radiation therapy, drug therapy, immunotherapy, and the like. Embodiments of the invention may also include the determination or assessment of a gene expression profile for the selection of an appropriate therapy.

Methods of treating a subject with a pathological condition comprising one or more of the steps of (a) determining an expression profile of one or more genes selected from Table 1, 3, 4, and/or 5; (b) assessing the sensitivity of the subject to therapy based on the expression profile; (c) selecting a therapy based on the assessed sensitivity; and (d) treating the subject using selected therapy are disclosed. Typically, the pathological condition will have as a component, indicator, or result the mis-regulation of one or more gene of Table 1, 3, 4, and/or 5.

Further embodiments include the identification and assessment of an expression profile indicative of miR-126 status in a cell or tissue comprising expression assessment of one or more gene from Table 1, 3, 4, and/or 5, or any combination thereof.

The term "miRNA" is used according to its ordinary and plain meaning and refers to a microRNA molecule found in eukaryotes that is involved in RNA-based gene regulation. See, e.g., Carrington *et al.,* 2003. The term can be used to refer to the single-stranded RNA molecule processed from a precursor or in certain instances the precursor itself.

In some embodiments, it may be useful to know whether a cell expresses a particular miRNA endogenously or whether such expression is affected under particular conditions or when it is in a particular disease state. Thus, in some embodiments of the invention, methods include assaying a cell or a sample containing a cell for the presence of one or more marker gene or mRNA or other analyte indicative of the expression level of a gene of interest. Consequently, in some embodiments, methods include a step of generating an RNA profile for a sample. The term "RNA profile" or "gene expression profile" refers to a set of data regarding the expression pattern for one or more gene or genetic marker in the sample (*e.g.*, a plurality of nucleic acid probes that identify one or more markers from Tables 1, 3, 4, and/or 5); it is contemplated that the nucleic acid profile can be obtained using a set of RNAs, using for example nucleic acid amplification or hybridization techniques well know to one of ordinary skill in the art. The difference in the expression profile in the sample from the patient and a reference expression profile, such as an expression profile from a normal or non-pathologic sample, is indicative of a pathologic, disease, or cancerous condition. A nucleic acid or probe set comprising or identifying a segment of a corresponding mRNA can include all or part of 1, 2, 3, 4, 5, 6, 7, 8, 9, 10, 11, 12 ,13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 100, 200, 500, or more nucleotides, including any integer or range derivable there between, of a gene, or genetic marker, or a nucleic acid, mRNA or a probe representative thereof that is listed in Tables 1, 3, 4, and/or 5 or identified by the methods described herein.

Compositions and methods for assessing, prognosing, or treating a pathological condition in a patient are disclosed comprising measuring or determining an expression profile of one or more marker(s) in a sample from the patient, wherein a difference in the expression profile in the sample from the patient and an expression profile of a normal sample or reference expression profile is indicative of pathological condition and particularly cancer (*e.g.*, In certain aspects of the invention, the cellular pathway, gene, or genetic marker is or is representative of one or more pathway or marker described in Table 1, 3, 4, and/or 5, including any combination thereof.

Aspects of the disclosure include diagnosing, assessing, or treating a pathologic condition or preventing a pathologic condition from manifesting. For example, the methods can be used to screen for a pathological condition; assess prognosis of a pathological condition; stage a pathological condition; assess response of a pathological condition to therapy; or to modulate the expression of a gene, genes, or related pathway as a first therapy or to render a subject sensitive or more responsive to a second therapy. In particular aspects, assessing the pathological condition of the patient can be assessing prognosis of the patient. Prognosis may include, but is not limited to an estimation of the time or expected time of survival, assessment of response to a therapy, and the like. In certain aspects, the altered expression of one or more gene or marker is prognostic for a patient having a pathologic condition, wherein the marker is one or more of Table 1, 3, 4, and/or 5, including any combination thereof.

**Table 1. Genes with increased (positive values) or decreased (negative values) expression following transfection of human cancer cells with pre-miR hsa-miR-126.**

| **Gene Symbol** | **RefSeq Transcript ID** (Pruitt *et al.,* 2005) | **Δ log₂** |
|---|---|---|
| - | XM_371853 | 0.932645493 |
| ABAT | NM_000663 /// NM_020686 | 0.714406175 |
| | NM_004996 /// NM_019862 /// NM_019898 /// | |
| ABCC1 | NM_019899 /// NM_019900 /// NM_019901 | -1.370726927 |
| ABHD3 | NM_138340 | -1.850325878 |
| ACSM3 | NM_005622 /// NM _02000 | 1.02825646 |
| ACTR2 | NM_001005386 /// NM_005722 | 0.8699003 |
| ADAM9 | NM_001005845 /// NM_003816 | -1.175130131 |
| ADK | NM_001123 /// NM_006721 | -1.160696557 |
| AES | NM_001130 /// NM_198969 /// NM_198970 | -1.20994927 |
| AHNAK | NM_001620 /// NM_024060 | -1.362496554 |
| ALDH6A1 | NM_005589 | 0.920729494 |
| | NM_001145 /// NM_002937 /// | |
| ANG /// RNASE4 | NM_194430 /// NM_194431 | 0.837651946 |
| ANKRD46 | NM_198401 | 1.489357842 |
| ANPEP | NM_001150 | 1.015859419 |
| ANTXR1 | NM_018153 /// NM_032208 /// NM_053034 | -1.152388862 |
| APOH | NM_000042 | 1.464769764 |
| APP | NM_000484 /// NM_201413 /// NM_201414 | 0.79513041 |
| AQP3 | NM_004925 | 1.683494303 |
| ARFRP1 | NM_003224 | -0.707888983 |
| ARG2 | NM 001172 | 0.802643889 |
| ARHGAP11A | NM_014783 /// NM_199357 | -0.859064851 |
| ARHGDIA | NM_004309 | -1.479298425 |
| ARID5B | NM_032199 | 0.81758645 |
| ARL2BP | NM_012106 | 0.930117945 |
| ARTS-1 | NM_016442 | 0.885978383 |
| ASNS | NM_001673 /// NM_133436 /// NM_183356 | -0.70922125 |
| ATP6V0E | NM_003945 | 1.492546023 |
| ATP6V1D | NM_015994 | -1.206032987 |
| B4GALT4 | NM_003778 /// NM_212543 | -2.009786779 |
| B4GALT6 | NM_004775 | -0.814480886 |
| BCL2A1 | NM_004049 | -0.865661051 |
| BCL6 | NM_001706 /// NM_138931 | 1.022676812 |
| BF | NM_001710 | 1.374561737 |
| BHLHB2 | NM_003670 | -0.732093722 |
| BTG2 | NM_006763 | 0.71613205 |
| C19orf28 | NM_174983 | -1.583035095 |
| C1orf121 | NM_016076 | -1.018547016 |
| C1R | NM 001733 | 1.371857223 |
| C2orf26 | NM_023016 | -1.688347985 |
| C2orf33 | NM_020194 | -1.066450989 |
| C3 | NM_000064 | 1.750839431 |
| C5orf15 | NM_020199 | -1.09946192 |
| C6orf75 | NM_001031712 /// NM_021820 | -1.159367109 |
| C8orf1 | NM_004337 | -1.200945344 |
| C8orf32 | NM_018024 | -1.162127286 |
| CA11 | NM_001217 | 0.883203637 |
| CCND1 | NM_053056 | -0.787125621 |
| CCNG1 | NM_004060 /// NM_199246 | 0.720877561 |
| CD9 | NM_001769 | -1.226285741 |
| CDC37L1 | NM_017913 | -1.197737388 |
| CDH17 | NM_004063 | 1.248005394 |
| CDH4 | NM_001794 | -1.072466062 |
| CEACAM1 | NM_001024912 /// NM_001712 | 1.002240316 |
| CEBPD | NM_005195 | 0.919605248 |
| CFH /// CFHL1 | NM_000186 /// NM_001014975 /// NM_002113 | 0.727009767 |
| CGI-38 | NM_015964 /// NM_016140 | 1.151724832 |
| CGI-48 | NM_016001 | 1.409467543 |
| CHGB | NM_001819 | 2.065861363 |
| CHST11 | NM_018413 | -0.898267014 |
| CMKOR1 | NM_020311 | -0.953658982 |
| COL4A1 | NM_001845 | -1.251472248 |
| COL4A2 | NM_001846 | -1.104000878 |
| COPS7A | NM_016319 | -0.713729574 |
| CP | NM_000096 | 2.114989939 |
| CPE | NM_001873 | 0.845367555 |
| CPS1 | NM_001875 | 0.737097779 |
| CTDSP2 | NM_005730 | 1.119227888 |
| CTDSPL | NM_001008392 /// NM_005808 | 0.734756589 |
| CTGF | NM_001901 | 0.792411336 |
| CTPS | NM_001905 | -1.069899984 |
| CTSS | NM_004079 | 0.928240677 |
| CXCL5 | NM_002994 | 0.870566945 |
| CYP3A5 | NM_000777 | 0.811086872 |
| DAAM1 | NM_014992 | 0.759834375 |
| DCBLD2 | NM_080927 | -1.328719371 |
| DCUN1D1 | NM_020640 | -1.329350022 |
| DDC | NM_000790 | 1.008327023 |
| DDX3Y | NM_004660 | 0.737980013 |
| DHRS2 | NM_005794 /// NM_182908 | 1.488681085 |
| DIO2 | NM_000793 /// NM_001007023 /// NM_013989 | -1.212624259 |
| DIPA | NM_006848 | -0.805448452 |
| DKFZP586A0522 | NM_014033 | 1.053615971 |
| DLG5 | NM_004747 | -1.466726314 |
| DNAJB9 | NM_012328 | 0.959942564 |
| DPYSL3 | NM_001387 | -0.947045282 |
| DSU | NM_018000 | 0.942279047 |
| DUSP6 | NM_001946 /// NM_022652 | 0.715273666 |
| EEF1D | NM_001960 /// NM_032378 | 0.987070932 |
| EFHD2 | NM_024329 | -1.108805315 |
| | NM_005228 /// NM_201282 /// | |
| EGFR | NM_201283 /// NM_201284 | -1.332830672 |
| EHF | NM_012153 | 2.016905078 |
| EML1 | NM_001008707 /// NM_004434 | -1.062197647 |
| ENPP4 | NM_014936 | 0.806430572 |
| EPHB2 | NM_004442 /// NM_017449 | -0.876298257 |
| ERBB3 | NM_001005915 /// NM_001982 | 1.04088957 |
| EREG | NM_001432 | -0.739760712 |
| F2RL1 | NM_005242 | -0.784621872 |
| F5 | NM_000130 | 1.114984931 |
| F8A1 | NM_012151 | -0.848526764 |
| FAM46A | NM_017633 | 0.849885425 |
| | NM_000043 /// NM_152871 /// NM_152872 /// | |
| FAS | NM_152873 /// NM_152874 /// NM_152875 | 0.948587312 |
| FBXO11 | NM_012167 /// NM_018693 /// NM_025133 | 0.829505914 |
| FCMD | NM_006731 | -0.871816304 |
| FGB | NM_005141 | 1.117629067 |
| | NM_000604 /// NM_015850 /// NM_023105 /// | |
| FGFR1 | NM_023106 /// NM_023107 /// NM_023108 | -0.703118408 |
| FGFR4 | NM_002011 /// NM_022963 /// NM_213647 | 0.905869427 |
| | NM_004467 /// NM_147203 /// NM_201552 /// | |
| FGL1 | NM_201553 | 2.075521164 |
| FLJ11184 | NM_018352 | -0.926430092 |
| FLJ13910 | NM_022780 | 1.050439033 |
| FLJ20364 | NM_017785 | -1.020575851 |
| FLJ21159 | NM_024826 | -1.11995013 |
| FLJ31568 | NM_152509 | 0.731975063 |
| FLRT3 | NM_013281 /// NM_198391 | 1.339665795 |
| FMO5 | NM_001461 | 1.276684233 |
| FOSL1 | NM_005438 | -0.934827265 |
| GABRA5 | NM_000810 | -1.611906839 |
| GALNT12 | NM_024642 | -0.898090103 |
| GALNT4 | NM_003774 | 1.916082489 |
| GART | NM_000819 /// NM_175085 | -1.022001714 |
| GATM | NM_001482 | 1.126888066 |
| | NM_000161 /// NM_001024024 /// | |
| GCH1 | NM_001024070 /// NM_001024071 | 0.93888458 |
| | NM_005270 /// NM_030379 /// NM_030380 /// | |
| GLI2 | NM_030381 | -0.8678312 |
| GLT25D1 | NM_024656 | -1.17034322 |
| | NM_001033044 /// NM_001033056 /// | |
| GLUL | NM_002065 | 0.734013499 |
| GNA13 | NM_006572 | 0.739402224 |
| GPR64 | NM_005756 | -1.209041679 |
| GREM1 | NM_013372 | -1.04918052 |
| GTF2B | NM_001514 | -0.979542167 |
| GTSE1 | NM_016426 | -0.771181159 |
| H2AFY | NM_004893 /// NM_138609 /// NM_138610 | -0.841043284 |
| H3F3B | NM_005324 | -1.391335687 |
| HERC4 | NM_001017972 /// NM_015601 /// NM_022079 | -1.408789739 |
| HES1 | NM_005524 | 0.815351802 |
| HIPK3 | NM_005734 | 0.825488898 |
| HKDC1 | NM_025130 | -0.891137084 |
| HLA-DMA | NM_006120 | 1.267193674 |
| HLA-DMB | NM_002118 | 1.253032623 |
| | NM_002131 /// NM_145899 /// NM_145901 /// | |
| HMGA1 | NM_145902 /// NM_145903 /// NM_145904 | -0.870293114 |
| IER3IP1 | NM_016097 | 1.015224727 |
| IFI16 | NM_005531 | 0.701376096 |
| IFIT1 | NM_001001887 /// NM_001548 | 0.766029617 |
| IL11 | NM_000641 | -1.980329167 |
| IL6R | NM_000565 /// NM_181359 | 1.80035893 |
| IL6ST | NM_002184 /// NM_175767 | 0.7356025 |
| | NM_001014794 /// NM_001014795 /// | |
| ILK | NM_004517 | -1.193369748 |
| INHBC | NM_005538 | 0.988048988 |
| ITGAV | NM_002210 | -1.397061642 |
| | NM_000213 /// NM_001005619 /// | |
| ITGB4 | NM_001005731 | -1.083868931 |
| IVNS1ABP | NM_006469 /// NM_016389 | -1.12637182 |
| JUN | NM_002228 | -1.257461721 |
| KCNJ16 | NM_018658 /// NM_170741 /// NM_170742 | 0.863727562 |
| KCNJ2 | NM_000891 | 0.943183898 |
| KCNK5 | NM_003740 | 0.908771236 |
| KCTD9 | NM_017634 | -1.102291183 |
| KIAA0152 | NM_014730 | -1.120821727 |
| KIAA0317 | NM_014821 | -0.998442704 |
| KIAA0882 | NM_015130 | 0.706054046 |
| KLC2 | NM_022822 | -1.308006302 |
| KLF4 | NM_004235 | -1.010190659 |
| LARP6 | NM_018357 /// NM_197958 | -1.272557153 |
| LBA1 | XM_047357 | 1.070433025 |
| LCN2 | NM_005564 | 1.032051492 |
| LOC257407 | --- | 1.210506019 |
| LOC93349 | NM_138402 | 1.021232618 |
| LRP12 | NM_013437 | -2.094170389 |
| LXN | NM_020169 | 1.016851203 |
| M6PRBP1 | NM_005817 | -1.073783905 |
| MAP1B | NM_005909 /// NM_032010 | -0.705975117 |
| MAP3K1 | XM_042066 | 1.608824148 |
| MAPK6 | NM_002748 | -0.8099719 |
| MASK | NM_016542 | -1.400096504 |
| MAWBP | NM_001033083 /// NM_022129 | 1.125643858 |
| MAZ | NM_002383 | -1.133295139 |
| MCL1 | NM_021960 /// NM_182763 | 1.3936595 |
| MET | NM_000245 | -0.950795574 |
| MICAL2 | NM_014632 | -1.182303804 |
| MMP7 | NM_002423 | 1.126537362 |
| MR1 | NM_001531 | 0.701977324 |
| | NM_001001924 /// NM_001001925 /// | |
| | NM_001001927 /// NM_001001931 /// | |
| MTUS1 | NM_020749 | 0.700939263 |
| MYBL1 | XM_034274 | -1.008471905 |
| NAB1 | NM_005966 | -0.724200536 |
| NCF2 | NM_000433 | -0.870560657 |
| NEK4 | NM_003157 | -1.19705434 |
| NF1 | NM_000267 | -1.382836417 |
| | NM_000268 /// NM_016418 /// NM_181825 /// | |
| NF2 | NM_181826 /// NM_181827 /// NM_181828 | -0.828783467 |
| NID1 | NM_002508 | 0.883339703 |
| NPTX1 | NM 002522 | -1.203822239 |
| NR1H4 | NM_005123 | 1.063112995 |
| NR2F1 | NM 005654 | 0.875442672 |
| NR4A2 | NM_006186 /// NM_173171 /// NM_173172 /// NM_173173 | 1.190313852 |
| NRIP1 | NM_003489 | 0.719600704 |
| NUCKS | NM_022731 | 2.049765811 |
| OLFML3 | NM_020190 | -1.332817337 |
| OXTR | NM_000916 | -1.374096123 |
| PCAF | NM_003884 | -1.071216608 |
| PDCD4 | NM_014456 /// NM_145341 | 1.094361696 |
| PDK4 | NM_002612 | 1.686179306 |
| PDXK | NM_003681 | -1.14206778 |
| PDZK1IP1 | NM_005764 | 0.890579923 |
| PEX10 | NM_002617 /// NM_153818 | -0.717726179 |
| Pfs2 | NM_016095 | -0.767396197 |
| PGK1 | NM_000291 | 1.563367184 |
| PHACTR2 | NM_014721 | 0.879126697 |
| PHTF2 | NM_020432 | -1.334037819 |
| PLA1A | NM_015900 | 1.622927127 |
| PLA2G4A | NM_024420 | 0.889892262 |
| PLCB1 | NM_015192 /// NM_182734 | 1.421263838 |
| | NM_000445 /// NM_201378 /// NM_201379 /// | |
| PLEC1 | NM_201380 /// NM_201381 /// NM_201382 | -0.903511275 |
| PLK1 | NM_005030 | -0.729493842 |
| PLOD2 | NM_000935 /// NM 182943 | -1.204499492 |
| PMCH | NM_002674 | 0.969722931 |
| PMM1 | NM_002676 | -1.034238063 |
| PPP3CB | NM_021132 | -1.185401118 |
| PRKCA | NM_002737 | -1.082689496 |
| PRNP | NM_000311 /// NM_183079 | -0.886192661 |
| PRO 1843 | --- | 1.385935811 |
| PROS1 | NM_000313 | 1.020902494 |
| PSME3 | NM_005789 /// NM_176863 | -0.842705144 |
| PTENP1 | --- | 0.890418049 |
| PTK9 | NM_002822 /// NM_198974 | 1.027625233 |
| PTP4A1 | NM_003463 | 0.725947194 |
| PXN | NM_002859 | -0.802852162 |
| QDPR | NM_000320 | -1.047654882 |
| | NM_006775 /// NM_206853 /// | |
| QKI | NM_206854 /// NM_206855 | 0.7094405 |
| RAB11FIP2 | NM_014904 | -0.841728285 |
| RAB2 | NM_002865 | 1.458930704 |
| RAB40B | NM_006822 | 1.165879677 |
| | NM_001003789 /// NM_007081 /// | |
| RABL2B /// RABL2A | NM_007082 /// NM_013412 | -0.880465804 |
| RAFTLIN | NM_015150 | 0.975257863 |
| RARRES31 | NM_002888 /// NM 206963 | 1.522692672 |
| RARRES3 | NM_004585 | 1.399020876 |
| RASSF2 | NM_014737 /// NM_170773 /// NM_170774 | -1.355108018 |
| RBP4 | NM_006744 | 1.037368088 |
| RDX | NM_002906 | 1.15499944 |
| RGC32 | NM_014059 | 1.701069337 |
| RHEB | NM_005614 | 1.113285538 |
| RHOB | NM_004040 | -1.015026834 |
| RHOBTB1 | NM_001032380 /// NM_014836 /// NM_198225 | 1.197459377 |
| RIG | - | 0.999287543 |
| RIP | NM_001033002 /// NM_032308 | 1.29885388 |
| RNASE4 | NM_002937 /// NM_194430 /// NM_194431 | 1.351135013 |
| | NM_004290 /// NM_183398 /// NM_183399 | |
| RNF14 | /// NM_183400 /// NM_183401 | -1.292621345 |
| RPL14 | NM_001034996 /// NM_003973 | 0.793723753 |
| RPL38 | NM_000999 | 1.08360474 |
| RPS11 | NM_001015 | 1.096577404 |
| RRAGD | NM_021244 | 1.192555492 |
| SCARB2 | NM_005506 | 0.963469956 |
| SCML1 | NM_006746 | 0.948285968 |
| SDC1 | NM_001006946 /// NM_002997 | -0.972633287 |
| SDC4 | NM_002999 | -1.316418904 |
| SEC24A | XM_094581 | 0.772444817 |
| SELENBP1 | NM_003944 | 1.19286408 |
| SEPP1 | NM_005410 | 2.013889494 |
| SEPT9 | NM_006640 | -0.728160156 |
| SERPINA6 | NM_001756 | 1.031675368 |
| SERPINE1 | NM_000602 | -1.338382632 |
| SF3B4 | NM_005850 | -1.04437329 |
| SGPL1 | NM_003901 | -1.589647095 |
| SGPP1 | NM_030791 | -1.772804615 |
| SH3YL1 | NM_015677 | 1.025625373 |
| SLC26A2 | NM_000112 | -1.614518169 |
| SLC2A3 | NM_006931 | 0.746674382 |
| SLC35D1 | NM_015139 | -1.443097447 |
| SLC39A6 | NM_012319 | -2.468156693 |
| | NM_001012661 /// NM_001012662 /// | |
| | NM_001012663 | |
| SLC3A2 | /// NM_001012664 /// NM_001013251 | -1.220704927 |
| SLC4A4 | NM_003759 | -1.660738525 |
| SLC4A7 | NM_003615 | -0.906443133 |
| SLC7A11 | NM_014331 | -0.72104098 |
| SLC7A5 | NM_003486 | -1.957841113 |
| SMARCA2 | NM_003070 /// NM_139045 | 0.81189234 |
| SMTN | NM_006932 /// NM_134269 /// NM_134270 | -1.071324774 |
| SNRPD1 | NM_006938 | -0.702694811 |
| SOCS2 | NM_003877 | -0.719322364 |
| SORBS3 | NM_001018003 /// NM_005775 | -1.10626247 |
| | NM_001003790 /// NM_001003791 /// | |
| SPFH2 | NM_007175 | 0.934575358 |
| SRD5A1 | NM_001047 | -0.971316597 |
| STC1 | NM_003155 | 0.869595146 |
| STK24 | NM_001032296 /// NM_003576 | -1.02056753 |
| STX3A | NM_004177 | 0.798339266 |
| SUMO2 | NM_001005849 /// NM_006937 | 0.999648454 |
| TAGLN | NM_001001522 /// NM_003186 | -0.922062614 |
| TARDBP | NM_007375 | -1.049883834 |
| TFG | NM_001007565 /// NM_006070 | 0.998134931 |
| TFPI | NM_001032281 ///NM_006287 | 1.00261952 |
| TGFBR2 | NM_001024847 /// NM_003242 | 1.067718108 |
| TGFBR3 | NM_003243 | 1.081563312 |
| THBS1 | NM_003246 | -0.992235361 |
| TJP2 | NM_004817 /// NM_201629 | 0.830136748 |
| TLR3 | NM_003265 | 1.095939971 |
| TM4SF20 | NM_024795 | 0.931882437 |
| TncRNA | --- | 1.612862616 |
| TNFAIP6 | NM_007115 | -1.542256921 |
| TNFRSF12A | NM_016639 | -1.070070443 |
| TNFSF10 | NM_003810 | 1.066284021 |
| TNRC9 | XM_049037 | 0.814062386 |
| TNS1 | NM_022648 | 1.234553118 |
| TP73L | NM_003722 | 0.760202485 |
| TRA1 | NM_003299 | 1.98186454 |
| | NM_014788 /// NM_033219 /// | |
| TRIM14 | NM_033220 /// NM_033221 | -1.157451263 |
| TRIM22 | NM_006074 | 1.37893169 |
| TSC | NM_017899 | 1.371738309 |
| TSPAN7 | NM_004615 | 0.804886676 |
| TSPAN8 | NM_004616 | 1.265741135 |
| TTC3 | NM_001001894 /// NM_003316 | 0.707937469 |
| TUBB2 /// TUBB-PARALOG | NM_001069 /// NM_178012 | -0.903995298 |
| TXN | NM_003329 | 1.542878926 |
| UAP1 | NM_003115 | -1.613036348 |
| UBE2L6 | NM_004223 /// NM_198183 | 0.91191604 |
| UPK1B | NM_006952 | -1.042163942 |
| VDAC1 | NM_003374 | -1.180958209 |
| VDAC3 | NM_005662 | 1.062739922 |
| VIL2 | NM_003379 | 0.760424213 |
| WDR1 | NM_005112 /// NM_017491 | -0.837271564 |
| WDR41 | NM_018268 | -1.490323974 |
| WEE1 | NM_003390 | 0.889202932 |
| WNT7B | NM_058238 | -1.11406107 |
| XTP2 | NM_015172 | 0.908286656 |
| YKT6 | NM_006555 | -1.175903828 |
| YOD1 | NM_018566 | 0.86901236 |
| YTHDF3 | NM_152758 | -0.939674929 |
| ZNF467 | NM_207336 | 1.211298505 |

Methods of modulating a cellular pathway are disclosed comprising administering to the cell an amount of an isolated nucleic acid comprising a miR-126 nucleic acid sequence or a miR-126 inhibitor. A cell, tissue, or subject may be a cancer cell, a cancerous tissue or harbor cancerous tissue, or a cancer patient.

Methods of modulating a cellular pathway are disclosed comprising administering to the cell an amount of an isolated nucleic acid comprising a miR-126 nucleic acid sequence in an amount sufficient to modulate the expression, function, status, or state of a cellular pathway, in particular those pathways described in Table 2 or the pathways known to include one or more genes from Table 1, 3, 4, and/or 5. Modulation of a cellular pathway includes, but is not limited to modulating the expression of one or more gene(s). Modulation of a gene can include inhibiting the function of an endogenous miRNA or providing a functional miRNA to a cell, tissue, or subject. Modulation refers to the expression levels or activities of a gene or its related gene product (e.g., mRNA) or protein, e.g., the mRNA levels may be modulated or the translation of an mRNA may be modulated. Modulation may increase or up regulate a gene or gene product or it may decrease or down regulate a gene or gene product (e.g., protein levels or activity).

Disclosed are methods of administering an miRNA or mimic thereof, and/or treating a subject or patient having, suspected of having, or at risk of developing a pathological condition comprising one or more of step (a) administering to a patient or subject an amount of an isolated nucleic acid comprising a miR-126 nucleic acid sequence or a miR-126 inhibitor in an amount sufficient to modulate expression of a cellular pathway; and (b) administering a second therapy, wherein the modulation of the cellular pathway sensitizes the patient or subject, or increases the efficacy of a second therapy. An increase in efficacy can include a reduction in toxicity, a reduced dosage or duration of the second therapy, or an additive or synergistic effect. A cellular pathway may include, but is not limited to one or more pathway described in Table 2 below or a pathway that is know to include one or more genes of Tables 1, 3, 4, and/or 5. The second therapy may be administered before, during, and/or after the isolated nucleic acid or miRNA or inhibitor is administered

A second therapy can include administration of a second miRNA or therapeutic nucleic acid such as a siRNA or antisense oligonucleotide, or may include various standard therapies, such as pharmaceuticals, chemotherapy, radiation therapy, drug therapy, immunotherapy, and the like. Embodiments of the invention may also include the determination or assessment of gene expression or gene expression profile for the selection of an appropriate therapy. In a particular aspect, a second therapy is chemotherapy. A chemotherapy can include, but is not limited to paclitaxel, cisplatin, carboplatin, doxorubicin, oxaliplatin, larotaxel, taxol, lapatinib, docetaxel, methotrexate, capecitabine, vinorelbine, cyclophosphamide, gemcitabine, amrubicin, cytarabine, etoposide, camptothecin, dexamethasone, dasatinib, tipifamib, bevacizumab, sirolimus, temsirolimus, everolimus, lonafamib, cetuximab, erlotinib, gefitinib imatinib mesylate, rituximab, trastuzumab, nocodazole, sorafenib, sunitinib, bortezomib, alemtuzumab, gemtuzumab, tositumomab or ibritumomab.

Contemplated methods include methods of treating a subject with a disease or condition comprising one or more of the steps of (a) determining an expression profile of one or more genes selected from Table 1, 3, 4, and/or 5; (b) assessing the sensitivity of the subject to therapy based on the expression profile; (c) selecting a therapy based on the assessed sensitivity; and (d) treating the subject using a selected therapy. Typically, the disease or condition will have as a component, indicator, or resulting mis-regulation of one or more gene of Table 1, 3, 4, and/or 5.

In certain aspects, 2, 3, 4, 5, 6, 7, 8, 9, 10, or more miRNA may be used in sequence or in combination. For instance, any combination of miR-126 or a miR-126 inhibitor with another miRNA Further embodiments include the identification and assessment of an expression profile indicative of miR-126 status in a cell or tissue comprising expression assessment of one or more gene from Table 1, 3, 4, and/or 5, or any combination thereof.

The term "miRNA" is used according to its ordinary and plain meaning and refers to a microRNA molecule found in eukaryotes that is involved in RNA-based gene regulation. See, e.g., Carrington *et al.,* 2003. The term can be used to refer to the single-stranded RNA molecule processed from a precursor or in certain instances the precursor itself.

It may be useful to know whether a cell expresses a particular miRNA endogenously or whether such expression is affected under particular conditions or when it is in a particular disease state. Thus, in some embodiments of the invention, methods include assaying a cell or a sample containing a cell for the presence of one or more marker gene or mRNA or other analyte indicative of the expression level of a gene of interest. Consequently, in some embodiments, methods include a step of generating an RNA profile for a sample. The term "RNA profile" or "gene expression profile" refers to a set of data regarding the expression pattern for one or more gene or genetic marker or miRNA in the sample (*e.g.*, a plurality of nucleic acid probes that identify one or more markers from Tables 1, 3, 4, and/or 5); it is contemplated that the nucleic acid profile can be obtained using a set of RNAs, using for example nucleic acid amplification or hybridization techniques well know to one of ordinary skill in the art. The difference in the expression profile in the sample from the patient and a reference expression profile, such as an expression profile of one or more genes or miRNAs, are indicative of which miRNAs to be administered.

It is disclosed that miR-126 or miR-126 inhibitor and let-7 can be administered to patients with breast carcinoma, cervical carcinoma, chronic lymphoblastic leukemia, colorectal carcinoma, glioma, glioblastoma, gastric carcinoma, hepatocellular carcinoma, leukemia, lung carcinoma, multiple myeloma, non-small cell lung carcinoma, ovarian carcinoma, oesophageal carcinoma, pancreatic carcinoma, prostate carcinoma, squamous cell carcinoma of the head and neck, thyroid carcinoma.

The disclosure includes administering miR-126 or miR-126 inhibitor and miR-15 to patients with breast carcinoma, B-cell lymphoma, cervical carcinoma, colorectal carcinoma, glioma, glioblastoma, gastric carcinoma, hepatocellular carcinoma, lung carcinoma, multiple myeloma, non-small cell lung carcinoma, ovarian carcinoma, oesophageal carcinoma, pancreatic carcinoma, prostate carcinoma, rhabdomyosarcoma, squamous cell carcinoma of the head and neck, thyroid carcinoma.

It is disclosed that miR-126 or miR-126 inhibitor and miR-16 are administered to patients with breast carcinoma, B-cell lymphoma, colorectal carcinoma, glioblastoma, gastric carcinoma, hepatocellular carcinoma, multiple myeloma, non-small cell lung carcinoma, ovarian carcinoma, oesophageal carcinoma, pancreatic carcinoma, prostate carcinoma, rhabdomyosarcoma, squamous cell carcinoma of the head and neck, thyroid carcinoma.

It is disclosed that miR-126 or miR-126 inhibitor and miR-20 are administered to patients with breast carcinoma, cervical carcinoma, colorectal carcinoma, glioma, glioblastoma, gastric carcinoma, hepatocellular carcinoma leukemia, lipoma, multiple myeloma, non-small cell lung carcinoma, ovarian carcinoma, oesophageal carcinoma, osteosarcoma, pancreatic carcinoma, prostate carcinoma, squamous cell carcinoma of the head and neck, thyroid carcinoma.

Methods where miR-126 or miR-126 inhibitor and miR-21 are administered to patients with breast carcinoma, colorectal carcinoma, glioma, glioblastoma, gastric carcinoma, hepatocellular carcinoma, non-small cell lung carcinoma, ovarian carcinoma, oesophageal carcinoma, pancreatic carcinoma, prostate carcinoma, rhabdomyosarcoma, squamous cell carcinoma of the head and neck are disclosed.

It is disclosed that miR-126 or miR-126 inhibitor and miR-26a are administered to patients with anaplastic large cell lymphoma, breast carcinoma, B-cell lymphoma, cervical carcinoma, chronic lymphoblastic leukemia, colorectal carcinoma, glioma, glioblastoma, gastric carcinoma, hepatocellular carcinoma, leukemia, lung carcinoma, multiple myeloma, non-small cell lung carcinoma, ovarian carcinoma, oesophageal carcinoma, osteosarcoma, pancreatic carcinoma, prostate carcinoma, rhabdomyosarcoma, testicular tumor.

It is disclosed that miR-126 or miR-126 inhibitor and miR-34a are administered to patients with anaplastic large cell lymphoma, breast carcinoma, B-cell lymphoma, cervical carcinoma, chronic lymphoblastic leukemia, colorectal carcinoma, glioma, glioblastoma, gastric carcinoma, hepatocellular carcinoma, leukemia, lung carcinoma, multiple myeloma, mesothelioma, non-small cell lung carcinoma, ovarian carcinoma, oesophageal carcinoma, osteosarcoma, pancreatic carcinoma, prostate carcinoma, rhabdomyosarcoma, squamous cell carcinoma of the head and neck, thyroid carcinoma, testicular tumor.

It is disclosed that miR-126 or miR-126 inhibitor and miR-143 are administered to patients with anaplastic large cell lymphoma, breast carcinoma, B-cell lymphoma, cervical carcinoma, chronic lymphoblastic leukemia, colorectal carcinoma, glioma, glioblastoma, gastric carcinoma, hepatocellular carcinoma, leukemia, lung carcinoma, multiple myeloma, non-small cell lung carcinoma, ovarian carcinoma, oesophageal carcinoma, osteosarcoma, pancreatic carcinoma, prostate carcinoma, squamous cell carcinoma of the head and neck, thyroid carcinoma, testicular tumor.

It is disclosed that miR-126 or miR-126 inhibitor and miR-147 are administered to patients with breast carcinoma, cervical carcinoma, colorectal carcinoma, glioma, glioblastoma, gastric carcinoma, hepatocellular carcinoma, leukemia, lipoma, multiple myeloma, non-small cell lung carcinoma, ovarian carcinoma, oesophageal carcinoma, osteosarcoma, pancreatic carcinoma, prostate carcinoma, squamous cell carcinoma of the head and neck, thyroid carcinoma.

It is disclosed that miR-126 or miR-126 inhibitor and miR-188 are administered to patients with anaplastic large cell lymphoma, breast carcinoma, B-cell lymphoma, cervical carcinoma, chronic lymphoblastic leukemia, colorectal carcinoma, glioma, glioblastoma, gastric carcinoma, hepatocellular carcinoma, leukemia, lung carcinoma, multiple myeloma, non-small cell lung carcinoma, ovarian carcinoma, oesophageal carcinoma, pancreatic carcinoma, prostate carcinoma, squamous cell carcinoma of the head and neck, thyroid carcinoma, testicular tumor.

It is disclosed that miR-126 or miR-126 inhibitor and miR-200 are administered to patients with breast carcinoma, cervical carcinoma, colorectal carcinoma, glioma, glioblastoma, gastric carcinoma, hepatocellular carcinoma, leukemia, lung carcinoma, mesothelioma, non-small cell lung carcinoma, ovarian carcinoma, oesophageal carcinoma, osteosarcoma, pancreatic carcinoma, prostate carcinoma, rhabdomyosarcoma, squamous cell carcinoma of the head and neck, thyroid carcinoma.

It is disclosed that miR-126 or miR-126 inhibitor and miR-215 are administered to patients with anaplastic large cell lymphoma, breast carcinoma, B-cell lymphoma, cervical carcinoma, chronic lymphoblastic leukemia, colorectal carcinoma, glioma, glioblastoma, gastric carcinoma, hepatocellular carcinoma, leukemia, lung carcinoma, lipoma, multiple myeloma, mesothelioma, non-small cell lung carcinoma, ovarian carcinoma, oesophageal carcinoma, osteosarcoma, pancreatic carcinoma, prostate carcinoma, rhabdomyosarcoma, squamous cell carcinoma of the head and neck, thyroid carcinoma, testicular tumor.

It is disclosed that miR-126 or miR-126 inhibitor and miR-216 are administered to patients with breast carcinoma, cervical carcinoma, colorectal carcinoma, glioma, glioblastoma, gastric carcinoma, hepatocellular carcinoma, leukemia, lung carcinoma, non-small cell lung carcinoma, ovarian carcinoma, oesophageal carcinoma, osteosarcoma, prostate carcinoma, squamous cell carcinoma of the head and neck, testicular tumor.

It is disclosed that miR-126 or miR-126 inhibitor and miR-292-3p are administered to patients with anaplastic large cell lymphoma, breast carcinoma, B-cell lymphoma, cervical carcinoma, colorectal carcinoma, glioma, glioblastoma, gastric carcinoma, hepatocellular carcinoma, leukemia, lung carcinoma, lipoma, multiple myeloma, non-small cell lung carcinoma, ovarian carcinoma, oesophageal carcinoma, osteosarcoma, pancreatic carcinoma, prostate carcinoma, rhabdomyosarcoma, squamous cell carcinoma of the head and neck, thyroid carcinoma, testicular tumor.

It is disclosed that miR-126 or miR-126 inhibitor and miR-331 are administered to patients with anaplastic large cell lymphoma, breast carcinoma, B-cell lymphoma, cervical carcinoma, chronic lymphoblastic leukemia, colorectal carcinoma, glioma, glioblastoma, gastric carcinoma, hepatocellular carcinoma, leukemia, lung carcinoma, multiple myeloma, ovarian carcinoma, oesophageal carcinoma, osteosarcoma, pancreatic carcinoma, prostate carcinoma, rhabdomyosarcoma, squamous cell carcinoma of the head and neck, thyroid carcinoma, testicular tumor.

It is contemplated that when miR-126 or a miR-126 inhibitor is given in combination with one or more other miRNA molecules, the two different miRNAs or inhibitors may be given at the same time or sequentially. In some embodiments, therapy proceeds with one miRNA or inhibitor and that therapy is followed up with therapy with the other miRNA or inhibitor 1, 2, 3, 4, 5, 6, 7, 8, 9, 10, 15, 20, 25, 30, 35, 40, 45, 50, 55 minutes, 1, 2, 3, 4, 5, 6, 7, 8, 9, 10, 11, 12, 13, 14, 15, 16, 17, 18, 19, 20, 21, 22, 23, 24 hours, 1, 2, 3, 4, 5, 6, 7 days, 1, 2, 3, 4, 5 weeks, or 1, 2, 3, 4, 5, 6, 7, 8, 9, 10, 11, or 12 months or any such combination later.

Further embodiments include the identification and assessment of an expression profile indicative of miR-126 status in a cell or tissue comprising expression assessment of one or more gene from Table 1, 3, 4, and/or 5, or any combination thereof.

The term "miRNA" is used according to its ordinary and plain meaning and refers to a microRNA molecule found in eukaryotes that is involved in RNA-based gene regulation. See, e.g., *Carrington et al.,* 2003. The term can be used to refer to the single-stranded RNA molecule processed from a precursor or in certain instances the precursor itself or a mimetic thereof.

It may be useful to know whether a cell expresses a particular miRNA endogenously or whether such expression is affected under particular conditions or when it is in a particular disease state. Thus, in some embodiments of the invention, methods include assaying a cell or a sample containing a cell for the presence of one or more miRNA marker gene or mRNA or other analyte indicative of the expression level of a gene of interest. Consequently, in some embodiments, methods include a step of generating an RNA profile for a sample. The term "RNA profile" or "gene expression profile" refers to a set of data regarding the expression pattern for one or more gene or genetic marker in the sample (*e.g.*, a plurality of nucleic acid probes that identify one or more markers or genes from Tables 1, 3, 4, and/or 5); it is contemplated that the nucleic acid profile can be obtained using a set of RNAs, using for example nucleic acid amplification or hybridization techniques well know to one of ordinary skill in the art. The difference in the expression profile in the sample from a patient and a reference expression profile, such as an expression profile from a normal or non-pathologic sample, or a digitized reference, is indicative of a pathologic, disease, or cancerous condition. In certain aspects the expression profile is an indicator of a propensity to or probability *of* (*i.e.,* risk factor for a disease or condition) developing such a condition(s). Such a risk or propensity may indicate a treatment, increased monitoring, prophylactic measures, and the like. A nucleic acid or probe set may comprise or identify a segment of a corresponding mRNA and may include all or part of 1, 2, 3, 4, 5, 6, 7, 8, 9, 10, 11, 12 ,13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 100, 200, 500, or more segments, including any integer or range derivable there between, of a gene or genetic marker, or a nucleic acid, mRNA or a probe representative thereof that is listed in Tables 1, 3, 4, and/or 5 or identified by the methods described herein.

Certain embodiments of the invention are directed to compositions and methods for assessing, prognosing, or treating a pathological condition in a patient comprising measuring or determining an expression profile of one or more miRNA or marker(s) in a sample from the patient, wherein a difference in the expression profile in the sample from the patient and an expression profile of a normal sample or reference expression profile is indicative of pathological condition and particularly cancer (*e.g.*, In certain aspects of the invention, the miRNAs, cellular pathway, gene, or genetic marker is or is representative of one or more pathway or marker described in Table 1, 2, 3, 4, and/or 5, including any combination thereof.

Diagnosing, assessing, or treating a pathologic condition or preventing a pathologic condition from manifesting are disclosed. For example, the methods can be used to screen for a pathological condition; assess prognosis of a pathological condition; stage a pathological condition; assess response of a pathological condition to therapy; or to modulate the expression of a gene, genes, or related pathway as a first therapy or to render a subject sensitive or more responsive to a second therapy. In particular aspects, assessing the pathological condition of the patient can be assessing prognosis of the patient. Prognosis may include, but is not limited to an estimation of the time or expected time of survival, assessment of response to a therapy, and the like. In certain aspects, the altered expression of one or more gene or marker is prognostic for a patient having a pathologic condition, wherein the marker is one or more of Table 1, 3, 4, and/or 5, including any combination thereof.

**Table 2. Significantly affected functional cellular pathways following hsa-miR-126 overexpression in human cancer cells.**

| **Number of Genes** | **Pathway Functions** |
|---|---|
| 23 | Cancer, Cellular Movement, Tumor Morphology |
| 16 | Cellular Growth and Proliferation, Skeletal and Muscular System Development and Function, Cancer |
| 16 | Cellular Movement, Cellular Assembly and Organization, Drug Metabolism |
| 16 | Cellular Assembly and Organization, Cancer, Skeletal and Muscular Disorders |
| 15 | Cell Morphology, Cellular Assembly and Organization, Cellular Development |
| 15 | Carbohydrate Metabolism, Connective Tissue Disorders, Inflammatory Disease |
| 15 | Cell Cycle, Lipid Metabolism, Molecular Transport |
| 13 | Cell-To-Cell Signaling and Interaction, Immune Response, Immune and Lymphatic System Development and Function |
| 11 | Cellular Function and Maintenance, Cellular Assembly and Organization, Cell Cycle |
| 1 | Cell Cycle, Connective Tissue Development and Function, Immune Response |
| 1 | Developmental Disorder, Endocrine System Disorders, Lipid Metabolism |
| 1 | Immune Response, Cellular Assembly and Organization, Gene Expression |
| 1 | Cell Death, Cell-To-Cell Signaling and Interaction, Cellular Assembly and Organization |
| 1 | Molecular Transport, Protein Trafficking, Cell-To-Cell Signaling and Interaction |
| 1 | Cell Signaling, Molecular Transport, Neurological Disease |

**Table 3. Predicted target genes of hsa-miR-126 for Ref Seq ID reference - Pruitt et al., 2005.**

| **Gene Symbol** | **RefSeq Transcript ID (Pruitt *et al.,* 2005)** | **Description** |
|---|---|---|
| ACPL2 | NM_152282 | acid phosphatase-like 2 |
| ADAM9 | NM_001005845 | ADAM metallopeptidase domain 9 isoform 2 |
| AGPAT3 | NM_020132 | 1-acylglycerol-3-phosphate O-acyltransferase 3 |
| AIPL1 | NM_001033054 | aryl hydrocarbon receptor interacting |
| AKAP13 | NM_006738 | A-kinase anchor protein 13 isoform 1 |
| AND25 | NM_015493 | ankyrin repeat domain 25 |
| ANTXR2 | NM_058172 | anthrax toxin receptor 2 |
| APC2 | NM_005883 | adenomatosis polyposis coli 2 |
| APOA5 | NM_052968 | apolipoprotein AV |
| ARL8A | NM_138795 | ADP-ribosylation factor-like 10B |
| ARMCX1 | NM_016608 | armadillo repeat containing, X-linked 1 |
| B4GALT4 | NM_003778 | UDP-Gal:betaGlcNAc beta 1,4- |
| BCL2 | NM_000633 | B-cell lymphoma protein 2 alpha isoform |
| BICD2 | NM_001003800 | bicaudal D homolog 2 isoform 1 |
| C17orf81 | NM_203413 | S-phase 2 protein isoform 2 |
| C1orf187 | NM_198545 | chromosome 1 open reading frame 187 |
| C1orf55 | NM_152608 | hypothetical protein LOC163859 |
| C20orf28 | NM_015417 | hypothetical protein LOC25876 |
| C20orf42 | NM_017671 | chromosome 20 open reading frame 42 |
| C8orf51 | NM_024035 | hypothetical protein LOC78998 |
| C9orf66 | NM_152569 | hypothetical protein LOC157983 |
| CACNA2D4 | NM_001005737 | voltage-gated calcium channel alpha(2)delta-4 |
| CAMSAP1 | NM_015447 | calmodulin regulated spectrin-associated protein |
| CAPN3 | NM_212464 | calpain 3 isoform g |
| CARF | NM_017632 | collaborates/cooperates with ARF (alternate |
| CENTD1 | NM_015230 | centaurin delta 1 isoform a |
| CENTG1 | NM_014770 | centaurin, gamma 1 |
| CHST3 | NM_004273 | carbohydrate (chondroitin 6) sulfotransferase 3 |
| CHST6 | NM_021615 | carbohydrate (N-acetylglucosamine 6-O) |
| CLCA3 | NM_004921 | calcium activated chloride channel 3 precursor |
| CNP | NM_033133 | 2',3'-cyclic nucleotide 3' phosphodiesterase |
| CRK | NM_005206 | v-crk sarcoma virus CT10 oncogene homolog |
| CTSB | NM_001908 | cathepsin B preproprotein |
| DDX11 | NM_030655 | DEAD/H (Asp-Glu-Ala-Asp/His) box polypeptide 11 |
| DFFB | NM_001004285 | DNA fragmentation factor, 40 kD, beta |
| DIP2C | NM_014974 | hypothetical protein LOC22982 |
| DNMT3A | NM_022552 | DNA cytosine methyltransferase 3 alpha isoform |
| EDG4 | NM_004720 | endothelial differentiation, lysophosphatidic |
| EFHD2 | NM_024329 | EF hand domain family, member D2 |
| EGFL7 | NM_016215 | EGF-like-domain, multiple 7 |
| ELOF1 | NM_032377 | elongation factor 1 homolog (ELF1, S. |
| EMILIN3 | NM_052846 | elastin microfibril interfacer 3 |
| EP400 | NM_015409 | E1A binding protein p400 |
| EPDR1 | NM_017549 | upregulated in colorectal cancer gene 1 protein |
| EVI5 | NM_005665 | ecotropic viral integration site 5 |
| F8A1 | NM_012151 | coagulation factor VIII-associated protein |
| FAM109A | NM_144671 | hypothetical protein LOC144717 |
| FAM46C | NM_017709 | hypothetical protein LOC54855 |
| FARP1 | NM_005766 | FERM, RhoGEF, and pleckstrin domain protein 1 |
| FBXL2 | NM_012157 | F-box and leucine-rich repeat protein 2 |
| FBXO33 | NM_203301 | F-box protein 33 |
| FLJ10159 | NM_018013 | hypothetical protein LOC55084 |
| FLJ10769 | NM_018210 | hypothetical protein LOC55739 |
| FLJ16542 | NM_001004301 | hypothetical protein LOC126017 |
| FLJ21687 | NM_024859 | PDZ domain containing, X chromosome |
| FLJ25530 | NM_152722 | hepatocyte cell adhesion molecule |
| FLJ38973 | NM_153689 | hypothetical protein LOC205327 |
| FLJ45121 | NM_207451 | hypothetical protein LOC400556 |
| GATAD2B | NM_020699 | GATA zinc finger domain containing 2B |
| GOLGA8E | NM_001012423 | golgi autoantigen, golgin family member |
| GOLGA8G | NM_001012420 | hypothetical protein LOC283768 |
| GOLPH3 | NM_022130 | golgi phosphoprotein 3 |
| GRIN2B | NM_000834 | N-methyl-D-aspartate receptor subunit 2B |
| HERPUD1 | NM_001010989 | homocysteine-inducible, endoplasmic reticulum |
| HIP1 | NM_005338 | huntingtin interacting protein 1 |
| IL21R | NM_021798 | interleukin 21 receptor precursor |
| IL6ST | NM_175767 | interleukin 6 signal transducer isoform 2 |
| IRS1 | NM_005544 | insulin receptor substrate 1 |
| IRS2 | NM_003749 | insulin receptor substrate 2 |
| ITGA6 | NM_000210 | integrin alpha chain, alpha 6 |
| KAL1 | NM_000216 | Kallmann syndrome 1 protein |
| KBTBD8 | NM_032505 | T-cell activation kelch repeat protein |
| KCNJ1 | NM_000220 | potassium inwardly-rectifying channel J1 isoform |
| KIAA0556 | NM_015202 | hypothetical protein LOC23247 |
| KIAA0683 | NM_016111 | hypothetical protein LOC9894 |
| KIAA1456 | NM_020844 | hypothetical protein LOC57604 |
| KIAA1559 | NM_020917 | zinc finger protein 14-like |
| KIAA1715 | NM_030650 | Lunapark |
| KIAA1755 | NM_001029864 | hypothetical protein LOC85449 |
| KLHL3 | NM_017415 | kelch-like 3 (Drosophila) |
| LARGE | NM_004737 | like-glycosyltransferase |
| LARP6 | NM_018357 | acheron isoform 1 |
| LHCGR | NM_000233 | luteinizing hormone/choriogonadotropin receptor |
| LOC147808 | NM_203374 | hypothetical protein LOC147808 |
| LOC283849 | NM_178516 | hypothetical protein LOC283849 |
| LOC339524 | NM_207357 | hypothetical protein LOC339524 |
| LOC399706 | NM_001010910 | hypothetical protein LOC399706 |
| LOC401410 | NM_001008742 | hypothetical protein LOC401410 |
| LTBR | NM_002342 | lymphotoxin beta receptor |
| MAWBP | NM_022129 | MAWD binding protein isoform a |
| MGC42367 | NM_207362 | hypothetical protein LOC343990 |
| MGC4268 | NM_031445 | hypothetical protein LOC83607 |
| MGEA5 | NM_012215 | meningioma expressed antigen 5 (hyaluronidase) |
| MOSC1 | NM_022746 | MOCO sulphurase C-terminal domain containing 1 |
| MTAP | NM_002451 | 5'-methylthioadenosine phosphorylase |
| MTG1 | NM_138384 | GAP_binding protein |
| NF2 | NM_181826 | neurofibromin 2 isoform 3 |
| NFASC | NM_015090 | neurofascin precursor |
| NUAK1 | NM_014840 | AMPK-related protein kinase 5 |
| OPA3 | NM_001017989 | OPA3 protein isoform a |
| ORMDL3 | NM_139280 | ORM1-like 3 |
| PARN | NM_002582 | poly(A)-specific ribonuclease (deadenylation |
| PARP16 | NM_017851 | poly (ADP-ribose) polymerase family, member 16 |
| PDAP1 | NM_014891 | PDGFA associated protein 1 |
| PEX5 | NM_000319 | peroxisomal biogenesis factor 5 |
| PHF15 | NM_015288 | PHD finger protein 15 |
| PHF21 B | NM_138415 | PHD finger protein 21 B |
| PHOSPHO1 | NM_178500 | phosphatase, orphan 1 |
| PIK3CD | NM_005026 | phosphoinositide-3-kinase, catalytic, delta |
| PIK3R2 | NM_005027 | phosphoinositide-3-kinase, regulatory subunit 2 |
| PITPNC1 | NM_012417 | phosphatidylinositol transfer protein, |
| PKD1L1 | NM_138295 | polycystin-1L1 |
| PKD2 | NM_000297 | polycystin 2 |
| PLK2 | NM_006622 | polo-like kinase 2 |
| PMCHL1 | NM_031887 | pro-melanin-concentrating hormone-like 1 |
| PMM1 | NM_002676 | phosphomannomutase 1 |
| PPP3CB | NM_021132 | protein phosphatase 3 (formerly 2B), catalytic |
| PRPF4B | NM_003913 | serine/threonine-protein kinase PRP4K |
| PRX | NM_020956 | periaxin isoform 1 |
| PTPN9 | NM_002833 | protein tyrosine phosphatase, non-receptor type |
| QDPR | NM_000320 | quinoid dihydropteridine reductase |
| RAB12 | NM_001025300 | RAB12, member RAS oncogene family |
| RASSF2 | NM_014737 | Ras association domain family 2 |
| RGS3 | NM_021106 | regulator of G-protein signalling 3 isoform 2 |
| RHOU | NM_021205 | ras homolog gene family, member U |
| RNF165 | NM_152470 | ring finger protein 165 |
| RNF182 | NM_152737 | ring fmger protein 182 |
| SAMD12 | NM_207506 | sterile alpha motif domain containing 12 |
| SCAMP4 | NM_079834 | secretory carrier membrane protein 4 |
| SDC2 | NM_002998 | syndecan 2 precursor |
| SEMA4D | NM_006378 | semaphorin 4D |
| SERPINB 13 | NM_012397 | serine (or cysteine) proteinase inhibitor, clade |
| SFRS11 | NM_004768 | splicing factor p54 |
| SGK2 | NM_016276 | serum/glucocorticoid regulated kinase 2 isoform |
| SLC15A4 | NM_145648 | solute carrier family 15, member 4 |
| SLC4A4 | NM_003759 | solute carrier family 4, sodium bicarbonate |
| SLC7A5 | NM_003486 | solute carrier family 7 (cationic amino acid |
| SLC7A8 | NM_012244 | solute carrier family 7 (cationic amino acid |
| SLC9A6 | NM_006359 | solute carrier family 9 (sodium/hydrogen |
| SLTM | NM_017968 | modulator of estrogen induced transcription |
| SMOC2 | NM_022138 | secreted modular calcium-binding protein 2 |
| SOX2 | NM_003106 | sex-determining region Y-box 2 |
| SOX21 | NM_007084 | SRY-box 21 |
| SPG20 | NM_015087 | spartin |
| SPON1 | NM_006108 | spondin 1, extracellular matrix protein |
| SPRED1 | NM_152594 | sprouty-related protein 1 with EVH-1 domain |
| STX12 | NM_177424 | syntaxin 12 |
| SYT8 | NM_138567 | synaptotagmin VIII |
| TCF2 | NM_006481 | transcription factor 2 isoform b |
| THAP6 | NM_144721 | THAP domain containing 6 |
| THUMPD1 | NM_017736 | THUMP domain containing 1 |
| TMEM32 | NM_173470 | transmembrane protein 32 |
| TMEM40 | NM_018306 | transmembrane protein 40 |
| TNFRSF10B | NM_003842 | tumor necrosis factor receptor superfamily, |
| TOM1 | NM_005488 | target of mybl |
| TPD52L1 | NM_001003396 | tumor protein D52-like 1 isoform 3 |
| TRAF7 | NM_032271 | ring finger and WD repeat domain 1 isoform 1 |
| TRPC4AP | NM_015638 | TRPC4-associated protein isoform a |
| TRPS1 | NM_014112 | zinc finger transcription factor TRPS 1 |
| TSC1 | NM_000368 | tuberous sclerosis 1 protein isoform 1 |
| TTC22 | NM_017904 | hypothetical protein LOC55001 |
| UBE2Q1 | NM_017582 | ubiquitin-conjugating enzyme E2Q |
| UBQLN2 | NM_013444 | ubiquilin 2 |
| VCAM1 | NM_001078 | vascular cell adhesion molecule 1 isoform a |
| WSB1 | NM_134264 | WD SOCS-box protein 1 isoform 3 |
| ZADH2 | NM_175907 | zinc binding alcohol dehydrogenase, domain |
| ZNF219 | NM_016423 | zinc finger protein 219 |
| ZNF713 | NM_182633 | zinc finger protein 713 |

**Table 4. hsa-miR-126 predicted targets that exhibited altered mRNA expression levels in human cancer cells after transfection with pre-miR-126.**

| **Gene Svmbol** | **RefSeq Transcript ID** (Pruitt *et al.,* 2005) | **Description** |
|---|---|---|
| ADAM9 | NM_001005845 | ADAM metallopeptidase domain 9 isoform 2 |
| B4GALT4 | NM_003778 | UDP-Gal:betaGlcNAc beta 1,4- |
| EFHD2 | NM_024329 | EF hand domain family, member D2 |
| F8A1 | NM_012151 | coagulation factor VIII-associated protein |
| IL6ST | NM_175767 | interleukin 6 signal transducer isoform 2 |
| LARP6 | NM_018357 | acheron isoform 1 |
| MAWBP | NM_022129 | MAWD binding protein isoform a |
| NF2 | NM_181826 | neurofibromin 2 isoform 3 |
| PMM1 | NM_002676 | phosphomannomutase 1 |
| PPP3CB | NM_021132 | protein phosphatase 3 (formerly 2B), catalytic |
| QDPR | NM_000320 | quinoid dihydropteridine reductase |
| RASSF2 | NM_014737 | Ras association domain familv 2 |
| SLC4A4 | NM_003759 | solute carrier family 4, sodium bicarbonate |
| SLC7A5 | NM_003486 | solute carrier family 7 (cationic amino acid |

The predicted gene targets Table 3. Predicted target genes of hsa-miR-126 whose mRNA expression levels are affected by hsa-miR-126 represent particularly useful candidates for cancer therapy and therapy of other diseases through manipulation of their expression levels.

Disclosed is determining expression of one or more marker, gene, or nucleic acid segment representative of one or more genes, by using an amplification assay, a hybridization assay, or protein assay, a variety of which are well known to one of ordinary skill in the art. In certain aspects, an amplification assay can be a quantitative amplification assay, such as quantitative RT-PCR or the like. In still further aspects, a hybridization assay can include array hybridization assays or solution hybridization assays. The nucleic acids from a sample may be labeled from the sample and/or hybridizing the labeled nucleic acid to one or more nucleic acid probes. Nucleic acids, mRNA, and/or nucleic acid probes may be coupled to a support. Such supports are well known to those of ordinary skill in the art and include, but are not limited to glass, plastic, metal, or latex. In particular aspects of the invention, the support can be planar or in the form of a bead or other geometric shapes or configurations known in the art. Proteins are typically assayed by immunoblotting, chromatography, or mass spectrometry or other methods known to those of ordinary skill in the art.

Disclosed are also kits containing compositions of the invention or compositions to implement methods of the invention. In some embodiments, kits can be used to evaluate one or more marker molecules, and/or express one or more miRNA or miRNA inhibitor. In certain embodiments, a kit contains, contains at least or contains at most 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 100, 150, 200 or more probes, recombinant nucleic acid, or synthetic nucleic acid molecules related to the markers to be assessed or an miRNA or miRNA inhibitor to be expressed or modulated, and may include any range or combination derivable therein. Kits may comprise components, which may be individually packaged or placed in a container, such as a tube, bottle, vial, syringe, or other suitable container means. Individual components may also be provided in a kit in concentrated amounts; in some embodiments, a component is provided individually in the same concentration as it would be in a solution with other components. Concentrations of components may be provided as 1x, 2x, 5x, 10x, or 20x or more. Kits for using probes, synthetic nucleic acids, recombinant nucleic acids, or non-synthetic nucleic acids of the invention for therapeutic, prognostic, or diagnostic applications are included as part of the invention. Specifically contemplated are any such molecules corresponding to any miRNA reported to influence biological activity or expression of one or more marker gene or gene pathway described herein. In certain aspects, negative and/or positive controls are included in some kit embodiments. The control molecules can be used to verify transfection efficiency and/or control for transfection-induced changes in cells..

Disclosed is a kit for assessment of a pathological condition or the risk of developing a pathological condition in a patient by nucleic acid profiling of a sample comprising, in suitable container means, two or more nucleic acid hybridization or amplification reagents. The kit can comprise reagents for labeling nucleic acids in a sample and/or nucleic acid hybridization reagents. The hybridization reagents typically comprise hybridization probes. Amplification reagents include, but are not limited to amplification primers, reagents, and enzymes.

It is contemplated that an expression profile is generated by steps that include: (a) labeling nucleic acid in the sample; (b) hybridizing the nucleic acid to a number of probes, or amplifying a number of nucleic acids, and (c) determining and/or quantitating nucleic acid hybridization to the probes or detecting and quantitating amplification products, wherein an expression profile is generated. See U.S. Provisional Patent Application 60/575,743 and the U.S. Provisional Patent Application 60/649,584, and U.S. Patent Application Serial No. 11/141,707 and U.S. Patent Application Serial No. 11/273,640.

Disclosed methods involve diagnosing and/or assessing the prognosis of a patient based on a miRNA and/or a marker nucleic acid expression profile. In certain embodiments, the elevation or reduction in the level of expression of a particular gene or genetic pathway or set of nucleic acids in a cell is correlated with a disease state or pathological condition compared to the expression level of the same in a normal or non-pathologic cell or tissue sample. This correlation allows for diagnostic and/or prognostic methods to be carried out when the expression level of one or more nucleic acid is measured in a biological sample being assessed and then compared to the expression level of a normal or non-pathologic cell or tissue sample. It is specifically contemplated that expression profiles for patients, particularly those suspected of having or having a propensity for a particular disease or condition such as cancer, can be generated by evaluating any of or sets of the miRNAs and/or nucleic acids discussed in this application. The expression profile that is generated from the patient will be one that provides information regarding the particular disease or condition. In many embodiments, the profile is generated using nucleic acid hybridization or amplification, (e.g., array hybridization or RT-PCR). In certain aspects, an expression profile can be used in conjunction with other diagnostic and/or prognostic tests, such as histology, protein profiles in the serum and/or cytogenetic assessment.

**Table 5. Tumor associated mRNAs altered by hsa-miR-126 having prognostic or therapeutic value for the treatment of various malignancies.**

| **Gene Symbol** | **Gene Title** | **Cellular Process** | **Cancer Type** | **Reference** |
|---|---|---|---|---|
| BCL6 | BCL-6 | apoptosis | NHL | (Carbone *et al*., 1998; Butler *et al.,* 2002) |
| CCND1 | cyclin D1 | cell cycle | MCL, BC, SCCHN, OepC, HCC, CRC, BldC, EC, OC, M, AC, GB, GC, PaC | (Donnellan and Chetty, 1998) |
| CCNG1 | cyclin G1 | cell cycle | OS, BC, PC | (Skotzko *et al.,* 1995; Reimer *et al.,* 1999) |
| CEBPD | C/EBP delta | transcription | PC | (Yang *et al.,* 2001) |
| CTGF | CTGF/IGFBP-8 | cell adhesion, migration | BC, GB, OepC, RMS, CRC, PC, | (Hishikawa *et al.,* 1999; Shimo *et al.,* 2001; Koliopanos et *al.,* 2002; Pan *et al.,* 2002; Croci *et al.,* 2004; Lin *et al.,* 2005; Yang *et al*., 2005) |
| EGFR | EGFR | signal transduction | SCCHN, G, BC, LC, OC, OepC, NSCLC | (Hynes and Lane, 2005) |
| EPHB2 | EPH receptor B2 | signal transduction | PC, GC, CRC, OC, G, BC | (Huusko *et al.,* 2004; Nakada *et al.,* 2004; Wu *et al.,* 2004; Jubb *et al.*, 2005; Davalos *et al.,* 2006; Guo *et al.,* 2006; Kokko *et al*., 2006; Wu *et al.,* 2006b) |
| ERBB3 | HER-3 | signal transduction | PC, BC, pilocytic AC, GC, CRC, OC, BldC | (Lemoine *et al*., 1992; Rajkumar *et al.,* 1996; Leng *et al.*, 1997; Maurer *et al.,* 1998; Kobayashi *et al*., 2003; Koumakpayi *et al.,* 2006; Xue *et al.,* 2006) |
| EREG | epiregulin | signal transduction | BldC, CRC, PaC, PC | (Baba *et al.,* 2000; Torring *et al.,* 2000; Zhu *et al.,* 2000; Thogersen *et al.,* 2001) |
| FAS | Fas | apoptosis | NSCLC, G, L, CRC, OepC | (Moller *et al.,* 1994; Gratas *et al.,* 1998; Martinez-Lorenzo *et al*., 1998; Shinoura *et al.,* 2000; Viard-Leveugle *et al.,* 2003) |
| FGFR1 | FGF receptor-1 | signal transduction | L, CRC, BC, RCC, OC, M, NSCLC | (Chandler *et al.,* 1999) |
| FGFR4 | FGF receptor-4 | signal transduction | TC, BC, OC, PaC | (Jaakkola *et al.*, 1993; Shah *et al.,* 2002; Ezzat *et al.,* 2005) |
| ILK | integrin-linked kinase | signal transduction | PC, CRC, GC, EWS, M, BC | (Hannigan *et al.,* 2005) |
| JUN | c-Jun | transcription | HL, HCC | (Eferl *et al.,* 2003; Weiss and Bohmann, 2004) |
| LCN2 | lipocalin 2 / NGAL | cell adhesion | PaC, CRC, HCC, BC, OC | (Bartsch and Tschesche, 1995; Furutani *et al.,* 1998; Fernandez *et al.,* 2005; Lee *et al.,* 2006) |
| MCL1 | Mcl-1 | apoptosis | HCC, MM, TT, CLL, ALCL, BCL, PC | (Krajewska *et al.,* 1996; Kitada *et al.,* 1998; Cho-Vega *et al.,* 2004; Rust *et al.,* 2005; Sano *et al.,* 2005; Wuilleme-Toumi *et al.,* 2005; Fleischer *et al.,* 2006; Sieghart *et al*., 2006) |
| MET | c-Met | signal transduction | SPRC, HCC, GC, SCCHN, OS, RMS, GB, BC, M, CRC, GI, PaC, PC, OC | (Boccaccio and Comoglio, 2006) |
| MYBL1 | A-Myb | transcription | BL | (Golay *et al*., 1996) |
| NF1 | NF-1 | signal transduction | G, AC, NF, PCC, ML | (Rubin and Gutmann, 2005) |
| NF2 | Merlin/NF-2 | cell adhesion | Schw, TC, HCC, MG, MT of lung | (McClatchey and Giovannini, 2005) |
| PDCD4 | Pdcd-4 | apoptosis | G, HCC, L, RCC | (Chen *et al.,* 2003; Jansen *et al.,* 2004; Zhang *et al.,* 2006; Gao *et al.,* 2007) |
| PLCB1 | PLC-beta1 | signal transduction | AML | (Lo Vasco *et al.,* 2004) |
| PLK1 | polo-like kinase 1 | chromosomal stability | NSCLC, OrpC, OepC, GC, M, BC, OC, EC, CRC, GB, PapC, PaC, PC, HB, NHL | (Strebhardt and Ullrich, 2006) |
| PRKCA | PKC alpha | signal transduction | BldC, PC, EC, BC, CRC, HCC, M, GC, OC | *(*Weichert *et al*., 2003; *Jiang et al*., 2004; Lahn and Sundell, 2004; Koivunen *et al.,* 2006) |
| PXN | paxillin | cell adhesion, motility | SCLC, M | (Salgia *et al.,* 1999; Hamamura *et al.,* 2005) |
| RARRES1 | RAR responder 1 | migration, invasion | CRC, PC | (Zhang *et al.,* 2004; Wu *et al.,* 2006a) |
| RASSF2 | RASSF2 | signal transduction | GC, CRC, OC, LC | (Vos *et al.,* 2003; Akino *et al.,* 2005; Endoh *et al.,* 2005; Lambros *et al.,* 2005) |
| TGFBR2 | TGF beta receptor type II | signal transduction | BC, CRC | (Markowitz, 2000; Lucke *et al.,* 2001; Biswas *et al.,* 2004) |
| TGFBR3 | TGF beta receptor III | signal transduction | CeC, high grade NHL, CRC, BC, PC, RCC, EC | (Venkatasubbarao et *al., 2000;* Bandyopadhyay *et al.,* 2002; Copland *et al.,* 2003; Woszczyk *et al.,* 2004; Florio *et al.,* 2005; Soufla *et al*., 2005; Turley *et al.,* 2007) |
| TNFSF10 | TRAIL | apoptosis | CRC, G, LC, PC, multiple ML | (Fesik, 2005) |
| TP73L | p63 | transcription | CeC, PC, SCCHN, LC, BldC, BC, GC | (Moll and Slade, 2004) |
| TXN | thioredoxin (trx) | thioredoxin redox system | LC, PaC, CeC, HCC | (Marks, 2006) |
| WEE1 | Wee-1 kinase | cell cycle | NSCLC | (Yoshida *et al.,* 2004) |
| WNT7B | Wnt-7b | signal transduction | BC, BldC | (Huguet *et al.,* 1994; Bui *et al.,* 1998) |

| | | | | |
|---|---|---|---|---|
| Abbreviations: AC, astrocytoma; ALCL, anaplastic large cell lymphoma; AML, acute myeloid leukemia; BC, breast carcinoma; BCL, B-cell lymphoma; BL, Burkitt's lymphoma; BldC, bladder carcinoma; CeC, cervical carcinoma; CLL, chronic lymphoblastic leukemia; CRC, colorectal carcinoma; EC, endometrial carcinoma; EWS, Ewing's sarcoma; G, glioma; GB, glioblastoma; GC, gastric carcinoma; GI, gastrinoma; HB, hepatoblastoma; HCC, hepatocellular carcinoma; HL, Hodgkin lymphoma; L, leukemia; LC, lung carcinoma; M, melanoma; MCL, mantle cell lymphoma; MG, meningioma; ML, myeloid leukemia; MM, multiple myeloma; MT, mesothelioma; NF, neurofibroma; NHL, non-Hodgkin lymphoma; NSCLC, non-small cell lung carcinoma; OC, ovarian carcinoma; OepC, oesophageal carcinoma; OrpC, oropharyngeal carcinoma; OS, osteosarcoma; PaC, pancreatic carcinoma; PapC, papillary carcinoma; PC, prostate carcinoma; PCC, pheochromocytoma; RCC, renal cell carcinoma; RMS, rhabdomyosarcoma; SCCHN, squamous cell carcinoma of the head and neck; Schw, schwannoma; SPRC, sporadic papillary renal carcinoma; TC, thyroid carcinoma; TT, testicular tumor; SCLC, small cell lung cancer. | | | | |

The methods can further comprise one or more of the steps including: (a) obtaining a sample from the patient, (b) isolating nucleic acids from the sample, (c) labeling the nucleic acids isolated from the sample, and (d) hybridizing the labeled nucleic acids to one or more probes. Nucleic acids of the invention include one or more nucleic acid comprising at least one segment having a sequence or complementary sequence of to a nucleic acid representative of one or more of genes or markers in Table 1, 3, 4, and/or 5.

It is contemplated that any method or composition described herein can be implemented with respect to any other method or composition described herein and that different embodiments may be combined. It is specifically contemplated that any methods and compositions discussed herein with respect to miRNA molecules, miRNA, genes, and Certain embodiments of the invention include determining expression of one or more marker, gene, or nucleic acid representative thereof, by using an amplification assay, a hybridization assay, or protein assay, a variety of which are well known to one of ordinary skill in the art. In certain aspects, an amplification assay can be a quantitative amplification assay, such as quantitative RT-PCR or the like. In still further aspects, a hybridization assay can include array hybridization assays or solution hybridization assays. The nucleic acids from a sample may be labeled from the sample and/or hybridizing the labeled nucleic acid to one or more nucleic acid probes. Nucleic acids, mRNA, and/or nucleic acid probes may be coupled to a support. Such supports are well known to those of ordinary skill in the art and include, but are not limited to glass, plastic, metal, or latex. In particular aspects of the invention, the support can be planar or in the form of a bead or other geometric shapes or configurations known in the art. Proteins are typically assayed by immunoblotting, chromatography, or mass spectrometry or other methods known to those of ordinary skill in the art.

The present invention also concerns kits containing compositions of the invention or compositions to implement methods of the invention. In some embodiments, kits can be used to evaluate one or more marker molecules, and/or express one or more miRNA. In certain embodiments, a kit contains, contains at least or contains at most 1, 2, 3, 4, 5, 6, 7, 8, 9, 10, 11, 12, 13, l4, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 100, 150, 200 or more probes, recombinant nucleic acid, or synthetic nucleic acid molecules related to the markers to be assessed or an miRNA to be expressed or modulated, and may include any range or combination derivable therein. Kits may comprise components, which may be individually packaged or placed in a container, such as a tube, bottle, vial, syringe, or other suitable container means. Individual components may also be provided in a kit in concentrated amounts; in some embodiments, a component is provided individually in the same concentration as it would be in a solution with other components. Concentrations of components may be provided as 1x, 2x, 5x, 10x, or 20x or more. Kits for using probes, synthetic nucleic acids, recombinant nucleic acids, or non-synthetic nucleic acids of the invention for therapeutic, prognostic, or diagnostic applications are included as part of the invention. Specifically contemplated are any such molecules corresponding to any miRNA reported to influence biological activity or expression of one or more marker gene or gene pathway described herein. In certain aspects, negative and/or positive controls are included in some kit embodiments. The control molecules can be used to verify transfection efficiency and/or control for transfection-induced changes in cells.

Certain embodiments are directed to a kit for assessment of a pathological condition or the risk of developing a pathological condition in a patient by nucleic acid profiling of a sample comprising, in suitable container means, two or more nucleic acid hybridization or amplification reagents. The kit can comprise reagents for labeling nucleic acids in a sample and/or nucleic acid hybridization reagents. The hybridization reagents typically comprise hybridization probes. Amplification reagents include, but are not limited to amplification primers, reagents, and enzymes.

In some embodiments of the invention, an expression profile is generated by steps that include: (a) labeling nucleic acid in the sample; (b) hybridizing the nucleic acid to a number of probes, or amplifying a number of nucleic acids, and (c) determining and/or quantitating nucleic acid hybridization to the probes or detecting and quantitating amplification products, wherein an expression profile is generated. See U.S. Provisional Patent Application 60/575,743 and the U.S. Provisional Patent Application 60/649,584, and U.S. Patent Application Serial No. 11/141,707 and U.S. Patent Application Serial No. 11/273,640.

Methods of the invention involve diagnosing and/or assessing the prognosis of a patient based on a miRNA and/or a marker nucleic acid expression profile. In certain embodiments, the elevation or reduction in the level of expression of a particular gene or genetic pathway or set of nucleic acids in a cell is correlated with a disease state or pathological condition compared to the expression level of the same in a normal or non-pathologic cell or tissue sample. This correlation allows for diagnostic and/or prognostic methods to be carried out when the expression level of one or more nucleic acid is measured in a biological sample being assessed and then compared to the expression level of a normal or non-pathologic cell or tissue sample. It is specifically contemplated that expression profiles for patients, particularly those suspected of having or having a propensity for a particular disease or condition such as cancer, can be generated by evaluating any of or sets of the miRNAs and/or nucleic acids discussed in this application. The expression profile that is generated from the patient will be one that provides information regarding the particular disease or condition. In many embodiments, the profile is generated using nucleic acid hybridization or amplification, (e.g., array hybridization or RT-PCR). In certain aspects, an expression profile can be used in conjunction with other diagnostic and/or prognostic tests, such as histology, protein profiles in the serum and/or cytogenetic assessment.

The methods can further comprise one or more of the steps including: (a) obtaining a sample from the patient, (b) isolating nucleic acids from the sample, (c) labeling the nucleic acids isolated from the sample, and (d) hybridizing the labeled nucleic acids to one or more probes. Nucleic acids of the invention include one or more nucleic acid comprising at least one segment having a sequence or complementary sequence of to a nucleic acid representative of one or more of genes or markers in Table 1, 3, 4, and/or 5.

It is contemplated that any method or composition described herein can be implemented with respect to any other method or composition described herein and that different embodiments may be combined. It is specifically contemplated that any methods and compositions discussed herein with respect to miRNA molecules, miRNA, genes and nucleic acids representative of genes may be implemented with respect to synthetic nucleic acids. In some embodiments the synthetic nucleic acid is exposed to the proper conditions to allow it to become a processed or mature nucleic acid, such as a miRNA under physiological circumstances. The claims originally filed are contemplated to cover claims that are multiply dependent on any filed claim or combination of filed claims.

Also, any embodiment of the invention involving specific genes (including representative fragments there of), mRNA, or miRNAs by name is contemplated also to cover embodiments involving miRNAs whose sequences are at least 80, 81, 82, 83, 84, 85, 86, 87, 88, 89, 90, 91, 92, 93, 94, 95, 96, 97, 98, 99% identical to the mature sequence of the specified miRNA.

It will be further understood that shorthand notations are employed such that a generic description of a gene or marker thereof, or of a miRNA refers to any of its gene family members (distinguished by a number) or representative fragments thereof, unless otherwise indicated. It is understood by those of skill in the art that a "gene family" refers to a group of genes having the same coding sequence or miRNA coding sequence. Typically, miRNA members of a gene family are identified by a number following the initial designation. For example, miR-16-1 and miR-16-2 are members of the miR-16 gene family and "mir-7" refers to miR-7-1, miR-7-2 and miR-7-3. Moreover, unless otherwise indicated, a shorthand notation refers to related miRNAs (distinguished by a letter). Exceptions to these shorthand notations will be otherwise identified.

Other embodiments of the invention are discussed throughout this application. Any embodiment discussed with respect to one aspect of the invention applies to other aspects of the invention as well and *vice versa.* The embodiments in the Example and Detailed Description section are understood to be embodiments of the invention that are applicable to all aspects of the invention.

The terms "inhibiting," "reducing," or "prevention," or any variation of these terms, when used in the claims and/or the specification includes any measurable decrease or complete inhibition to achieve a desired result.

The use of the word "a" or "an" when used in conjunction with the term "comprising'' in the claims and/or the specification may mean "one," but it is also consistent with the meaning of "one or more," "at least one," and "one or more than one."

Throughout this application, the term "about" is used to indicate that a value includes the standard deviation of error for the device or method being employed to determine the value.

The use of the term "or" in the claims is used to mean "and/or'' unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

### DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

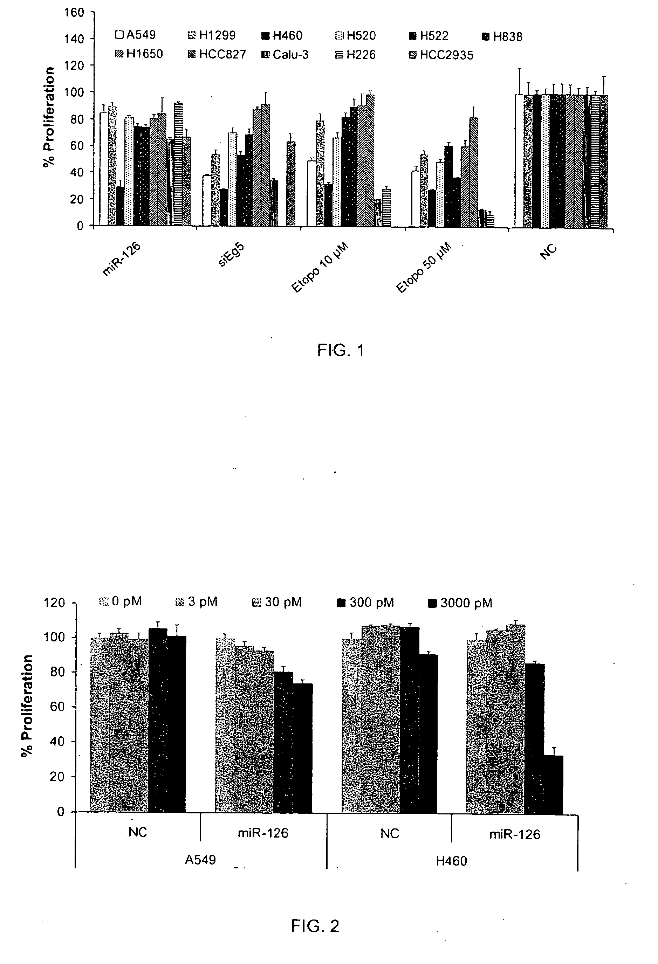

FIG. 1. Percent (%) proliferation of hsa-miR-126 treated cells relative to cells treated with negative control miRNA (100%). Abbreviations: miR-126, hsa-miR-126; siEg5, siRNA against the motor protein kinesin 11 (Eg5); Etopo, etoposide; NC, negative control miRNA. Standard deviations are indicated in the graph.

**FIG. 2****.** Dose dependent inhibition of various cell lines by hsa-miR-126 using Alamar Blue proliferation assays. Cell proliferation is reported as % proliferation relative to % proliferation of mock-transfected cells (0 pM = 100% proliferation). Standard deviations are indicated in the graph. Abbreviations: miR-126, hsa-miR-126; NC, negative control miRNA.

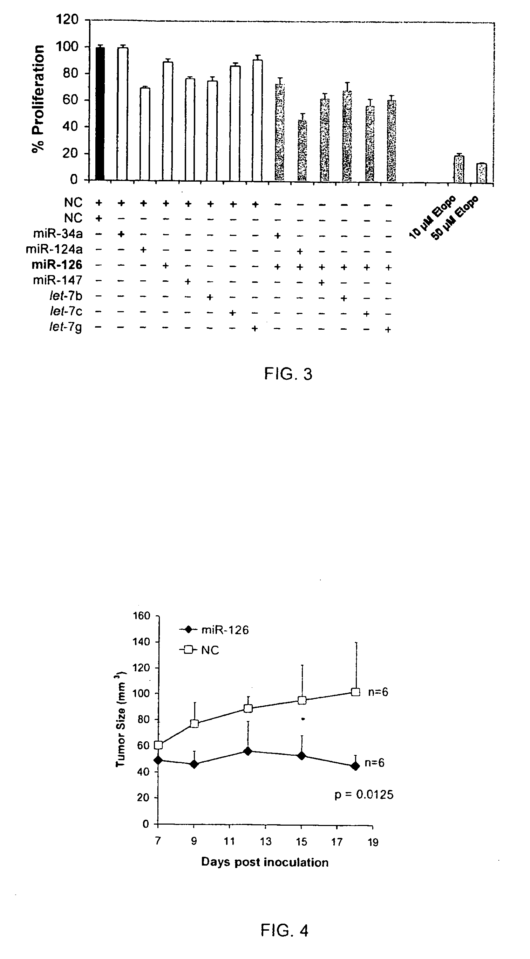

**FIG. 3****.** Percent (%) proliferation of H460 lung cancer cells following administration of various combinations of microRNAs. A positive sign under each bar in the graph indicates that the miRNA was present in the administered combination. Standard deviations are shown in the graph. Abbreviations: miR-34a, hsa-miR-34a; miR-124a, hsa-miR-124a; miR-126, hsa-miR-126; miR-147, hsa-miR-147; let-7b, hsa-let-7b; let-7c, hsa-let-7c; let-7g, hsa-let-7g; Etopo, etoposide; NC, negative control miRNA.

**FIG. 4****.** Average tumor volumes in six (n=6) mice harboring xenografts of A549 lung cancer cells treated with hsa-miR-126 (black diamonds) or with a negative control miRNA (NC, white squares). Standard deviations are shown in the graph. The p value, indicating statistical significance, is shown for values obtained on day 18 (p = 0.0125). Abbreviation: miR-126, hsa-miR-126; NC, negative control miRNA.

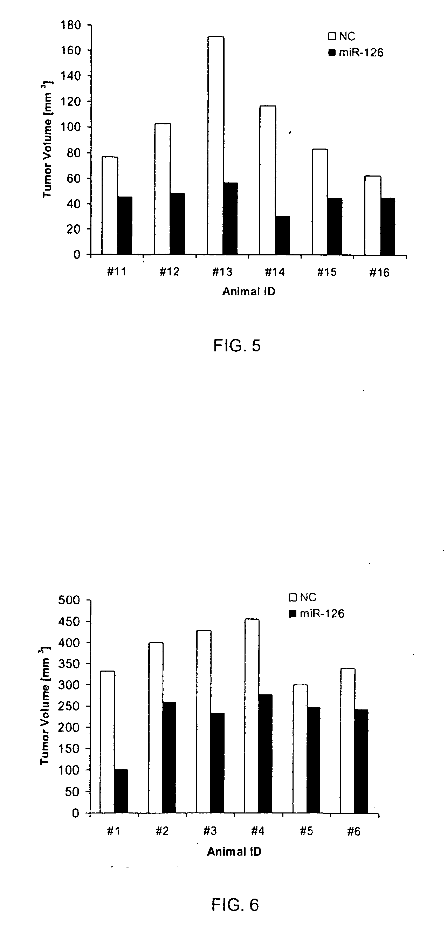

**FIG. 5****.** Volumes of individual A549 tumors on day 18 post inoculation. White bars represent volumes from tumors treated with negative control miRNA (NC); black bars represent volumes from A549 tumors that received hsa-miR-126. Tumor volumes are expressed in mm³. Abbreviations: miR-126, hsa-miR-126. ID, identification.

**FIG. 6****.** Volumes of individual H460 tumors on day 7 post inoculation. White bars represent volumes from tumors treated with negative control miRNA (NC); black bars represent volumes from H460 tumors that received hsa-miR-126. Tumor volumes are expressed in mm³. Abbreviations: miR-126, hsa-miR-126. ID, identification.

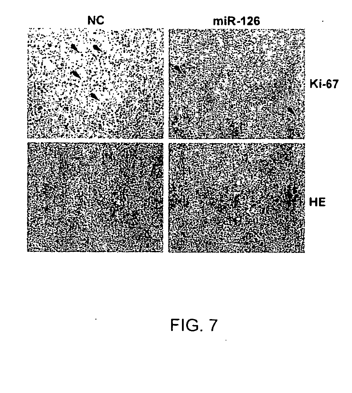

**FIG. 7****.** Histology of tumors that developed from A549 lung cancer cells treated with negative control miRNA (left, top and bottom) or hsa-miR-126 (right, top and bottom). The bottom photos show tumors stained with hematoxylin and eosin (HE); the top photos show an immunohistochemistry analysis using antibodies against the Ki-67 antigen (dark spotted areas, exemplarily denoted by arrowheads). Ki-67 is a nuclear marker indicative for readily proliferating cells. Abbreviation: miR-126, hsa-miR-126; NC, negative control miRNA.

### DETAILED DESCRIPTION OF THE INVENTION

The present invention is defined in the claims. The following description is understood to relate to the invention within the limitations of the claims.

Disclosed are compositions and methods relating to the identification and characterization of genes and biological pathways related to these genes as represented by the expression of the identified genes, as well as use of miRNAs related to such, for therapeutic, prognostic, and diagnostic applications, particularly those methods and compositions related to assessing and/or identifying pathological conditions directly or indirectly related to miR-126 expression or the aberrant expression thereof.

Methods for the assessment, analysis, and/or therapy of a cell or subject are disclosed where certain genes have a reduced or increased expression (relative to normal) as a result of an increased or decreased expression of any one or a combination of miR-126 family members (including, but not limited to SEQ ID NO:1 to SEQ ID NO:24) and/or genes with an increased expression (relative to normal) as a result of an increased or decreased expression thereof. The expression profile and/or response to miR-126 expression or inhibition may be indicative of a disease or an individual with a pathological condition, e.g., cancer.

Prognostic assays featuring any one or combination of the miRNAs listed or the markers listed (including nucleic acids representative thereof) could be used in assessment of a patient to determine what if any treatment regimen is justified. As with the diagnostic assays mentioned above, the absolute values that define low expression will depend on the platform used to measure the miRNA(s). The same methods described for the diagnostic assays could be used for a prognostic assays.

### I. THERAPEUTIC METHODS

Embodiments of the invention concern nucleic acids that perform the activities of or inhibit endogenous miRNAs when introduced into cells. In certain aspects, nucleic acids are synthetic or non-synthetic miRNA. Sequence-specific miRNA inhibitors can be used to inhibit sequentially or in combination the activities of one or more endogenous miRNAs in cells, as well those genes and associated pathways modulated by the endogenous miRNA.

The present invention concerns, in some embodiments, short nucleic acid molecules that function as miRNAs or as inhibitors of miRNA in a cell. The term "short" refers to a length of a single polynucleotide that is 15, 16, 17, 18, 19, 20, 21, 22, 23, 24, 25, 50, 100, or 150 nucleotides or fewer, including all integers or ranges range derivable there between. The nucleic acid molecules are typically synthetic. The term "synthetic" refers to a nucleic acid molecule that is isolated and not produced naturally in a cell. In certain aspects the sequence (the entire sequence) and/or chemical structure deviates from a naturally-occurring nucleic acid molecule, such as an endogenous precursor miRNA or miRNA molecule or complement thereof. While in some embodiments, nucleic acids of the invention do not have an entire sequence that is identical or complementary to a sequence of a naturally-occurring nucleic acid, such molecules may encompass all or part of a naturally-occurring sequence or a complement thereof.. It is contemplated, however, that a synthetic nucleic acid administered to a cell may subsequently be modified or altered in the cell such that its structure or sequence is the same as non-synthetic or naturally occurring nucleic acid, such as a mature miRNA sequence. For example, a synthetic nucleic acid may have a sequence that differs from the sequence of a precursor miRNA, but that sequence may be altered once in a cell to be the same as an endogenous, processed miRNA or an inhibitor thereof.. The term "isolated" means that the nucleic acid molecules of the invention are initially separated from different (in terms of sequence or structure) and unwanted nucleic acid molecules such that a population of isolated nucleic acids is at least about 90% homogenous, and may be at least about 95, 96, 97, 98, 99, or 100% homogenous with respect to other polynucleotide molecules. In many embodiments of the invention, a nucleic acid is isolated by virtue of it having been synthesized *in vitro* separate from endogenous nucleic acids in a cell. It will be understood, however, that isolated nucleic acids may be subsequently mixed or pooled together. In certain aspects, synthetic miRNA of the invention are RNA or RNA analogs. miRNA inhibitors may be DNA or RNA, or analogs thereof. miRNA and miRNA inhibitors of the invention are collectively referred to as "synthetic nucleic acids."

In some embodiments, there is a miRNA or a synthetic miRNA having a length of between 17 and 130 residues. The present invention concerns miRNA or synthetic miRNA molecules that are, are at least, or are at most 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 140, 145, 150, 160, 170, 180, 190, 200 or more residues in length, including any integer or any range there between.

In certain embodiments, synthetic miRNA have (a) a "miRNA region" whose sequence or binding region from 5' to 3' is identical or complementary to all or a segment of a mature miRNA sequence, and (b) a "complementary region" whose sequence from 5' to 3' is between 60% and 100% complementary to the miRNA sequence in (a). In certain embodiments, these synthetic miRNA are also isolated, as defined above. The term "miRNA region" refers to a region on the synthetic miRNA that is at least 75, 80, 85, 90, 95, or 100% identical, including all integers there between, to the entire sequence of a mature, naturally occurring miRNA sequence or a complement thereof. In certain embodiments, the miRNA region is or is at least 90, 91, 92, 93, 94, 95, 96, 97, 98, 99, 99.1, 99.2, 99.3, 99.4, 99.5, 99.6, 99.7, 99.8, 99.9 or 100% identical to the sequence of a naturally-occurring miRNA or complement thereof.

The term "complementary region" or "complement" refers to a region of a nucleic acid or mimetic that is or is at least 60% complementary to the mature, naturally occurring miRNA sequence. The complementary region is or is at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 99.1, 99.2, 99.3, 99.4, 99.5, 99.6, 99.7, 99.8, 99.9 or 100% complementary, or any range derivable therein. With single polynucleotide sequences, there may be a hairpin loop structure as a result of chemical bonding between the miRNA region and the complementary region. In other embodiments, the complementary region is on a different nucleic acid molecule than the miRNA region, in which case the complementary region is on the complementary strand and the miRNA region is on the active strand.

In other embodiments of the invention, there are synthetic nucleic acids that are miRNA inhibitors. A miRNA inhibitor is between about 17 to 25 nucleotides in length and comprises a 5' to 3' sequence that is at least 90% complementary to the 5' to 3' sequence of a mature miRNA. In certain embodiments, a miRNA inhibitor molecule is 17, 18, 19, 20, 21, 22, 23, 24, or 25 nucleotides in length, or any range derivable therein. Moreover, an miRNA inhibitor may have a sequence (from 5' to 3') that is or is at least 70, 75, 80, 85, 90, 91, 92, 93, 94, 95, 96, 97, 98, 99, 99.1, 99.2, 99.3, 99.4, 99.5, 99.6, 99.7, 99.8, 99.9 or 100% complementary, or any range derivable therein, to the 5' to 3' sequence of a mature miRNA, particularly a mature, naturally occurring miRNA. One of skill in the art could use a portion of the miRNA sequence that is complementary to the sequence of a mature miRNA as the sequence for a miRNA inhibitor. Moreover, that portion of the nucleic acid sequence can be altered so that it is still comprises the appropriate percentage of complementarity to the sequence of a mature miRNA.

In some embodiments, of the invention, a synthetic miRNA or inhibitor contains one or more design element(s). These design elements include, but are not limited to: (i) a replacement group for the phosphate or hydroxyl of the nucleotide at the 5' terminus of the complementary region; (ii) one or more sugar modifications in the first or last 1 to 6 residues of the complementary region; or, (iii) noncomplementarity between one or more nucleotides in the last 1 to 5 residues at the 3' end of the complementary region and the corresponding nucleotides of the miRNA region. A variety of design modifications are known in the art, see below.

In certain embodiments, a synthetic miRNA has a nucleotide at its 5' end of the complementary region in which the phosphate and/or hydroxyl group has been replaced with another chemical group (referred to as the "replacement design"). In some cases, the phosphate group is replaced, while in others, the hydroxyl group has been replaced. In particular embodiments, the replacement group is biotin, an amine group, a lower alkylamine group, an acetyl group, 2'O-Me (2'oxygen-methyl), DMTO (4,4'-dimethoxytrityl with oxygen), fluoroscein, a thiol, or acridine, though other replacement groups are well known to those of skill in the art and can be used as well. This design element can also be used with a miRNA inhibitor.

Additional embodiments concern a synthetic miRNA having one or more sugar modifications in the first or last 1 to 6 residues of the complementary region (referred to as the "sugar replacement design"). In certain cases, there is one or more sugar modifications in the first 1, 2, 3, 4, 5, 6 or more residues of the complementary region, or any range derivable therein. In additional cases, there are one or more sugar modifications in the last 1, 2, 3, 4, 5, 6 or more residues of the complementary region, or any range derivable therein, have a sugar modification. It will be understood that the terms "first" and "last" are with respect to the order of residues from the 5' end to the 3' end of the region. In particular embodiments, the sugar modification is a 2'O-Me modification. In further embodiments, there are one or more sugar modifications in the first or last 2 to 4 residues of the complementary region or the first or last 4 to 6 residues of the complementary region. This design element can also be used with a miRNA inhibitor. Thus, a miRNA inhibitor can have this design element and/or a replacement group on the nucleotide at the 5' terminus, as discussed above.

In other embodiments of the invention, there is a synthetic miRNA or inhibitor in which one or more nucleotides in the last 1 to 5 residues at the 3' end of the complementary region are not complementary to the corresponding nucleotides of the miRNA region ("noncomplementarity") (referred to as the "noncomplementarity design"). The noncomplementarity may be in the last 1, 2, 3, 4, and/or 5 residues of the complementary miRNA. In certain embodiments, there is noncomplementarity with at least 2 nucleotides in the complementary region.

It is contemplated that synthetic miRNA of the invention have one or more of the replacement, sugar modification, or noncomplementarity designs. In certain cases, synthetic RNA molecules have two of them, while in others these molecules have all three designs in place.

The miRNA region and the complementary region may be on the same or separate polynucleotides. In cases in which they are contained on or in the same polynucleotide, the miRNA molecule will be considered a single polynucleotide. In embodiments in which the different regions are on separate polynucleotides, the synthetic miRNA will be considered to be comprised of two polynucleotides.

When the RNA molecule is a single polynucleotide, there can be a linker region between the miRNA region and the complementary region. In some embodiments, the single polynucleotide is capable of forming a hairpin loop structure as a result of bonding between the miRNA region and the complementary region. The linker constitutes the hairpin loop. It is contemplated that in some embodiments, the linker region is, is at least, or is at most 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 residues in length, or any range derivable therein. In certain embodiments, the linker is between 3 and 30 residues (inclusive) in length.

In addition to having a miRNA or inhibitor region and a complementary region, there may be flanking sequences as well at either the 5' or 3' end of the region. In some embodiments, there is or is at least 1, 2, 3, 4, 5, 6, 7, 8, 9, 10 nucleotides or more, or any range derivable therein, flanking one or both sides of these regions.

Methods of the invention include reducing or eliminating activity of one or more miRNAs in a cell comprising introducing into a cell a miRNA inhibitor (which may be described generally herein as an miRNA, so that a description of miRNA, where appropriate, also will refer to a miRNA inhibitor); or supplying or enhancing the activity of one or more miRNAs in a cell. The present invention also concerns inducing certain cellular characteristics by providing to a cell a particular nucleic acid, such as a specific synthetic miRNA molecule or a synthetic miRNA inhibitor molecule. However, in methods of the invention, the miRNA molecule or miRNA inhibitor need not be synthetic. They may have a sequence that is identical to a naturally occurring miRNA or they may not have any design modifications. In certain embodiments, the miRNA molecule and/or the miRNA inhibitor are synthetic, as discussed above.

The particular nucleic acid molecule provided to the cell is understood to correspond to a particular miRNA in the cell, and thus, the miRNA in the cell is referred to as the "corresponding miRNA." In situations in which a named miRNA molecule is introduced into a cell, the corresponding miRNA will be understood to be the induced or inhibited miRNA or induced or inhibited miRNA function. It is contemplated, however, that the miRNA molecule introduced into a cell is not a mature miRNA but is capable of becoming or functioning as a mature miRNA under the appropriate physiological conditions. In cases in which a particular corresponding miRNA is being inhibited by a miRNA inhibitor, the particular miRNA will be referred to as the "targeted miRNA." It is contemplated that multiple corresponding miRNAs may be involved. In particular embodiments, more than one miRNA molecule is introduced into a cell. Moreover, in other embodiments, more than one miRNA inhibitor is introduced into a cell. Furthermore, a combination of miRNA molecule(s) and miRNA inhibitor(s) may be introduced into a cell. The inventors contemplate that a combination of miRNA may act at one or more points in cellular pathways of cells with aberrant phenotypes and that such combination may have increased efficacy on the target cell while not adversely effecting normal cells. Thus, a combination of miRNA may have a minimal adverse effect on a subject or patient while supplying a sufficient therapeutic effect, such as amelioration of a condition, growth inhibition of a cell, death of a targeted cell, alteration of cell phenotype or physiology, slowing of cellular growth, sensitization to a second therapy, sensitization to a particular therapy, and the like.

Methods include identifying a cell or patient in need of inducing those cellular characteristics. Also, it will be understood that an amount of a synthetic nucleic acid that is provided to a cell or organism is an "effective amount," which refers to an amount needed (or a sufficient amount) to achieve a desired goal, such as inducing a particular cellular characteristic(s).

In certain embodiments of the methods include providing or introducing to a cell a nucleic acid molecule corresponding to a mature miRNA in the cell in an amount effective to achieve a desired physiological result.

Moreover, methods can involve providing synthetic or nonsynthetic miRNA molecules. It is contemplated that in these embodiments, that the methods may or may not be limited to providing only one or more synthetic miRNA molecules or only one or more nonsynthetic miRNA molecules. Thus, in certain embodiments, methods may involve providing both synthetic and nonsynthetic miRNA molecules. In this situation, a cell or cells are most likely provided a synthetic miRNA molecule corresponding to a particular miRNA and a nonsynthetic miRNA molecule corresponding to a different miRNA. Furthermore, any method articulated using a list of miRNAs using Markush group language may be articulated without the Markush group language and a disjunctive article (*i.e.*, or) instead, and vice versa.

In some embodiments, there is a method for reducing or inhibiting cell proliferation comprising introducing into or providing to the cell an effective amount of (i) a miRNA inhibitor molecule or (ii) a synthetic or nonsynthetic miRNA molecule that corresponds to a miRNA sequence. In certain embodiments the methods involves introducing into the cell an effective amount of (i) an miRNA inhibitor molecule having a 5' to 3' sequence that is at least 90% complementary to the 5' to 3' sequence of one or more mature miRNA.

Certain embodiments of the invention include methods of treating a pathologic condition, in particular cancer, e.g., lung or liver cancer. In one aspect, the method comprises contacting a target cell with one or more nucleic acid, synthetic miRNA, or miRNA comprising at least one nucleic acid segment having all or a portion of a miRNA sequence. The segment may be 5, 6, 7, 8, 9, 10, 11, 12, 13, 14, 15, 16, 17, 18, 19, 20, 21, 22, 23, 24, 25, 30 or more nucleotides or nucleotide analog, including all integers there between. An aspect of the invention includes the modulation of gene expression, miRNA expression or function or mRNA expression or function within a target cell, such as a cancer cell.

Typically, an endogenous gene, miRNA or mRNA is modulated in the cell. In particular embodiments, the nucleic acid sequence comprises at least one segment that is at least 70, 75, 80, 85, 90, 95, or 100% identical in nucleic acid sequence to one or more miRNA or gene sequence. Modulation of the expression or processing of an endogenous gene, miRNA, or mRNA can be through modulation of the processing of a mRNA, such processing including transcription, transportation and/or translation with in a cell. Modulation may also be effected by the inhibition or enhancement of miRNA activity with a cell, tissue, or organ. Such processing may affect the expression of an encoded product or the stability of the mRNA. In still other embodiments, a nucleic acid sequence can comprise a modified nucleic acid sequence. In certain aspects, one or more miRNA sequence may include or comprise a modified nucleobase or nucleic acid sequence.

It will be understood in methods of the invention that a cell or other biological matter such as an organism (including patients) can be provided a miRNA or miRNA molecule corresponding to a particular miRNA by administering to the cell or organism a nucleic acid molecule that functions as the corresponding miRNA once inside the cell. The form of the molecule provided to the cell may not be the form that acts a miRNA once inside the cell. Thus, it is contemplated that in some embodiments, a synthetic miRNA or a nonsynthetic miRNA is provided a synthetic miRNA or a nonsynthetic miRNA, such as one that becomes processed into a mature and active miRNA once it has access to the cell's miRNA processing machinery. In certain embodiments, it is specifically contemplated that the miRNA molecule provided to the biological matter is not a mature miRNA molecule but a nucleic acid molecule that can be processed into the mature miRNA once it is accessible to miRNA processing machinery. The term "nonsynthetic" in the context of miRNA means that the miRNA is not "synthetic," as defined herein. Furthermore, it is contemplated that in embodiments of the invention that concern the use of synthetic miRNAs, the use of corresponding nonsynthetic miRNAs is also considered an aspect of the invention, and vice versa. It will be understand that the term "providing" an agent is used to include "administering" the agent to a patient.

In certain embodiments, methods also include targeting a miRNA to modulate in a cell or organism. The term "targeting a miRNA to modulate" means a nucleic acid of the invention will be employed so as to modulate the selected miRNA. In some embodiments the modulation is achieved with a synthetic or non-synthetic miRNA that corresponds to the targeted miRNA, which effectively provides the targeted miRNA to the cell or organism (positive modulation). In other embodiments, the modulation is achieved with a miRNA inhibitor, which effectively inhibits the targeted miRNA in the cell or organism (negative modulation).

In some embodiments, the miRNA targeted to be modulated is a miRNA that affects a disease, condition, or pathway. In certain embodiments, the miRNA is targeted because a treatment can be provided by negative modulation of the targeted miRNA. In other embodiments, the miRNA is targeted because a treatment can be provided by positive modulation of the targeted miRNA or its targets.

In certain methods of the invention, there is a further step of administering the selected miRNA modulator to a cell, tissue, organ, or organism (collectively "biological matter") in need of treatment related to modulation of the targeted miRNA or in need of the physiological or biological results discussed herein (such as with respect to a particular cellular pathway or result like decrease in cell viability). Consequently, in some methods of the invention there is a step of identifying a patient in need of treatment that can be provided by the miRNA modulator(s). It is contemplated that an effective amount of a miRNA modulator can be administered in some embodiments. In particular embodiments, there is a therapeutic benefit conferred on the biological matter, where a "therapeutic benefit" refers to an improvement in the one or more conditions or symptoms associated with a disease or condition or an improvement in the prognosis, duration, or status with respect to the disease. It is contemplated that a therapeutic benefit includes, but is not limited to, a decrease in pain, a decrease in morbidity, a decrease in a symptom. For example, with respect to cancer, it is contemplated that a therapeutic benefit can be inhibition of tumor growth, prevention of metastasis, reduction in number of metastases, inhibition of cancer cell proliferation, induction of cell death in cancer cells, inhibition of angiogenesis near cancer cells, induction of apoptosis of cancer cells, reduction in pain, reduction in risk of recurrence, induction of chemo- or radiosensitivity in cancer cells, prolongation of life, and/or delay of death directly or indirectly related to cancer.

Furthermore, it is contemplated that the miRNA compositions may be provided as part of a therapy to a patient, in conjunction with traditional therapies or preventative agents. Moreover, it is contemplated that any method discussed in the context of therapy may be applied as preventatively, particularly in a patient identified to be potentially in need of the therapy or at risk of the condition or disease for which a therapy is needed.

In addition, methods of the invention concern employing one or more nucleic acids corresponding to a miRNA and a therapeutic drug. The nucleic acid can enhance the effect or efficacy of the drug, reduce any side effects or toxicity, modify its bioavailability, and/or decrease the dosage or frequency needed. In certain embodiments, the therapeutic drug is a cancer therapeutic. Consequently, in some embodiments, there is a method of treating cancer in a patient comprising administering to the patient the cancer therapeutic and an effective amount of at least one miRNA molecule that improves the efficacy of the cancer therapeutic or protects non-cancer cells. Cancer therapies also include a variety of combination therapies with both chemical and radiation based treatments. Combination chemotherapies include but are not limited to, for example, 5-fluorouracil, alemtuzumab, amrubicin, bevacizumab, bleomycin, bortezomib, busulfan, camptothecin, capecitabine, cisplatin (CDDP), carboplatin, cetuximab, chlorambucil, cisplatin (CDDP), EGFR inhibitors (gefitinib and cetuximab), procarbazine, mechlorethamine, cyclophosphamide, camptothecin, COX-2 inhibitors (e.g., celecoxib), cyclophosphamide, cytarabine, ) ifosfamide, melphalan, chlorambucil, busulfan, nitrosurea, dactinomycin, dasatinib, daunorubicin, dexamethasone, docetaxel, doxorubicin (adriamycin), EGFR inhibitors (gefitinib and cetuximab), erlotinib, estrogen receptor binding agents, bleomycin, plicomycin, mitomycin, etoposide (VP16), everolimus, tamoxifen, raloxifene, estrogen receptor binding agents, taxol, taxotere, gemcitabien, navelbine, farnesyl-protein transferase inhibitors, gefitinib, gemcitabine, gemtuzumab, ibritumomab, ifosfamide, imatinib mesylate, larotaxel, lapatinib, lonafarnib, mechlorethamine, melphalan, transplatinum, 5-fluorouracil, vincristin, vinblastin and methotrexate, mitomycin, navelbine, nitrosurea, nocodazole, oxaliplatin, paclitaxel, plicomycin, procarbazine, raloxifene, rituximab, sirolimus, sorafenib, sunitinib, tamoxifen, taxol, taxotere, temsirolimus, tipifarnib, tositumomab, transplatinum, trastuzumab, vinblastin, vincristin, or vinorelbine or any analog or derivative variant of the foregoing.

Generally, inhibitors of miRNAs can be given to decrease the activity of an endogenous miRNA. For example, inhibitors of miRNA molecules that increase cell proliferation can be provided to cells to increase proliferation or inhibitors of such molecules can be provided to cells to decrease cell proliferation. The present invention contemplates these embodiments in the context of the different physiological effects observed with the different miRNA molecules and miRNA inhibitors disclosed herein. These include, but are not limited to, the following physiological effects: increase and decreasing cell proliferation, increasing or decreasing apoptosis, increasing transformation, increasing or decreasing cell viability, activating or inhibiting a kinase (*e.g.*, Erk)ERK, activating/inducing or inhibiting hTert, inhibit stimulation of growth promoting pathway (e.g., Stat 3 signaling), reduce or increase viable cell number, and increase or decrease number of cells at a particular phase of the cell cycle. Methods of the invention are generally contemplated to include providing or introducing one or more different nucleic acid molecules corresponding to one or more different miRNA molecules. It is contemplated that the following, at least the following, or at most the following number of different nucleic acid or miRNA molecules may be provided or introduced: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or any range derivable therein. This also applies to the number of different miRNA molecules that can be provided or introduced into a cell.

### II. PHARMACEUTICAL FORMULATIONS AND DELIVERY

Methods of the present invention include the delivery of an effective amount of a miRNA or an expression construct encoding the same. An "effective amount" of the pharmaceutical composition, generally, is defined as that amount sufficient to detectably and repeatedly to achieve the stated desired result, for example, to ameliorate, reduce, minimize or limit the extent of the disease or its symptoms. Other more rigorous definitions may apply, including elimination, eradication or cure of disease.

### A. Administration

In certain embodiments, it is desired to kill cells, inhibit cell growth, inhibit metastasis, decrease tumor or tissue size, and/or reverse or reduce the malignant or disease phenotype of cells. The routes of administration will vary, naturally, with the location and nature of the lesion or site to be targeted, and include, *e.g.*, intradermal, subcutaneous, regional, parenteral, intravenous, intramuscular, intranasal, systemic, and oral administration and formulation. Direct injection, intratumoral injection, or injection into tumor vasculature is specifically contemplated for discrete, solid, accessible tumors, or other accessible target areas. Local, regional, or systemic administration also may be appropriate. For tumors of >4 cm, the volume to be administered will be about 4-10 ml (preferably 10 ml), while for tumors of <4 cm, a volume of about 1-3 ml will be used (preferably 3 ml).

Multiple injections delivered as a single dose comprise about 0.1 to about 0.5 ml volumes. Compositions of the invention may be administered in multiple injections to a tumor or a targeted site. In certain aspects, injections may be spaced at approximately 1 cm intervals.

In the case of surgical intervention, the present invention may be used preoperatively, to render an inoperable tumor subject to resection. Alternatively, the present invention may be used at the time of surgery, and/or thereafter, to treat residual or metastatic disease. For example, a resected tumor bed may be injected or perfused with a formulation comprising a miRNA or combinations thereof. Administration may be continued post-resection, for example, by leaving a catheter implanted at the site of the surgery. Periodic post-surgical treatment also is envisioned. Continuous perfusion of an expression construct or a viral construct also is contemplated.

Continuous administration also may be applied where appropriate, for example, where a tumor or other undesired affected area is excised and the tumor bed or targeted site is treated to eliminate residual, microscopic disease. Delivery via syringe or catherization is contemplated. Such continuous perfusion may take place for a period from about 1-2 hours, to about 2-6 hours, to about 6-12 hours, to about 12-24 hours, to about 1-2 days, to about 1-2 wk or longer following the initiation of treatment. Generally, the dose of the therapeutic composition via continuous perfusion will be equivalent to that given by a single or multiple injections, adjusted over a period of time during which the perfusion occurs.

Treatment regimens may vary as well and often depend on tumor type, tumor location, immune condition, target site, disease progression and health and age of the patient. Certain tumor types will require more aggressive treatment. The clinician will be best suited to make such decisions based on the known efficacy and toxicity (if any) of the therapeutic formulations.

In certain embodiments, the tumor or affected area being treated may not, at least initially, be resectable. Treatments with compositions of the invention may increase the resectability of the tumor due to shrinkage at the margins or by elimination of certain particularly invasive portions. Following treatments, resection may be possible. Additional treatments subsequent to resection may serve to eliminate microscopic residual disease at the tumor or targeted site.

Treatments may include various "unit doses." A unit dose is defined as containing a predetermined quantity of a therapeutic composition(s). The quantity to be administered, and the particular route and formulation, are within the skill of those in the clinical arts. A unit dose need not be administered as a single injection but may comprise continuous infusion over a set period of time. With respect to a viral component of the present invention, a unit dose may conveniently be described in terms of µg or mg of miRNA or miRNA mimetic. Alternatively, the amount specified may be the amount administered as the average daily, average weekly, or average monthly dose.

miRNA can be administered to the patient in a dose or doses of about or of at least about 0.5, 1, 5, 10, 15, 20, 25, 30, 35, 40, 45, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 µg or mg, or more, or any range derivable therein. Alternatively, the amount specified may be the amount administered as the average daily, average weekly, or average monthly dose, or it may be expressed in terms of mg/kg, where kg refers to the weight of the patient and the mg is specified above. In other embodiments, the amount specified is any number discussed above but expressed as mg/m² (with respect to tumor size or patient surface area).

### B. Injectable Compositions and Formulations

In some embodiments, the method for the delivery of a miRNA or an expression construct encoding such or combinations thereof is via systemic administration. However, the pharmaceutical compositions disclosed herein may also be administered parenterally, subcutaneously, directly, intratracheally, intravenously, intradermally, intramuscularly, or even intraperitoneally as described in U.S. Patents 5,543,158; 5,641,515 and 5,399,363.

Injection of nucleic acids may be delivered by syringe or any other method used for injection of solution, as long as the nucleic acid and any associated components can pass through the particular gauge of needle required for injection. A syringe system has also been described for use in gene therapy that permits multiple injections of predetermined quantities of a solution precisely at any depth (U.S. Patent 5,846,225).

Solutions of the active compounds as free base or pharmacologically acceptable salts may be prepared in water suitably mixed with a surfactant, such as hydroxypropylcellulose. Dispersions may also be prepared in glycerol, liquid polyethylene glycols, mixtures thereof, and in oils. Under ordinary conditions of storage and use, these preparations contain a preservative to prevent the growth of microorganisms. The pharmaceutical forms suitable for injectable use include sterile aqueous solutions or dispersions and sterile powders for the extemporaneous preparation of sterile injectable solutions or dispersions (U.S. Patent 5,466,468. In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

In certain formulations, a water-based formulation is employed while in others, it may be lipid-based. In particular embodiments of the invention, a composition comprising a tumor suppressor protein or a nucleic acid encoding the same is in a water-based formulation. In other embodiments, the formulation is lipid based.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous, intratumoral, intralesional, and intraperitoneal administration. In this connection, sterile aqueous media which can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety, and purity standards as required by FDA Office of Biologics standards.

As used herein, a "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The phrase "pharmaceutically acceptable" refers to molecular entities and compositions that do not produce an allergic or similar untoward reaction when administered to a human.

The nucleic acid(s) are administered in a manner compatible with the dosage formulation, and in such amount as will be therapeutically effective. The quantity to be administered depends on the subject to be treated, including, e.g., the aggressiveness of the disease or cancer, the size of any tumor(s) or lesions, the previous or other courses of treatment. Precise amounts of active ingredient required to be administered depend on the judgment of the practitioner. Suitable regimes for initial administration and subsequent administration are also variable, but are typified by an initial administration followed by other administrations. Such administration may be systemic, as a single dose, continuous over a period of time spanning 10, 20, 30, 40, 50, 60 minutes, and/or 1, 2, 3, 4, 5, 6, 7, 8, 9, 10, 11, 12, 13, 14, 15, 16, 17, 18, 19, 20, 21, 22, 23, 24 or more hours, and/or 1, 2, 3, 4, 5, 6, 7, days or more. Moreover, administration may be through a time release or sustained release mechanism, implemented by formulation and/or mode of administration.

### C. Combination Treatments

In certain embodiments, the compositions and methods of the present invention involve a miRNA, or expression construct encoding such. These miRNA compositions can be used in combination with a second therapy to enhance the effect of the miRNA therapy, or increase the therapeutic effect of another therapy being employed. These compositions would be provided in a combined amount effective to achieve the desired effect, such as the killing of a cancer cell and/or the inhibition of cellular hyperproliferation. This process may involve contacting the cells with the miRNA or second therapy at the same or different time. This may be achieved by contacting the cell with one or more compositions or pharmacological formulation that includes or more of the agents, or by contacting the cell with two or more distinct compositions or formulations, wherein one composition provides (1) miRNA; and/or (2) a second therapy. A second composition or method may be administered that includes a chemotherapy, radiotherapy, surgical therapy, immunotherapy, or gene therapy.

It is contemplated that one may provide a patient with the miRNA therapy and the second therapy within about 12-24 h of each other and, more preferably, within about 6-12 h of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

In certain embodiments, a course of treatment will last 1, 2, 3, 4, S, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 days or more. It is contemplated that one agent may be given on day 1, 2, 3, 4, 5, 6, 7, 8, 9, 10, 11, 12, 13, 14, 15, 16, l7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, and/or 90, any combination thereof, and another agent is given on day 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, and/or 90, or any combination thereof. Within a single day (24-hour period), the patient may be given one or multiple administrations of the agent(s). Moreover, after a course of treatment, it is contemplated that there is a period of time at which no treatment is administered. This time period may last 1, 2, 3, 4, 5, 6, 7 days, and/or 1, 2, 3, 4, 5 weeks, and/or 1, 2, 3, 4, 5, 6, 7, 8, 9, 10, 11, 12 months or more, depending on the condition of the patient, such as their prognosis, strength, health, etc.

Various combinations may be employed, for example miRNA therapy is "A" and a second therapy is "B":

A/B/A B/A/B BB/A A/A/B AB/B B/A/A A/B/B/B B/AB/B

B/B/B/A B/B/A/B A/ABB A/B/A/B A/B/B/A B/B/A/A

B/A/B/A B/A/A/B A/A/A/B B/A/A/A AB/A/A A/A/B/A

Administration of any compound or therapy of the present invention to a patient will follow general protocols for the administration of such compounds, taking into account the toxicity, if any, of the vector or any protein or other agent. Therefore, in some embodiments there is a step of monitoring toxicity that is attributable to combination therapy. It is expected that the treatment cycles would be repeated as necessary. It also is contemplated that various standard therapies, as well as surgical intervention, may be applied in combination with the described therapy.

In specific aspects, it is contemplated that a second therapy, such as chemotherapy, radiotherapy, immunotherapy, surgical therapy or other gene therapy, is employed in combination with the miRNA therapy, as described herein.

### 1. Chemotherapy

A wide variety of chemotherapeutic agents may be used in accordance with the present invention. The term "chemotherapy" refers to the use of drugs to treat cancer. A "chemotherapeutic agent" is used to connote a compound or composition that is administered in the treatment of cancer. These agents or drugs are categorized by their mode of activity within a cell, for example, whether and at what stage they affect the cell cycle. Alternatively, an agent may be characterized based on its ability to directly cross-link DNA, to intercalate into DNA, or to induce chromosomal and mitotic aberrations by affecting nucleic acid synthesis. Most chemotherapeutic agents fall into the following categories: alkylating agents, antimetabolites, antitumor antibiotics, mitotic inhibitors, and nitrosoureas.

### a. Alkylating agents

Alkylating agents are drugs that directly interact with genomic DNA to prevent the cancer cell from proliferating. This category of chemotherapeutic drugs represents agents that affect all phases of the cell cycle, that is, they are not phase-specific. Alkylating agents can be implemented to treat chronic leukemia, non-Hodgkin's lymphoma, Hodgkin's disease, multiple myeloma, and particular cancers of the breast, lung, and ovary. They include: busulfan, chlorambucil, cisplatin, cyclophosphamide (cytoxan), dacarbazine, ifosfamide, mechlorethamine (mustargen), and melphalan. Troglitazaone can be used to treat cancer in combination with any one or more of these alkylating agents.

### b. Antimetabolites

Antimetabolites disrupt DNA and RNA synthesis. Unlike alkylating agents, they specifically influence the cell cycle during S phase. They have been used to combat chronic leukemias in addition to tumors of breast, ovary and the gastrointestinal tract. Antimetabolites include 5-fluorouracil (5-FU), cytarabine (Ara-C), fludarabine, gemcitabine, and methotrexate.

5-Fluorouracil (5-FU) has the chemical name of 5-fluoro-2,4(1H,3H)-pyrimidinedione. Its mechanism of action is thought to be by blocking the methylation reaction of deoxyuridylic acid to thymidylic acid. Thus, 5-FU interferes with the synthesis of deoxyribonucleic acid (DNA) and to a lesser extent inhibits the formation of ribonucleic acid (RNA). Since DNA and RNA are essential for cell division and proliferation, it is thought that the effect of 5-FU is to create a thymidine deficiency leading to cell death. Thus, the effect of 5-FU is found in cells that rapidly divide, a characteristic of metastatic cancers.

### c. Antitumor Antibiotics

Antitumor antibiotics have both antimicrobial and cytotoxic activity. These drugs also interfere with DNA by chemically inhibiting enzymes and mitosis or altering cellular membranes. These agents are not phase specific so they work in all phases of the cell cycle. Thus, they are widely used for a variety of cancers. Examples of antitumor antibiotics include bleomycin, dactinomycin, daunorubicin, doxorubicin (Adriamycin), and idarubicin, some of which are discussed in more detail below. Widely used in clinical setting for the treatment of neoplasms, these compounds are administered through bolus injections intravenously at doses ranging from 25-75 mg/m² at 21 day intervals for adriamycin, to 35-100 mg/m² for etoposide intravenously or orally.

### d. Mitotic Inhibitors

Mitotic inhibitors include plant alkaloids and other natural agents that can inhibit either protein synthesis required for cell division or mitosis. They operate during a specific phase during the cell cycle. Mitotic inhibitors comprise docetaxel, etoposide (VP16), paclitaxel, taxol, taxotere, vinblastine, vincristine, and vinorelbine.

### e. Nitrosureas

Nitrosureas, like alkylating agents, inhibit DNA repair proteins. They are used to treat non-Hodgkin's lymphomas, multiple myeloma, malignant melanoma, in addition to brain tumors. Examples include carmustine and lomustine.

### 2. Radiotherapy

Radiotherapy, also called radiation therapy, is the treatment of cancer and other diseases with ionizing radiation. Ionizing radiation deposits energy that injures or destroys cells in the area being treated by damaging their genetic material, making it impossible for these cells to continue to grow. Although radiation damages both cancer cells and normal cells, the latter are able to repair themselves and function properly. Radiotherapy may be used to treat localized solid tumors, such as cancers of the skin, tongue, larynx, brain, breast, or cervix. It can also be used to treat leukemia and lymphoma (cancers of the blood-forming cells and lymphatic system, respectively).

Radiation therapy used according to the present invention may include, but is not limited to, the use of γ-rays, X-rays, and/or the directed delivery of radioisotopes to tumor cells. Other forms of DNA damaging factors are also contemplated such as microwaves, proton beam irradiation (U.S. Patents 5,760,395 and 4,870,287) and UV-irradiation. It is most likely that all of these factors affect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells. Radiotherapy may comprise the use of radiolabeled antibodies to deliver doses of radiation directly to the cancer site (radioimmunotherapy). Once injected into the body, the antibodies actively seek out the cancer cells, which are destroyed by the cell-killing (cytotoxic) action of the radiation. This approach can minimize the risk of radiation damage to healthy cells.

Stereotactic radio-surgery (gamma knife) for brain and other tumors does not use a knife, but very precisely targeted beams of gamma radiotherapy from hundreds of different angles. Only one session of radiotherapy, taking about four to five hours, is needed. For this treatment a specially made metal frame is attached to the head. Then, several scans and x-rays are carried out to find the precise area where the treatment is needed. During the radiotherapy for brain tumors, the patient lies with their head in a large helmet, which has hundreds of holes in it to allow the radiotherapy beams through. Related approaches permit positioning for the treatment of tumors in other areas of the body.

### 3. Immunotherapy

In the context of cancer treatment, immunotherapeutics, generally, rely on the use of immune effector cells and molecules to target and destroy cancer cells. Trastuzumab (Herceptin™) is such an example. The immune effector may be, for example, an antibody specific for some marker on the surface of a tumor cell. The antibody alone may serve as an effector of therapy or it may recruit other cells to actually affect cell killing. The antibody also may be conjugated to a drug or toxin (chemotherapeutic, radionuclide, ricin A chain, cholera toxin, pertussis toxin, etc.) and serve merely as a targeting agent. Alternatively, the effector may be a lymphocyte carrying a surface molecule that interacts, either directly or indirectly, with a tumor cell target. Various effector cells include cytotoxic T cells and NK cells. The combination of therapeutic modalities, i.e., direct cytotoxic activity and inhibition or reduction of ErbB2 would provide therapeutic benefit in the treatment of ErbB2 overexpressing cancers.

In one aspect of immunotherapy, the tumor or disease cell must bear some marker that is amenable to targeting, i.e., is not present on the majority of other cells. Many tumor markers exist and any of these may be suitable for targeting in the context of the present invention. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, erb B and p155. An alternative aspect of immunotherapy is to combine anticancer effects with immune stimulatory effects. Immune stimulating molecules also exist including: cytokines such as IL-2, IL-4, IL-12, GM-CSF, gamma-IFN, and chemokines such as MIP-1, MCP-1, IL-8 and growth factors such as FLT3 ligand. Combining immune stimulating molecules, either as proteins or using gene delivery in combination with a tumor suppressor such as MDA-7 has been shown to enhance anti-tumor effects (Ju *et al.,* 2000). Moreover, antibodies against any of these compounds can be used to target the anti-cancer agents discussed herein.

Examples of immunotherapies currently under investigation or in use are immune adjuvants *e.g., Mycobacterium bovis, Plasmodium falciparum,* dinitrochlorobenzene and aromatic compounds (U.S. Patents 5,801,005 and 5,739,169; Hui and Hashimoto, 1998; Christodoulides *et al.,* 1998), cytokine therapy e.g., interferons α, β and γ; IL-1, GM-CSF and TNF (Bukowski *et al.,* 1998; Davidson *et al.,* 1998; Hellstrand *et al.,* 1998) gene therapy *e.g.,* TNF, IL-1, IL-2, p53 (Qin *et al.,* 1998; Austin-Ward and Villaseca, 1998; U.S. Patents 5,830,880 and 5,846,945) and monoclonal antibodies *e.g*., anti-ganglioside GM2, anti-HER-2, anti-p185; Pietras *et al.,* 1998; Hanibuchi *et al.,* 1998; U.S. Patent 5,824,311). Herceptin (trastuzumab) is a chimeric (mouse-human) monoclonal antibody that blocks the HER2-neu receptor. It possesses anti-tumor activity and has been approved for use in the treatment of malignant tumors (Dillman, 1999). A non-limiting list of several known anti-cancer immunotherapeutic agents and their targets includes, but is not limted to (Generic Name (Target)) Cetuximab (EGFR), Panitumumab (EGFR), Trastuzumab (erbB2 receptor), Bevacizumab (VEGF), Alemtuzumab (CD52), Gemtuzumab ozogamicin (CD33), Rituximab (CD20), Tositumomab (CD20), Matuzumab (EGFR), Ibritumomab tiuxetan (CD20), Tositumomab (CD20), HuPAM4 (MUC1), MORAb-009 (Mesothelin), G250 (carbonic anhydrase IX), mAb 8H9 (8H9 antigen), M195 (CD33), Ipilimumab (CTLA4), HuLuc63 (CS1), Alemtuzumab (CD53), Epratuzumab (CD22), BC8 (CD45), HuJ591 (Prostate specific membrane antigen), hA20 (CD20), Lexatumumab (TRAIL receptor-2), Pertuzumab (HER-2 receptor), Mik-beta-I (IL-2R), RAV12 (RAAG12), SGN-30 (CD30), AME-133v (CD20), HeFi-1 (CD30), BMS-663513 (CD137), Volociximab (anti-α5β1 integrin), GC1008 (TGFβ), HCD122 (CD40), Siplizumab (CD2), MORAb-003 (Folate receptor alpha), CNTO 328 (IL-6), MDX-060 (CD30), Ofatumumab (CD20), or SGN-33 (CD33). It is contemplated that one or more of these therapies may be employed with the miRNA therapies described herein.

A number of different approaches for passive immunotherapy of cancer exist. They may be broadly categorized into the following: injection of antibodies alone; injection of antibodies coupled to toxins or chemotherapeutic agents; injection of antibodies coupled to radioactive isotopes; injection of anti-idiotype antibodies; and finally, purging of tumor cells in bone marrow.

### 4. Gene Therapy

In yet another embodiment, a combination treatment involves gene therapy in which a therapeutic polynucleotide is administered before, after, or at the same time as one or more therapeutic miRNA. Delivery of a therapeutic polypeptide or encoding nucleic acid in conjunction with a miRNA may have a combined therapeutic effect on target tissues. A variety of proteins are encompassed within the invention, some of which are described below. Various genes that may be targeted for gene therapy of some form in combination with the present invention include, but are not limited to inducers of cellular proliferation, inhibitors of cellular proliferation, regulators of programmed cell death, cytokines and other therapeutic nucleic acids or nucleic acid that encode therapeutic proteins.

The tumor suppressor oncogenes function to inhibit excessive cellular proliferation. The inactivation of these genes destroys their inhibitory activity, resulting in unregulated proliferation. The tumor suppressors (*e.g*., therapeutic polypeptides) p53, FHIT, p16 and C-CAM can be employed.

In addition to p53, another inhibitor of cellular proliferation is p16. The major transitions of the eukaryotic cell cycle are triggered by cyclin-dependent kinases, or CDK's. One CDK, cyclin-dependent kinase 4 (CDK4), regulates progression through the G1. The activity of this enzyme may be to phosphorylate Rb at late G1. The activity of CDK4 is controlled by an activating subunit, D-type cyclin, and by an inhibitory subunit, the p16INK4 has been biochemically characterized as a protein that specifically binds to and inhibits CDK4, and thus may regulate Rb phosphorylation (Serrano *et al.,* 1993; Serrano *et al.,* 1995). Since the p16INK4 protein is a CDK4 inhibitor (Serrano, 1993), deletion of this gene may increase the activity of CDK4, resulting in hyperphosphorylation of the Rb protein, p16 also is known to regulate the function of CDK6.

p16INK4 belongs to a newly described class of CDK-inhibitory proteins that also includes p16B, p19, p21WAF1, and p27KIP1. The p16INK4 gene maps to 9p21, a chromosome region frequently deleted in many tumor types. Homozygous deletions and mutations of the p16INK4 gene are frequent in human tumor cell lines. This evidence suggests that the p16INK4 gene is a tumor suppressor gene. This interpretation has been challenged, however, by the observation that the frequency of the p16INK4 gene alterations is much lower in primary uncultured tumors than in cultured cell lines (Caldas *et al.,* 1994; Cheng *et al.,* 1994; Hussussian *et al.,* 1994; Kamb *et al.,* 1994; Mori *et al.,* 1994; Okamoto *et al.*, 1994; Nobori *et al.,* 1995; Orlow *et al.,* 1994; Arap *et al.,* 1995). Restoration of wild-type p16INK4 function by transfection with a plasmid expression vector reduced colony formation by some human cancer cell lines (Okamoto, 1994; Arap, 1995).

Other genes that may be employed according to the present invention include Rb, APC, DCC, NF-1, NF-2, WT-1, MEN-I, MEN-II, zac1, p73, VHL, MMAC1 / PTEN, DBCCR-1, FCC, rsk-3, p27, p27/p16 fusions, p21/p27 fusions, anti-thrombotic genes (e.g., COX-1, TFPI), PGS, Dp, E2F, ras, myc, neu, raf, erb, fins, trk, ret, gsp, hst, abl, EIA, p300, genes involved in angiogenesis (e.g., VEGF, FGF, thrombospondin, BAI-1, GDAIF, or their receptors) and MCC.

### 5. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatment of the present invention, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and microscopically controlled surgery (Mohs' surgery). It is further contemplated that the present invention may be used in conjunction with removal of superficial cancers, precancers, or incidental amounts of normal tissue.

Upon excision of part of all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### 6. Other Agents

It is contemplated that other agents may be used in combination with the present invention to improve the therapeutic efficacy of treatment. These additional agents include immunomodulatory agents, agents that affect the upregulation of cell surface receptors and GAP junctions, cytostatic and differentiation agents, inhibitors of cell adhesion, agents that increase the sensitivity of the hyperproliferative cells to apoptotic inducers, or other biological agents. Immunomodulatory agents include tumor necrosis factor; interferon alpha, beta, and gamma; IL-2 and other cytokines; F42K and other cytokine analogs; or MIP-1, MIP-1beta, MCP-1, RANTES, and other chemokines. It is further contemplated that the upregulation of cell surface receptors or their ligands such as Fas / Fas ligand, DR4 or DR5 / TRAIL (Apo-2 ligand) would potentiate the apoptotic inducing abilities of the present invention by establishment of an autocrine or paracrine effect on hyperproliferative cells. Increases intercellular signaling by elevating the number of GAP junctions would increase the anti-hyperproliferative effects on the neighboring hyperproliferative cell population. In other embodiments, cytostatic or differentiation agents can be used in combination with the present invention to improve the anti-hyperproliferative efficacy of the treatments. Inhibitors of cell adhesion are contemplated to improve the efficacy of the present invention. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with the present invention to improve the treatment efficacy.

Apo2 ligand (Apo2L, also called TRAIL) is a member of the tumor necrosis factor (TNF) cytokine family. TRAIL activates rapid apoptosis in many types of cancer cells, yet is not toxic to normal cells. TRAIL mRNA occurs in a wide variety of tissues. Most normal cells appear to be resistant to TRAIL's cytotoxic action, suggesting the existence of mechanisms that can protect against apoptosis induction by TRAIL. The first receptor described for TRAIL, called death receptor 4 (DR4), contains a cytoplasmic "death domain"; DR4 transmits the apoptosis signal carried by TRAIL. Additional receptors have been identified that bind to TRAIL. One receptor, called DR5, contains a cytoplasmic death domain and signals apoptosis much like DR4. The DR4 and DR5 mRNAs are expressed in many normal tissues and tumor cell lines. Recently, decoy receptors such as DcR1 and DcR2 have been identified that prevent TRAIL from inducing apoptosis through DR4 and DR5. These decoy receptors thus represent a novel mechanism for regulating sensitivity to a pro-apoptotic cytokine directly at the cell's surface. The preferential expression of these inhibitory receptors in normal tissues suggests that TRAIL may be useful as an anticancer agent that induces apoptosis in cancer cells while sparing normal cells. (Marsters *et al.,* 1999).

There have been many advances in the therapy of cancer following the introduction of cytotoxic chemotherapeutic drugs. However, one of the consequences of chemotherapy is the development/acquisition of drug-resistant phenotypes and the development of multiple drug resistance. The development of drug resistance remains a major obstacle in the treatment of such tumors and therefore, there is an obvious need for alternative approaches such as gene therapy.

Another form of therapy for use in conjunction with chemotherapy, radiation therapy or biological therapy includes hyperthermia, which is a procedure in which a patient's tissue is exposed to high temperatures (up to 106°F). External or internal heating devices may be involved in the application of local, regional, or whole-body hyperthermia. Local hyperthermia involves the application of heat to a small area, such as a tumor. Heat may be generated externally with high-frequency waves targeting a tumor from a device outside the body. Internal heat may involve a sterile probe , including thin, heated wires or hollow tubes filled with warm water, implanted microwave antennae, or radiofrequency electrodes.

A patient's organ or a limb is heated for regional therapy, which is accomplished using devices that produce high energy, such as magnets. Alternatively, some of the patient's blood may be removed and heated before being perfused into an area that will be internally heated. Whole-body heating may also be implemented in cases where cancer has spread throughout the body. Warm-water blankets, hot wax, inductive coils, and thermal chambers may be used for this purpose.

Hormonal therapy may also be used in conjunction with the present invention or in combination with any other cancer therapy previously described. The use of hormones may be employed in the treatment of certain cancers such as breast, prostate, ovarian, or cervical cancer to lower the level or block the effects of certain hormones such as testosterone or estrogen. This treatment is often used in combination with at least one other cancer therapy as a treatment option or to reduce the risk of metastases.

This application incorporates U.S. Application Serial No. 11/349,727 filed on February 8, 2006 claiming priority to U.S. Provisional Application Serial No. 60/650,807 filed February 8, 2005.

### III. MIRNA MOLECULES

MicroRNA molecules ("miRNAs") are generally 21 to 22 nucleotides in length, though lengths of 19 and up to 23 nucleotides have been reported. The miRNAs are each processed from a longer precursor RNA molecule ("precursor miRNA"). Precursor miRNAs are transcribed from non-protein-encoding genes. The precursor miRNAs have two regions of complementarity that enables them to form a stem-loop- or fold-back-like structure, which is cleaved in animals by a ribonuclease III-like nuclease enzyme called Dicer. The processed miRNA is typically a portion of the stem.

The processed miRNA (also referred to as "mature miRNA") becomes part of a large complex to down-regulate a particular target gene or its gene product. Examples of animal miRNAs include those that imperfectly basepair with the target, which halts translation (Olsen *et al.,* 1999; Seggerson *et al.,* 2002). siRNA molecules also are processed by Dicer, but from a long, double-stranded RNA molecule. siRNAs are not naturally found in animal cells, but they can direct the sequence-specific cleavage of an mRNA target through a RNA-induced silencing complex (RISC) (Denli *et al.,* 2003).

### A. Array Preparation

Certain embodiments of the present invention concerns the preparation and use of mRNA or nucleic acid arrays, miRNA or nucleic acid arrays, and/or miRNA or nucleic acid probe arrays, which are macroarrays or microarrays of nucleic acid molecules (probes) that are fully or nearly complementary (over the length of the prove) or identical (over the length of the prove) to a plurality of nucleic acid, mRNA or miRNA molecules, precursor miRNA molecules, or nucleic acids derived from the various genes and gene pathways modulated by miR-126 miRNAs and that are positioned on a support or support material in a spatially separated organization. Macroarrays are typically sheets of nitrocellulose or nylon upon which probes have been spotted. Microarrays position the nucleic acid probes more densely such that up to 10,000 nucleic acid molecules can be fit into a region typically I to 4 square centimeters. Microarrays can be fabricated by spotting nucleic acid molecules, *e.g*., genes, oligonucleotides, *etc.*, onto substrates or fabricating oligonucleotide sequences *in situ* on a substrate. Spotted or fabricated nucleic acid molecules can be applied in a high density matrix pattern of up to about 30 non-identical nucleic acid molecules per square centimeter or higher, e.g. up to about 100 or even 1000 per square centimeter. Microarrays typically use coated glass as the solid support, in contrast to the nitrocellulose-based material of filter arrays. By having an ordered array of marker RNA and/or miRNA-complementing nucleic acid samples, the position of each sample can be tracked and linked to the original sample.

A variety of different array devices in which a plurality of distinct nucleic acid probes are stably associated with the surface of a solid support are known to those of skill in the art. Useful substrates for arrays include nylon, glass, metal, plastic, latex, and silicon. Such arrays may vary in a number of different ways, including average probe length, sequence or types of probes, nature of bond between the probe and the array surface, *e.g.* covalent or non-covalent, and the like. The labeling and screening methods of the present invention and the arrays are not limited in its utility with respect to any parameter except that the probes detect miRNA, or genes or nucleic acid representative of genes; consequently, methods and compositions may be used with a variety of different types of nucleic acid arrays.

Representative methods and apparatus for preparing a microarray have been described, for example, in U.S. Patents 5,143,854; 5,202,231; 5,242,974; 5,288,644; 5,324,633; 5,384,261; 5,405,783; 5,412,087; 5,424,186; 5,429,807; 5,432,049; 5,436,327; 5,445,934; 5,468,613; 5,470,710; 5,472,672; 5,492,806; 5,525,464; 5,503,980; 5,510,270; 5,525,464; 5,527,681; 5,529,756; 5,532,128; 5,545,531; 5,547,839; 5,554,501; 5,556,752; 5,561,071; 5,571,639; 5,580,726; 5,580,732; 5,593,839; 5,599,695; 5,599,672; 5,610;287; 5,624,711; 5,631,134; 5,639,603; 5,654,413; 5,658,734; 5,661,028; 5,665,547; 5,667,972; 5,695,940; 5,700,637; 5,744,305; 5,800,992; 5,807,522; 5,830,645; 5,837,196; 5,871,928; 5,847,219; 5,876,932; 5,919,626; 6,004,755; 6,087,102; 6,368,799; 6,383,749; 6,617,112; 6,638,717; 6,720,138, as well as WO 93/17126; WO 95/11995; WO 95/21265; WO 95/21944; WO 95/35505; WO 96/31622; WO 97/10365; WO 97/27317; WO 99/35505; WO 09923256; WO 09936760; WO0138580; WO 0168255; WO 03020898; WO 03040410; WO 03053586; WO 03087297; WO03091426; WO03100012; WO 04020085; WO 04027093; EP 373 203; EP 785 280; EP 799 897 and UK 8 803 000 .

It is contemplated that the arrays can be high density arrays, such that they contain 2, 20, 25, 50, 80, 100 or more different probes. It is contemplated that they may contain 1000, 16,000, 65,000, 250,000 or 1,000,000 or more different probes. The probes can be directed to mRNA and/or miRNA targets in one or more different organisms or cell types. The oligonucleotide probes range from 5 to 50, 5 to 45, 10 to 40, 9 to 34, or 15 to 40 nucleotides in length in some embodiments. In certain embodiments, the oligonucleotide probes are 5, 10, 15, 20 to 20, 25, 30, 35, 40 nucleotides in length including all integers and ranges there between.

The location and sequence of each different probe sequence in the array are generally known. Moreover, the large number of different probes can occupy a relatively small area providing a high density array having a probe density of generally greater than about 60, 100, 600, 1000, 5,000, 10,000, 40,000, 100,000, or 400,000 different oligonucleotide probes per cm². The surface area of the array can be about or less than about 1, 1.6, 2, 3, 4, 5, 6, 7, 8, 9, or 10 cm².

Moreover, a person of ordinary skill in the art could readily analyze data generated using an array. Such protocols are disclosed above, and include information found in WO 9743450; WO 03023058; WO 03022421; WO 03029485; WO 03067217; WO 03066906; WO 03076928; WO 03093810; WO 03100448A1 .

### B. Sample Preparation

It is contemplated that the RNA and/or miRNA of a wide variety of samples can be analyzed using the arrays, index of probes, or array technology of the invention. While endogenous miRNA is contemplated for use with compositions and methods of the invention, recombinant miRNA - including nucleic acids that are complementary or identical to endogenous miRNA or precursor miRNA - can also be handled and analyzed as described herein. Samples may be biological samples, in which case, they can be from biopsy, fine needle aspirates, exfoliates, blood, tissue, organs, semen, saliva, tears, other bodily fluid, hair follicles, skin, or any sample containing or constituting biological cells, particularly cancer or hyperproliferative cells. In certain embodiments, samples may be, but are not limited to, biopsy, or cells purified or enriched to some extent from a biopsy or other bodily fluids or tissues. Alternatively, the sample may not be a biological sample, but be a chemical mixture, such as a cell-free reaction mixture (which may contain one or more biological enzymes).

### C. Hybridization

After an array or a set of probes is prepared and/or the nucleic acid in the sample or probe is labeled, the population of target nucleic acids is contacted with the array or probes under hybridization conditions, where such conditions can be adjusted, as desired, to provide for an optimum level of specificity in view of the particular assay being performed. Suitable hybridization conditions are well known to those of skill in the art and reviewed in Sambrook *et al.* (2001) and WO 95/21944. Of particular interest in many embodiments is the use of stringent conditions during hybridization. Stringent conditions are known to those of skill in the art.

It is specifically contemplated that a single array or set of probes may be contacted with multiple samples. The samples may be labeled with different labels to distinguish the samples. For example, a single array can be contacted with a tumor tissue sample labeled with Cy3, and normal tissue sample labeled with Cry5. Differences between the samples for particular miRNAs corresponding to probes on the array can be readily ascertained and quantified.

The small surface area of the array permits uniform hybridization conditions, such as temperature regulation and salt content. Moreover, because of the small area occupied by the high density arrays, hybridization may be carried out in extremely small fluid volumes (*e.g*., about 250 µl or less, including volumes of about or less than about 5, 10, 25, 50, 60, 70, 80, 90, 100 µl, or any range derivable therein). In small volumes, hybridization may proceed very rapidly.

### D. Differential Expression Analyses

Arrays of the invention can be used to detect differences between two samples. Specifically contemplated applications include identifying and/or quantifying differences between miRNA or gene expression from a sample that is normal and from a sample that is not normal, between a disease or condition and a cell not exhibiting such a disease or condition, or between two differently treated samples. Also, miRNA or gene expression may be compared between a sample believed to be susceptible to a particular disease or condition and one believed to be not susceptible or resistant to that disease or condition. A sample that is not normal is one exhibiting phenotypic or genotypic trait(s) of a disease or condition, or one believed to be not normal with respect to that disease or condition. It may be compared to a cell that is normal with respect to that disease or condition. Phenotypic traits include symptoms of, or susceptibility to, a disease or condition of which a component is or may or may not be genetic, or caused by a hyperproliferative or neoplastic cell or cells.

An array comprises a solid support with nucleic acid probes attached to the support. Arrays typically comprise a plurality of different nucleic acid probes that are coupled to a surface of a substrate in different, known locations. These arrays, also described as "microarrays" or colloquially "chips" have been generally described in the art, for example, U.S. Patents 5,143,854, 5,445,934, 5,744,305, 5,677,195, 6,040,193, 5,424,186 and Fodor *et al.,* (1991). Techniques for the synthesis of these arrays using mechanical synthesis methods are described in, *e.g*., U.S. Patent 5,384,261 . Although a planar array surface is used in certain aspects, the array may be fabricated on a surface of virtually any shape or even a multiplicity of surfaces. Arrays may be nucleic acids on beads, gels, polymeric surfaces, fibers such as fiber optics, glass or any other appropriate substrate, see U.S. Patents 5,770,358, 5,789,162, 5,708,153, 6,040,193 and 5,800,992. Arrays may be packaged in such a manner as to allow for diagnostics or other manipulation of an all inclusive device, see for example, U.S. Patents 5,856,174 and 5,922,591 . See also U.S. patent application Ser. No. 09/545,207, filed April, 7, 2000 for additional information concerning arrays, their manufacture, and their characteristics.

Particularly, arrays can be used to evaluate samples with respect to pathological condition such as cancer and related conditions. It is specifically contemplated that the invention can be used to evaluate differences between stages or sub-classifications of disease, such as between benign, cancerous, and metastatic tissues or tumors.

Phenotypic traits to be assessed include characteristics such as longevity, morbidity, expected survival, susceptibility or receptivity to particular drugs or therapeutic treatments (drug efficacy), and risk of drug toxicity. Samples that differ in these phenotypic traits may also be evaluated using the compositions and methods described.

In certain embodiments, miRNA and/or expression profiles may be generated to evaluate and correlate those profiles with pharmacokinetics or therapies. For example, these profiles may be created and evaluated for patient tumor and blood samples prior to the patient's being treated or during treatment to determine if there are miRNA or genes whose expression correlates with the outcome of the patient's treatment. Identification of differential miRNAs or genes can lead to a diagnostic assay for evaluation of tumor and/or blood samples to determine what drug regimen the patient should be provided. In addition, it can be used to identify or select patients suitable for a particular clinical trial. If an expression profile is determined to be correlated with drug efficacy or drug toxicity, that profile is relevant to whether that patient is an appropriate patient for receiving a drug, for receiving a combination of drugs, or for receiving a particular dosage of the drug.

In addition to the above prognostic assay, samples from patients with a variety of diseases can be evaluated to determine if different diseases can be identified based on miRNA and/or related gene expression levels. A diagnostic assay can be created based on the profiles that doctors can use to identify individuals with a disease or who are at risk to develop a disease. Alternatively, treatments can be designed based on miRNA profiling. Examples of such methods and compositions are described in the U.S. Provisional Patent Application entitled "Methods and Compositions Involving miRNA and miRNA Inhibitor Molecules" filed on May 23, 2005 in the names of David Brown, Lance Ford, Angie Cheng and Rich Jarvis.

### E. Other Assays

In addition to the use of arrays and microarrays, it is contemplated that a number of different assays could be employed to analyze miRNAs or related genes, their activities, and their effects. Such assays include, but are not limited to, nucleic acid amplification, polymerase chain reaction, quantitative PCR, RT-PCR, *in situ* hybridization, Northern hybridization, hybridization protection assay (HPA)(GenProbe), branched DNA (DNA) assay (Chiron), rolling circle amplification (RCA), single molecule hybridization detection (US Genomics), Invader assay (ThirdWave Technologies), and/or Bridge Litigation Assay (Genaco).

### IV. NUCLEIC ACIDS

The present invention concerns nucleic acids, modified or mimetic nucleic acids, miRNAs, mRNAs, genes, and representative fragments thereof that can be labeled, used in array analysis, or employed in diagnostic, therapeutic, or prognostic applications, particularly those related to pathological conditions such as cancer. The molecules may have been endogenously produced by a cell, or been synthesized or produced chemically or recombinantly. They may be isolated and/or purified. Each of the miRNAs described herein and includes the corresponding SEQ ID NO and accession numbers for these miRNA sequences. The name of a miRNA is often abbreviated and referred to without a "hsa-" prefix and will be understood as such, depending on the context. Unless otherwise indicated, miRNAs referred to in the application are human sequences identified as miR-X or let-X, where X is a number and/or letter.

In certain aspects, a miRNA probe designated by a suffix "5P" or "3P" can be used. "5P" indicates that the mature miRNA derives from the 5' end of the precursor and a corresponding "3P" indicates that it derives from the 3' end of the precursor, as described on the world wide web at sanger.ac.uk. Moreover, in some embodiments, a miRNA probe is used that does not correspond to a known human miRNA. It is contemplated that these non-human miRNA probes may be used in embodiments of the invention or that there may exist a human miRNA that is homologous to the non-human miRNA. In other embodiments, any mammalian cell, biological sample, or preparation thereof may be employed.

In some embodiments of the invention, methods and compositions involving miRNA may concern miRNA, markers (e.g., mRNAs), and/or other nucleic acids. Nucleic acids may be, be at least, or be at most 3, 4, 5, 6, 7, 8, 9, 10,11,12,13,14,15,16,17,18,19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 120, 130, 140, z0, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides, or any range derivable therein, in length. Such lengths cover the lengths of processed miRNA, miRNA probes, precursor miRNA, miRNA containing vectors, mRNA, mRNA probes, control nucleic acids, and other probes and primers.

In many embodiments, miRNA are 19-24 nucleotides in length, while miRNA probes are 19-35 nucleotides in length, depending on the length of the processed miRNA and any flanking regions added. miRNA precursors are generally between 62 and l10 nucleotides in humans.

Nucleic acids of the invention may have regions of identity or complementarity to another nucleic acid. It is contemplated that the region of complementarity or identity can be at least 5 contiguous residues, though it is specifically contemplated that the region is, is at least, or is at most 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 contiguous nucleotides. It is further understood that the length of complementarity within a precursor miRNA or other nucleic acid or between a miRNA probe and a miRNA or a miRNA gene are such lengths. Moreover, the complementarity may be expressed as a percentage, meaning that the complementarity between a probe and its target is 90% or greater over the length of the probe. In some embodiments, complementarity is or is at least 90%, 95% or 100%. In particular, such lengths may be applied to any nucleic acid comprising a nucleic acid sequence identified in any of SEQ ID NOs described herein, accession number, or any other sequence disclosed herein. Typically, the commonly used name of the miRNA is given (with its identifying source in the prefix, for example, "hsa" for human sequences) and the processed miRNA sequence. Unless otherwise indicated, a miRNA without a prefix will be understood to refer to a human miRNA. Moreover, a lowercase letter in a miRNA name may or may not be lowercase; for example, hsa-mir-130b can also be referred to as miR-130B. The term "miRNA probe" refers to a nucleic acid probe that can identify a particular miRNA or structurally related miRNAs.

It is understood that some nucleic acids are derived from genomic sequences or a gene. In this respect, the term "gene" is used for simplicity to refer to the genomic sequence encoding the precursor nucleic acid or miRNA for a given miRNA or gene. However, embodiments of the invention may involve genomic sequences of a miRNA that are involved in its expression, such as a promoter or other regulatory sequences.

The term "recombinant" may be used and this generally refers to a molecule that has been manipulated *in vitro* or that is a replicated or expressed product of such a molecule.

The term "nucleic acid" is well known in the art. A "nucleic acid" as used herein will generally refer to a molecule (one or more strands) of DNA, RNA or a derivative or analog thereof, comprising a nucleobase. A nucleobase includes, for example, a naturally occurring purine or pyrimidine base found in DNA (*e.g*., an adenine "A," a guanine "G," a thymine "T" or a cytosine "C") or RNA (*e.g*., an A, a G, an uracil "U" or a C). The term "nucleic acid" encompasses the terms "oligonucleotide" and "polynucleotide," each as a subgenus of the term "nucleic acid."

The term "miRNA" generally refers to a single-stranded molecule, but in specific embodiments, molecules implemented in the invention will also encompass a region or an additional strand that is partially (between 10 and 50% complementary across length of strand), substantially (greater than 50% but less than 100% complementary across length of strand) or fully complementary to another region of the same single-stranded molecule or to another nucleic acid. Thus, miRNA nucleic acids may encompass a molecule that comprises one or more complementary or self-complementary strand(s) or "complement(s)" of a particular sequence. For example, precursor miRNA may have a self-complementary region, which is up to 100% complementary. miRNA probes or nucleic acids of the invention can include, can be or can be at least 60, 65, 70, 75, 80, 85, 90, 95, 96, 97, 98, 99 or 100% complementary to their target.

It is understood that a "synthetic nucleic acid" of the invention means that the nucleic acid does not have all or part of a chemical structure or sequence of a naturally occurring nucleic acid. Consequently, it will be understood that the term "synthetic miRNA" refers to a "synthetic nucleic acid" that functions in a cell or under physiological conditions as a naturally occurring miRNA.

While embodiments of the invention may involve synthetic miRNAs or synthetic nucleic acids, in some embodiments of the invention, the nucleic acid molecule(s) need not be "synthetic." In certain embodiments, a non-synthetic nucleic acid or miRNA employed in methods and compositions of the invention may have the entire sequence and structure of a naturally occurring mRNA or miRNA precursor or the mature mRNA or miRNA. For example, non-synthetic miRNAs used in methods and compositions of the invention may not have one or more modified nucleotides or nucleotide analogs. In these embodiments, the non-synthetic miRNA may or may not be recombinantly produced. In particular embodiments, the nucleic acid in methods and/or compositions of the invention is specifically a synthetic miRNA and not a non-synthetic miRNA (that is, not a miRNA that qualifies as "synthetic"); though in other embodiments, the invention specifically involves a non-synthetic miRNA and not a synthetic miRNA. Any embodiments discussed with respect to the use of synthetic miRNAs can be applied with respect to non-synthetic miRNAs, and *vice versa.*

It will be understood that the term "naturally occurring" refers to something found in an organism without any intervention by a person; it could refer to a naturally-occurring wildtype or mutant molecule. In some embodiments a synthetic miRNA molecule does not have the sequence of a naturally occurring miRNA molecule. In other embodiments, a synthetic miRNA molecule may have the sequence of a naturally occurring miRNA molecule, but the chemical structure of the molecule, particularly in the part unrelated specifically to the precise sequence (non-sequence chemical structure) differs from chemical structure of the naturally occurring miRNA molecule with that sequence. In some cases, the synthetic miRNA has both a sequence and non-sequence chemical structure that are not found in a naturally-occurring miRNA. Moreover, the sequence of the synthetic molecules will identify which miRNA is effectively being provided or inhibited; the endogenous miRNA will be referred to as the "corresponding miRNA." Corresponding miRNA sequences that can be used in the context of the invention include, but are not limited to, all or a portion of those sequences in the SEQ IDs provided herein, as well as any other miRNA sequence, miRNA precursor sequence, or any sequence complementary thereof. In some embodiments, the sequence is or is derived from or contains all or part of a sequence identified herein to target a particular miRNA (or set of miRNAs) that can be used with that sequence. Any 1, 2, 3, 4, 5, 6, 7, 8, 9, 10, 11, 12, 13, 14, 15, 16, 17. 18, 19, 20, 30, 40, 50, 60, 70, 80, 90, 100, 110, 120, 130 140, 150, 160, 170, 180, 190, 200, 210, 220, 230, 240, 250, 260 or any number or range of sequences there between may be selected to the exclusion of all non-selected sequences.

As used herein, "hybridization", "hybridizes" or "capable of hybridizing" is understood to mean the forming of a double or triple stranded molecule or a molecule with partial double or triple stranded nature. The term "anneal" as used herein is synonymous with "hybridize." The term "hybridization", "hybridize(s)" or "capable of hybridizing" encompasses the terms "stringent condition(s)" or "high stringency" and the terms "low stringency" or "low stringency condition(s)."

As used herein "stringent condition(s)" or "high stringency" are those conditions that allow hybridization between or within one or more nucleic acid strand(s) containing complementary sequence(s), but preclude hybridization of random sequences. Stringent conditions tolerate little, if any, mismatch between a nucleic acid and a target strand. Such conditions are well known to those of ordinary skill in the art, and are preferred for applications requiring high selectivity. Non-limiting applications include isolating a nucleic acid, such as a gene or a nucleic acid segment thereof, or detecting at least one specific mRNA transcript or a nucleic acid segment thereof, and the like.

Stringent conditions may comprise low salt and/or high temperature conditions, such as provided by about 0.02 M to about 0.5 M NaCl at temperatures of about 42°C to about 70°C. It is understood that the temperature and ionic strength of a desired stringency are determined in part by the length of the particular nucleic acid(s), the length and nucleobase content of the target sequence(s), the charge composition of the nucleic acid(s), and to the presence or concentration of formamide, tetramethylammonium chloride or other solvent(s) in a hybridization mixture.

It is also understood that these ranges, compositions and conditions for hybridization are mentioned by way of non-limiting examples only, and that the desired stringency for a particular hybridization reaction is often determined empirically by comparison to one or more positive or negative controls. Depending on the application envisioned it is preferred to employ varying conditions of hybridization to achieve varying degrees of selectivity of a nucleic acid towards a target sequence. In a non-limiting example, identification or isolation of a related target nucleic acid that does not hybridize to a nucleic acid under stringent conditions may be achieved by hybridization at low temperature and/or high ionic strength. Such conditions are termed "low stringency" or "low stringency conditions," and non-limiting examples of low stringency include hybridization performed at about 0.15 M to about 0.9 M NaCl at a temperature range of about 20°C to about 50°C. Of course, it is within the skill of one in the art to further modify the low or high stringency conditions to suite a particular application.

### A. Nucleobase, Nucleoside, Nucleotide, and Modified Nucleotides

As used herein a "nucleobase" refers to a heterocyclic base, such as for example a naturally occurring nucleobase (*i.e*., an A, T, G, C or U) found in at least one naturally occurring nucleic acid (*i.e*., DNA and RNA), and naturally or non-naturally occurring derivative(s) and analogs of such a nucleobase. A nucleobase generally can form one or more hydrogen bonds ("anneal" or "hybridize") with at least one naturally occurring nucleobase in a manner that may substitute for naturally occurring nucleobase pairing (*e.g*., the hydrogen bonding between A and T, G and C, and A and U).

"Purine" and/or "pyrimidine" nucleobase(s) encompass naturally occurring purine and/or pyrimidine nucleobases and also derivative(s) and analog(s) thereof, including but not limited to, those a purine or pyrimidine substituted by one or more of an alkyl, caboxyalkyl, amino, hydroxyl, halogen (*i.e.*, fluoro, chloro, bromo, or iodo), thiol or alkylthiol moiety. Preferred alkyl (*e.g.*, alkyl, carboxyalkyl, *etc.*) moieties comprise of from about 1, about 2, about 3, about 4, about 5, to about 6 carbon atoms. Other non-limiting examples of a purine or pyrimidine include a deazapurine, a 2,6-diaminopurine, a 5-fluorouracil, a xanthine, a hypoxanthine, a 8-bromoguanine, a 8-chloroguanine, a bromothymine, a 8-aminoguanine, a 8-hydroxyguanine, a 8-methylguanine, a 8-thioguanine, an azaguanine, a 2-aminopurine, a 5-ethylcytosine, a 5-methylcyosine, a 5-bromouracil, a 5-ethyluracil, a 5-iodouracil, a 5-chlorouracil, a 5-propyluracil, a thiouracil, a 2-methyladenine, a methylthioadenine, a N,N-diemethyladenine, an azaadenines, a 8-bromoadenine, a 8-hydroxyadenine, a 6-hydroxyaminopurine, a 6-thiopurine, a 4-(6-aminohexyl/cytosine), and the like. Other examples are well known to those of skill in the art.

As used herein, a "nucleotide" refers to an individual chemical unit comprising a nucleobase covalently attached to a nucleobase linker moiety. A non-limiting example of a "nucleobase linker moiety" is a sugar comprising 5-carbon atoms (*i.e*., a "5-carbon sugar"), including but not limited to a deoxyribose, a ribose, an arabinose, or a derivative or an analog of a 5-carbon sugar. Non-limiting examples of a derivative or an analog of a 5-carbon sugar include a 2'-fluoro-2'-deoxyribose or a carbocyclic sugar where a carbon is substituted for an oxygen atom in the sugar ring. Different types of covalent attachment(s) of a nucleobase to a nucleobase linker moiety are known in the art (Kornberg and Baker, 1992).

As used herein, a "nucleotide" refers to a nucleoside further comprising a "backbone moiety". A backbone moiety generally covalently attaches a nucleotide to another molecule comprising a nucleotide, or to another nucleotide to form a nucleic acid. The "backbone moiety'' in naturally occurring nucleotides typically comprises a phosphorus moiety, which is covalently attached to a 5-carbon sugar. The attachment of the backbone moiety typically occurs at either the 3'- or 5'-position of the 5-carbon sugar. However, other types of attachments are known in the art, particularly when a nucleotide comprises derivatives or analogs of a naturally occurring 5-carbon sugar or phosphorus moiety.

A nucleic acid may comprise, or be composed entirely of, a derivative or analog of a nucleobase, a nucleobase linker moiety and/or backbone moiety that may be present in a naturally occurring nucleic acid. RNA with nucleic acid analogs may also be labeled according to methods of the invention. As used herein a "derivative" refers to a chemically modified or altered form of a naturally occurring molecule, while the terms "mimic" or "analog" refer to a molecule that may or may not structurally resemble a naturally occurring molecule or moiety, but possesses similar functions. As used herein, a "moiety" generally refers to a smaller chemical or molecular component of a larger chemical or molecular structure. Nucleobase, nucleoside and nucleotide analogs or derivatives are well known in the art, and have been described (see for example, Scheit, 1980 .

Additional non-limiting examples of nucleosides, nucleotides or nucleic acids include those in: U.S. Patents 5,681,947, 5,652,099 and 5,763,167, 5,614,617, 5,670,663, 5,872,232, 5,859,221, 5,446,137, 5,886,165, 5,714,606, 5,672,697, 5,466,786, 5,792,847, 5,223,618, 5,470,967, 5,378,825, 5,777,092, 5,623,070, 5,610,289, 5,602,240, 5,858,988, 5,214,136, 5,700,922, 5,708,154, 5,728,525, 5,637,683, 6,251,666, 5,480,980, and 5,728,525,

Labeling methods and kits of the invention specifically contemplate the use of nucleotides that are both modified for attachment of a label and can be incorporated into a miRNA molecule. Such nucleotides include those that can be labeled with a dye, including a fluorescent dye, or with a molecule such as biotin. Labeled nucleotides are readily available; they can be acquired commercially or they can be synthesized by reactions known to those of skill in the art.

Modified nucleotides for use in the invention are not naturally occurring nucleotides, but instead, refer to prepared nucleotides that have a reactive moiety on them. Specific reactive functionalities of interest include: amino, sulfhydryl, sulfoxyl, aminosulfhydryl, azido, epoxide, isothiocyanate, isocyanate, anhydride, monochlorotriazine, dichlorotriazine, mono-or dihalogen substituted pyridine, mono- or disubstituted diazine, maleimide, epoxide, aziridine, sulfonyl halide, acid halide, alkyl halide, aryl halide, alkylsulfonate, N-hydroxysuccinimide ester, imido ester, hydrazine, azidonitrophenyl, azide, 3-(2-pyridyl dithio)-propionamide, glyoxal, aldehyde, iodoacetyl, cyanomethyl ester, p-nitrophenyl ester, o-nitrophenyl ester, hydroxypyridine ester, carbonyl imidazole, and the other such chemical groups. In some embodiments, the reactive functionality may be bonded directly to a nucleotide, or it may be bonded to the nucleotide through a linking group. The functional moiety and any linker cannot substantially impair the ability of the nucleotide to be added to the miRNA or to be labeled. Representative linking groups include carbon containing linking groups, typically ranging from about 2 to 18, usually from about 2 to 8 carbon atoms, where the carbon containing linking groups may or may not include one or more heteroatoms, *e.g*. S, O, N etc., and may or may not include one or more sites of unsaturation. Of particular interest in many embodiments is alkyl linking groups, typically lower alkyl linking groups of 1 to 16, usually 1 to 4 carbon atoms, where the linking groups may include one or more sites of unsaturation. The functionalized nucleotides (or primers) used in the above methods of functionalized target generation may be fabricated using known protocols or purchased from commercial vendors, *e.g.*, Sigma, Roche, Ambion, Biosearch Technologies and NEN. Functional groups may be prepared according to ways known to those of skill in the art, including the representative information found in U.S. Patents 4,404,289; 4,405,711; 4,337,063 and 5,268,486, and U.K. Patent 1,529,202 .

Amine-modified nucleotides are used in several embodiments of the invention. The amine-modified nucleotide is a nucleotide that has a reactive amine group for attachment of the label. It is contemplated that any ribonucleotide (G, A, U, or C) or deoxyribonucleotide (G, A, T, or C) can be modified for labeling. Examples include, but are not limited to, the following modified ribo- and deoxyribo-nucleotides: 5-(3-aminoallyl)-UTP; 8-[(4-amino)butyl]-amino-ATP and 8-[(6-amino)butyl]-amino-ATP; N6-(4-amino)butyl-ATP, N6-(6-amino)butyl-ATP, N4-[2,2-oxy-bis-(ethylamine)]-CTP; N6-(6-Amino)hexyl-ATP; 8-[(6-Amino)hexyl]-amino-ATP; 5-propargylamino-CTP, 5-propargylamino-UTP; 5-(3-aminoallyl)-dUTP; 8-[(4-amino)butyl]-amino-dATP and 8-[(6-amino)butyl]-amino-dATP; N6-(4-amino)butyl-dATP, N6-(6-amino)butyl-dATP, N4-[2,2-oxy-bis-(ethylamine)]-dCTP; N6-(6-Amino)hexyl-dATP; 8-[(6-Amino)hexyl]-amino-dATP; 5-propargylamino-dCTP, and 5-propargylamino-dUTP. Such nucleotides can be prepared according to methods known to those of skill in the art. Moreover, a person of ordinary skill in the art could prepare other nucleotide entities with the same amine-modification, such as a 5-(3-aminoallyl)-CTP, GTP, ATP, dCTP, dGTP, dTTP, or dUTP in place of a 5-(3-aminoallyl)-UTP.

### B. Preparation of Nucleic Acids

A nucleic acid may be made by any technique known to one of ordinary skill in the art, such as for example, chemical synthesis, enzymatic production, or biological production. It is specifically contemplated that miRNA probes of the invention are chemically synthesized.

In some embodiments of the invention, miRNAs are recovered or isolated from a biological sample. The miRNA may be recombinant or it may be natural or endogenous to the cell (produced from the cell's genome). It is contemplated that a biological sample may be treated in a way so as to enhance the recovery of small RNA molecules such as miRNA. U.S. Patent Application Serial No. 10/667,126 describes such methods . Generally, methods involve lysing cells with a solution having guanidinium and a detergent.

Alternatively, nucleic acid synthesis is performed according to standard methods. See, for example, Itakura and Riggs (1980) and U.S. Patents 4,704,362, 5,221,619, and 5,583,013. Non-limiting examples of a synthetic nucleic acid (*e.g.,* a synthetic oligonucleotide), include a nucleic acid made by *in vitro* chemically synthesis using phosphotriester, phosphite, or phosphoramidite chemistry and solid phase techniques such as described in EP 266,032 , or *via* deoxynucleoside H-phosphonate intermediates as described by Froehler *et al.,* 1986 and U.S. Patent 5,705,629. Various different mechanisms of oligonucleotide synthesis have been disclosed in for example, U.S. Patents 4,659,774, 4,816,571, 5,141,813, 5,264,566, 4,959,463, 5,428,148, 5,554,744, 5,574,146, 5,602,244.

A non-limiting example of an enzymatically produced nucleic acid include one produced by enzymes in amplification reactions such as PCR^{™} (see for example, U.S. Patents 4,683,202 and 4,682, 195, or the synthesis of an oligonucleotide described in U.S. Patent 5,645,897. See also Sambrook *et al.,* 2001).

Oligonucleotide synthesis is well known to those of skill in the art. Various different mechanisms of oligonucleotide synthesis have been disclosed in for example, U.S. Patents 4,659,774, 4,816,571, 5,141,813, 5,264,566, 4,959,463, 5,428,148, 5,554,744, 5,574,146, 5,602,244.

Recombinant methods for producing nucleic acids in a cell are well known to those of skill in the art. These include the use of vectors (viral and non-viral), plasmids, cosmids, and other vehicles for delivering a nucleic acid to a cell, which may be the target cell (*e.g.*, a cancer cell) or simply a host cell (to produce large quantities of the desired RNA molecule). Alternatively, such vehicles can be used in the context of a cell free system so long as the reagents for generating the RNA molecule are present. Such methods include those described in Sambrook, 2003, Sambrook, 2001 and Sambrook, 1989 .

### C. Isolation of Nucleic Acids

Nucleic acids may be isolated using techniques well known to those of skill in the art, though in particular embodiments, methods for isolating small nucleic acid molecules, and/or isolating RNA molecules can be employed. Chromatography is a process often used to separate or isolate nucleic acids from protein or from other nucleic acids. Such methods can involve electrophoresis with a gel matrix, filter columns, alcohol precipitation, and/or other chromatography. If miRNA from cells is to be used or evaluated, methods generally involve lysing the cells with a chaotropic (e.g., guanidinium isothiocyanate) and/or detergent (*e.g*., N-lauroyl sarcosine) prior to implementing processes for isolating particular populations of RNA.

In particular methods for separating miRNA from other nucleic acids, a gel matrix is prepared using polyacrylamide, though agarose can also be used. The gels may be graded by concentration or they may be uniform. Plates or tubing can be used to hold the gel matrix for electrophoresis. Usually one-dimensional electrophoresis is employed for the separation of nucleic-acids. Plates are used to prepare a slab gel, while the tubing (glass or rubber, typically) can be used to prepare a tube gel. The phrase "tube electrophoresis" refers to the use of a tube or tubing, instead of plates, to form the gel. Materials for implementing tube electrophoresis can be readily prepared by a person of skill in the art or purchased, such as from C.B.S. Scientific Co., Inc. or Scie-Plas.

Methods may involve the use of organic solvents and/or alcohol to isolate nucleic acids, particularly miRNA used in methods and compositions of the invention. Some embodiments are described in U.S. Patent Application Serial No. 10/667,126 . Generally, this disclosure provides methods for efficiently isolating small RNA molecules from cells comprising: adding an alcohol solution to a cell lysate and applying the alcohol/lysate mixture to a solid support before eluting the RNA molecules from the solid support. In some embodiments, the amount of alcohol added to a cell lysate achieves an alcohol concentration of about 55% to 60%. While different alcohols can be employed, ethanol works well. A solid support may be any structure, and it includes beads, filters, and columns, which may include a mineral or polymer support with electronegative groups. A glass fiber filter or column has worked particularly well for such isolation procedures.

In specific embodiments, miRNA isolation processes include: a) lysing cells in the sample with a lysing solution comprising guanidinium, wherein a lysate with a concentration of at least about 1 M guanidinium is produced; b) extracting miRNA molecules from the lysate with an extraction solution comprising phenol; c) adding to the lysate an alcohol solution for forming a lysate/alcohol mixture, wherein the concentration of alcohol in the mixture is between about 35% to about 70%; d) applying the lysate/alcohol mixture to a solid support; e) eluting the miRNA molecules from the solid support with an ionic solution; and, f) capturing the miRNA molecules. Typically the sample is dried and resuspended in a liquid and volume appropriate for subsequent manipulation.

### V. LABELS AND LABELING TECHNIQUES

In some embodiments, the present invention concerns miRNA that are labeled. It is contemplated that miRNA may first be isolated and/or purified prior to labeling. This may achieve a reaction that more efficiently labels the miRNA, as opposed to other RNA in a sample in which the miRNA is not isolated or purified prior to labeling. In many embodiments of the invention, the label is non-radioactive. Generally, nucleic acids may be labeled by adding labeled nucleotides (one-step process) or adding nucleotides and labeling the added nucleotides (two-step process).

### A. Labeling Techniques

In some embodiments, nucleic acids are labeled by catalytically adding to the nucleic acid an already labeled nucleotide or nucleotides. One or more labeled nucleotides can be added to miRNA molecules. See U.S. Patent 6,723,509 .

In other embodiments, an unlabeled nucleotide or nucleotides is catalytically added to a miRNA, and the unlabeled nucleotide is modified with a chemical moiety that enables it to be subsequently labeled. In embodiments of the invention, the chemical moiety is a reactive amine such that the nucleotide is an amine-modified nucleotide. Examples of amine-modified nucleotides are well known to those of skill in the art, many being commercially available such as from Ambion, Sigma, Jena Bioscience, and TriLink.

In contrast to labeling of cDNA during its synthesis, the issue for labeling miRNA is how to label the already existing molecule. The present invention concerns the use of an enzyme capable of using a di- or tri-phosphate ribonucleotide or deoxyribonucleotide as a substrate for its addition to a miRNA. Moreover, in specific embodiments, it involves using a modified di- or tri-phosphate ribonucleotide, which is added to the 3' end of a miRNA. Enzymes capable of adding such nucleotides include, but are not limited to, poly(A) polymerase, terminal transferase, and polynucleotide phosphorylase. In specific embodiments of the invention, a ligase is contemplated as not being the enzyme used to add the label, and instead, a non-ligase enzyme is employed. Terminal transferase catalyzes the addition of nucleotides to the 3' terminus of a nucleic acid. Polynucleotide phosphorylase can polymerize nucleotide diphosphates without the need for a primer.

### B. Labels

Labels on miRNA or miRNA probes may be colorimetric (includes visible and UV spectrum, including fluorescent), luminescent, enzymatic, or positron emitting (including radioactive). The label may be detected directly or indirectly. Radioactive labels include ¹²⁵I, ³²P, ³³P, and ³⁵S. Examples of enzymatic labels include alkaline phosphatase, luciferase, horseradish peroxidase, and β-galactosidase. Labels can also be proteins with luminescent properties, *e.g*., green fluorescent protein and phycoerythrin.

The colorimetric and fluorescent labels contemplated for use as conjugates include, but are not limited to, Alexa Fluor dyes, BODIPY dyes, such as BODIPY FL; Cascade Blue; Cascade Yellow; coumarin and its derivatives, such as 7-amino-4-methylcoumarin, aminocoumarin and hydroxycoumarin; cyanine dyes, such as Cy3 and Cy5; eosins and erythrosins; fluorescein and its derivatives, such as fluorescein isothiocyanate; macrocyclic chelates of lanthanide ions, such as Quantum Dye™; Marina Blue; Oregon Green; rhodamine dyes, such as rhodamine red, tetramethylrhodamine and rhodamine 6G; Texas Red; , fluorescent energy transfer dyes, such as thiazole orange-ethidium heterodimer; and, TOTAB.

Specific examples of dyes include, but are not limited to, those identified above and the following: Alexa Fluor 350, Alexa Fluor 405, Alexa Fluor 430, Alexa Fluor 488, Alexa Fluor 500. Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, and, Alexa Fluor 750; amine-reactive BODIPY dyes, such as BODIPY 493/503, BODIPY 530/550, BODIPY 558/568, BODIPY 564/570, BODIPY 576/589, BODIPY 581/591, BODIPY 630/650, BODIPY 650/655, BODIPY FL, BODIPY R6G, BODIPY TMR, and, BODIPY-TR; Cy3, Cy5, 6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, SYPRO, TAMRA, 2',4',5',7'-Tetrabromosulfonefluorescein, and TET.

Specific examples of fluorescently labeled ribonucleotides are available from Molecular Probes, and these include, Alexa Fluor 488-5-UTP, Fluorescein-12-UTP, BODIPY FL-14-UTP, BODIPY TMR-14-UTP, Tetramethylrhodamine-6-UTP, Alexa Fluor 546-14-UTP, Texas Red-5-UTP, and BODIPY TR-14-UTP. Other fluorescent ribonucleotides are available from Amersham Biosciences, such as Cy3-UTP and Cy5-UTP.

Examples of fluorescently labeled deoxyribonucleotides include Dinitrophenyl (DNP)-11-dUTP, Cascade Blue-7-dUTP, Alexa Fluor 488-5-dUTP, Fluorescein-12-dUTP, Oregon Green 488-5-dUTP, BODIPY FL-14-dUTP, Rhodamine Green-5-dUTP, Alexa Fluor 532-5-dUTP, BODIPY TMR-14-dUTP, Tetramethylrhodamine-6-dUTP, Alexa Fluor 546-14-dUTP, Alexa Fluor 568-5-dUTP, Texas Red-12-dUTP, Texas Red-5-dUTP, BODIPY TR-14-dUTP, Alexa Fluor 594-5-dUTP, BODIPY 630/650-14-dUTP, BODIPY 650/665-14-dUTP; Alexa Fluor 488-7-OBEA-dCTP, Alexa Fluor 546-16-OBEA-dCTP, Alexa Fluor 594-7-OBEA-dCTP, Alexa Fluor 647-12-OBEA-dCTP.

It is contemplated that nucleic acids may be labeled with two different labels. Furthermore, fluorescence resonance energy transfer (FRET) may be employed in methods of the invention (*e.g.*, Klostermeier *et al.,* 2002; Emptage, 2001; Didenko, 2001.

Alternatively, the label may not be detectable *per se,* but indirectly detectable or allowing for the isolation or separation of the targeted nucleic acid. For example, the label could be biotin, digoxigenin, polyvalent cations, chelator groups and the other ligands, include ligands for an antibody.

### C. Visualization Techniques

A number of techniques for visualizing or detecting labeled nucleic acids are readily available. Such techniques include, microscopy, arrays, Fluorometry, Light cyclers or other real time PCR machines, FACS analysis, scintillation counters, Phosphoimagers, Geiger counters, MRI, CAT, antibody-based detection methods (Westerns, immunofluorescence, immunohistochemistry), histochemical techniques, HPLC (Griffey *et al*., 1997), spectroscopy, capillary gel electrophoresis (Cummins *et al.,* 1996), spectroscopy; mass spectroscopy; radiological techniques; and mass balance techniques.

When two or more differentially colored labels are employed, fluorescent resonance energy transfer (FRET) techniques may be employed to characterize association of one or more nucleic acid. Furthermore, a person of ordinary skill in the art is well aware of ways of visualizing, identifying, and characterizing labeled nucleic acids, and accordingly, such protocols may be used as part of the invention. Examples of tools that may be used also include fluorescent microscopy, a BioAnalyzer, a plate reader, Storm (Molecular Dynamics), Array Scanner, FACS (fluorescent activated cell sorter), or any instrument that has the ability to excite and detect a fluorescent molecule.

### VI. KITS

Any of the compositions described herein may be comprised in a kit. In a non-limiting example, reagents for isolating miRNA, labeling miRNA, and/or evaluating a miRNA population using an array, nucleic acid amplification, and/or hybridization can be included in a kit, as well reagents for preparation of samples from blood samples. The kit may further include reagents for creating or synthesizing miRNA probes. The kits will thus comprise, in suitable container means, an enzyme for labeling the miRNA by incorporating labeled nucleotide or unlabeled nucleotides that are subsequently labeled. In certain aspects, the kit can include amplification reagents. In other aspects, the kit may include various supports, such as glass, nylon, polymeric beads, and the like, and/or reagents for coupling any probes and/or target nucleic acids. It may also include one or more buffers, such as reaction buffer, labeling buffer, washing buffer, or a hybridization buffer, compounds for preparing the miRNA probes, and components for isolating miRNA. Other kits of the invention may include components for making a nucleic acid array comprising miRNA, and thus, may include, for example, a solid support.

Kits for implementing methods of the invention described herein are specifically contemplated. In some embodiments, there are kits for preparing miRNA for multi-labeling and kits for preparing miRNA probes and/or miRNA arrays. In these embodiments, kit comprise, in suitable container means, 1, 2, 3, 4, 5, 6, 7, 8, 9, 10, 11, 12 or more of the following: (1) poly(A) polymerase; (2) unmodified nucleotides (G, A, T, C, and/or U); (3) a modified nucleotide (labeled or unlabeled); (4) poly(A) polymerase buffer; and, (5) at least one microfilter; (6) label that can be attached to a nucleotide; (7) at least one miRNA probe; (8) reaction buffer; (9) a miRNA array or components for making such an array; (10) acetic acid; (11) alcohol; (12) solutions for preparing, isolating, enriching, and purifying miRNAs or miRNA probes or arrays. Other reagents include those generally used for manipulating RNA, such as formamide, loading dye, ribonuclease inhibitors, and DNase.

In specific embodiments, kits of the invention include an array containing miRNA probes, as described in the application. An array may have probes corresponding to all known miRNAs of an organism or a particular tissue or organ in particular conditions, or to a subset of such probes. The subset of probes on arrays of the invention may be or include those identified as relevant to a particular diagnostic, therapeutic, or prognostic application. For example, the array may contain one or more probes that is indicative or suggestive of (1) a disease or condition (acute myeloid leukemia), (2) susceptibility or resistance to a particular drug or treatment; (3) susceptibility to toxicity from a drug or substance; (4) the stage of development or severity of a disease or condition (prognosis); and (5) genetic predisposition to a disease or condition.

For any kit embodiment, including an array, there can be nucleic acid molecules that contain or can be used to amplify a sequence that is a variant of, identical to or complementary to all or part of any of SEQ IDs described herein. In certain embodiments, a kit or array of the invention can contain one or more probes for the miRNAs identified by the SEQ IDs described herein. Any nucleic acid discussed above may be implemented as part of a kit.

The components of the kits may be packaged either in aqueous media or in lyophilized form. The container means of the kits will generally include at least one vial, test tube, flask, bottle, syringe or other container means, into which a component may be placed, and preferably, suitably aliquoted. Where there is more than one component in the kit (labeling reagent and label may be packaged together), the kit also will generally contain a second, third or other additional container into which the additional components may be separately placed. However, various combinations of components may be comprised in a vial. The kits of the present invention also will typically include a means for containing the nucleic acids, and any other reagent containers in close confinement for commercial sale. Such containers may include injection or blow molded plastic containers into which the desired vials are retained.

When the components of the kit are provided in one and/or more liquid solutions, the liquid solution is an aqueous solution, with a sterile aqueous solution being particularly preferred.

However, the components of the kit may be provided as dried powder(s). When reagents and/or components are provided as a dry powder, the powder can be reconstituted by the addition of a suitable solvent. It is envisioned that the solvent may also be provided in another container means. In some embodiments, labeling dyes are provided as a dried power. It is contemplated that 10, 20, 30, 40, 50, 60, 70, 80, 90, 100, 120, 120, 130, 140, 150, 160, 170, 180, 190, 200, 300, 400, 500, 600, 700, 800, 900, 1000 µg or at least or at most those amounts of dried dye are provided in kits of the invention. The dye may then be resuspended in any suitable solvent, such as DMSO.

Such kits may also include components that facilitate isolation of the labeled miRNA. It may also include components that preserve or maintain the miRNA or that protect against its degradation. Such components may be RNAse-free or protect against RNAses. Such kits generally will comprise, in suitable means, distinct containers for each individual reagent or solution.

A kit will also include instructions for employing the kit components as well the use of any other reagent not included in the kit. Instructions may include variations that can be implemented.

Kits of the invention may also include one or more of the following: Control RNA; nuclease-free water; RNase-free containers, such as 1.5 ml tubes; RNase-free elution tubes; PEG or dextran; ethanol; acetic acid; sodium acetate; ammonium acetate; guanidinium; detergent; nucleic acid size marker; RNase-free tube tips; and RNase or DNase inhibitors.

It is contemplated that such reagents are embodiments of kits of the invention. Such kits, however, are not limited to the particular items identified above and may include any reagent used for the manipulation or characterization of miRNA.

### VII. EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### EXAMPLE 1:

### GENE EXPRESSION ANALYSIS FOLLOWING TRANSFECTION WITH HSA-MIR-126

miRNAs are believed to regulate gene expression by binding to target mRNA transcripts and (1) initiating transcript degradation or (2) altering protein translation from the transcript. Translational regulation leading to an up or down change in protein expression may lead to changes in activity and expression of downstream gene products and genes that are in turn regulated by those proteins. These numerous regulatory effects may be revealed as changes in the global mRNA expression profile. Microarray gene expression analyses were performed to identify genes that are mis-regulated by hsa-miR-126 expression.

Synthetic Pre-miR-126 (Ambion) or two negative control miRNAs (pre-miR-NC1, Ambion cat. no. AM17110 and pre-miR-NC2, Ambion, cat. no. AM17111) were reverse transfected into quadruplicate samples of A549 cells for each of three time points. Cells were transfected using siPORT NeoFX (Ambion) according to the manufacturer's recommendations using the following parameters: 200,000 cells per well in a 6 well plate, 5.0 µl of NeoFX, 30 nM final concentration of miRNA in 2.5 ml. Cells were harvested at 4 h, 24 h, and 72 h post transfection. Total RNA was extracted using RNAqueous-4PCR (Ambion) according to the manufacturer's recommended protocol.

mRNA array analyses were performed by Asuragen Services (Austin, TX), according to the company's standard operating procedures. Using the MessageAmp™ II-96 aRNA Amplification Kit (Ambion, cat #1819) 2 µg of total RNA were used for target preparation and labeling with biotin. cRNA yields were quantified using an Agilent Bioanalyzer 2100 capillary electrophoresis protocol. Labeled target was hybridized to Affymetrix mRNA arrays (Human HG-U133A 2.0 arrays) using the manufacturer's recommendations and the following parameters. Hybridizations were carried out at 45°C for 16 hr in an Affymetrix Model 640 hybridization oven. Arrays were washed and stained on an Affymetrix FS450 Fluidics station, running the wash script Midi_euk2v3_450. The arrays were scanned on a Affymetrix GeneChip Scanner 3000. Summaries of the image signal data, group mean values, p-values with significance flags, log ratios and gene annotations for every gene on the array were generated using the Affymetrix Statistical Algorithm MAS 5.0 (GCOS v1.3). Data were reported in a file (cabinet) containing the Affymetrix data and result files and in files (.cel) containing the primary image and processed cell intensities of the arrays. Data were normalized for the effect observed by the average of two negative control microRNA sequences and then were averaged together for presentation. A list of genes whose expression levels varied by at least 0.7 log₂ from the average negative control was assembled. Results of the microarray gene expression analysis are shown in Table 1.

Manipulation of the expression levels of the genes listed in Table 1 represents a potentially useful therapy for cancer and other diseases in which increased or reduced expression of hsa-miR-126 has a role in the disease.

### EXAMPLE 2:

### CELLULAR PATHWAYS AFFECTED BY HSA-MIR-126

The mis-regulation of gene expression by hsa-miR-126 (Table 1) affects many cellular pathways that represent potential therapeutic targets for the control of cancer and other diseases and disorders. The inventors determined the identity and nature of the cellular genetic pathways affected by the regulatory cascade induced by hsa-miR-126 expression. Cellular pathway analyses were performed using Ingenuity Pathways Analysis (Version 4.0, Ingenuity^{®} Systems, Redwood City, CA). Alteration of a given pathway was determined by Fisher's Exact test (Fisher, 1922). The most significantly affected pathways following overexpression of hsa-miR-126 in A549 cells are shown in Table 2.

These data demonstrate that hsa-miR-126 directly or indirectly affects the expression of numerous cancer-, cellular proliferation-, cellular development-, cell signaling-, and cell cycle-related genes and thus primarily affects functional pathways related to cancer, cellular growth, development, and proliferation. Those cellular processes all have integral roles in the development and progression of various cancers. Manipulation of the expression levels of genes in the cellular pathways shown in Table 2 represents a potentially useful therapy for cancer and other diseases in which increased or reduced expression of hsa-miR-126 has a role in the disease.

### EXAMPLE 3:

### PREDICTED GENE TARGETS OF HSA-MIR-126

Gene targets for binding of and regulation by hsa-miR-126 were predicted using the proprietary algorithm miRNATarget^{™} (Asuragen), which is an implementation of the method proposed by Krek *et al.* (2005). Predicted targets are shown in Table 3.

The predicted gene targets that exhibited altered mRNA expression levels in human cancer cells, following transfection with pre-miR hsa-miR-126, are shown in Table 4 below.

The predicted gene targets of hsa-miR-126 whose mRNA expression levels are affected by hsa-miR-126 represent particularly useful candidates for cancer therapy and therapy of other diseases through manipulation of their expression levels.

### EXAMPLE 4:

### CANCER RELATED GENE EXPRESSION ALTERED BY HSA-MIR-126

Cell proliferation and survival pathways are commonly altered in tumors (Hanahan and Weinberg, 2000). The inventors have shown that hsa-miR-126 directly or indirectly regulates the transcripts of proteins that are critical in the regulation of these pathways. Many of these targets have inherent oncogenic or tumor suppressor activity. Hsa-miR-126 targets that have prognostic and/or therapeutic value for the treatment of various malignancies are shown in Table 5.

Hsa-miR-126 targets of particular interest are genes and their products that function in the regulation of intracellular signal transduction in response to mitotic or apoptotic stimuli. When deregulated, many of these proteins contribute to the malignant phenotype in vitro and in vivo. Hsa-miR-126 affects intracellular signaling at various layers and controls the expression of secretory proteins, transmembrane growth factor receptors as well as cytoplasmic signaling molecules. Among secretory proteins are epiregulin (EREG) and tumor necrosis factor (TNF) ligand superfamily member 10 (TNFSF10; also known as TRAIL; TNF-related apoptosis-inducing ligand). Membrane-associated receptors are epidermal growth factor receptor (EGFR), fibroblast growth factor receptor 1 (FGFR1), Met, retinoic acid receptor responder 1 (RARRES1) and transforming growth factor beta (TGF-β) receptor 2 and 3 (TGFBR2, TGFBR3). Each of these proteins shows an evident role in carcinogenesis.

EGFR is the mammalian homolog of the v-Erb-B oncoprotein that was originally isolated from avian erythroblastosis virus (Roussel *et al.*, 1979). EGFR functions as a receptor tyrosine kinase that belongs to a family of EGFR receptor proteins (Hynes and Lane, 2005). As a homodimer or heterodimer, EGFR transmits mitotic signals and activates the mitogen-activated protein kinase (MAPK) and the phosphoinositide 3-kinase (PI3K) pathways. Increased EGFR activity is common in various cancer types and can be achieved by three major mechanisms: (i) EGFR is amplified and overexpressed in glioma and esophageal squamous cell carcinoma; (ii) an EGFR variant that lacks the extracellular domain is expressed in carcinomas of the breast, lung and ovary; (iii) somatic activating mutations of EGFR are frequently found in non-small cell lung cancer (NSCLC) (Hynes and Lane, 2005; Sunpaweravong *et al.,* 2005). These cancer-specific EGFR mutations provided the basis for the development of Iressa (gefitinib) and Tarceva (erlotinib), two FDA-approved drugs in the treatment of NSCLC (Siegel-Lakhai *et al.,* 2005). Epiregulin (EREG) belongs to the epidermal growth factor (EGF) family and binds to EGF receptors such as EGFR (Shelly *et al.*, 1998). Epiregulin expression is rare in adult tissues but is elevated in various cancer types (Toyoda *et al.,* 1997). Epiregulin may also play a direct role in tumorigenesis, as it contributes to tumor formation of colon cancer cells (Baba *et al.,* 2000). Since transfection of hsa-miR-126 decreases levels of EGFR and EREG transcripts, hsa-miR-126 might counteract an activation of EGFR signaling in cancer. Other receptor tyrosine kinases negatively regulated by hsa-miR-126 are Met and FGFR-1, the latter of which is commonly overexpressed in multiple cancer types and appears to have angiogenic activity (Chandler *et al.*, 1999). Met acts as the receptor for hepatocyte growth factor (HGF) and was isolated as an oncogene from a chemically transformed human cell line (Cooper *et al.,* 1984; Dean *et al.,* 1985). Met activating mutations are found in sporadic papillary renal cancer, childhood hepatocellular carcinoma and gastric cancer (Danilkovitch-Miagkova and Zbar, 2002). These somatic mutations are associated with increased aggressiveness and extensive metastases in various carcinomas. In several other cancer types, autocrine and paracrine mechanisms lead to an activation of Met signaling. The most frequent mechanism of Met activation, however, is overexpression which occurs in colorectal cancer, hepatocellular carcinoma, gastrinomas as well as carcinomas of the stomach, pancreas, prostate, ovary and breast (Boccaccio and Comoglio, 2006). Met is overexpression correlates with a metastatic tumor phenotype and poor prognosis (Birchmeier *et al.*, 2003). Fas, also known as CD95 or APO-1, is a transmembrane cell surface receptor that functions in the transduction of apoptotic signals in response to its ligand FasL (Houston and O'Connell, 2004). Reduced Fas expression is a common mechanism of cells to decrease the sensitivity to FasL-mediated cell death. Similarly, many different cancer types show lost or decreased Fas expression levels (Table 5). In colorectal carcinoma, Fas expression is progressively reduced in the transformation of normal epithelium to benign neoplasm, adenocarcinomas and metastases (Moller *et al.,* 1994). Thus, despite expression of FasL, tumor cells may escape the FasL induced apoptotic signal. Transient transfection of hsa-miR-126 results in an increase of Fas transcripts and therefore may restore sensitivity to FasL in cancer cells. In accord, hsa-miR-126 regulates RARRES1, TGFBR-2 and TGFBR-3 which are putative tumor suppressors. RARRES1 is a transmembrane protein that is lost or shows decreased expression levels in several types of cancer (Wu *et al.,* 2006a and references therein). TGFBR-2 forms a functional complex with TGFBR-1 and is the primary receptor for TGF-β (Massague *et al.*, 2000). Central role of TGF-β is inhibition of cellular growth of numerous cell types, such as epithelial, endothelial, hematopoietic neural and mesenchymal cells. Many mammary and colorectal carcinomas with microsatellite instability harbor inactivating mutations of TGFBR-2, and therefore escape the growth-inhibitory function of TGF-β (Markowitz *et al.*, 1995; Lucke *et al.*, 2001). TGFBR-3, also referred to as beta-glycan, binds all three TGF-β isoforms with high affinity. TGFBR-3 associates with TGFBR-2 to signal to downstream effector molecules (Blobe *et al.*, 2001). Similar to TGFBR-2, TGFBR-3 is frequently downregulated in multiple cancer types (Table 5). TRAIL is another example for pro-apoptotic proteins that are directly or indirectly regulated by hsa-miR-126. TRAIL (also known as APO-2L, APO-2 ligand or APO2L) interacts with its corresponding death receptors to stimulate caspase activity and programmed cell death (Fesik, 2005). Due to its function, TRAIL and TRAIL receptor agonists are currently being explored for a therapeutic response in cancer. Recombinant TRAIL induces apoptosis in various cancer cell lines and displayed anti-tumor activity in mouse models representative for colon, glioma, multiple myeloma and carcinomas of lung and prostate (Fesik, 2005 and refs therein). Other secretory proteins regulated by hsa-miR-126 that may have either oncogenic or growth-inhibitory activity - depending on the cellular context - include connective tissue growth factor (CTGF) and neutrophil gelatinase-associated lipocalin (NGAL), also known as lipocalin-2 (LCN2) (Hishikawa *et al.,* 1999; Croci *et al.*, 2004; Fernandez *et al.*, 2005; Lin *et al.*, 2005; Yang *et al.*, 2005; Lee *et al.*, 2006).

Intracellular signaling molecules affected by hsa-miR-126 include integrin linked kinase (ILK), polo-like kinase 1 (PLK1), protein kinase C alpha (PRKCA) and phospholipase C beta-1 (PLCB1). PLCB1 catalyzes the generation of inositol-1,4,5-trisphosphate (IP3) and diacylglycerol (DAG) from phosphatidylinositol-bis-phosphate (PIP2), regulating proliferative signals and checkpoints of the cell cycle (Lo Vasco *et al.,* 2004). ILK is a serine-threonine kinase that associates with cell-membrane bound integrins to regulate actin polymerization and cytoskeletal organization (Hannigan *et al.*, 2005). A co-factor of ILK is paxillin that directly interacts with ILK and facilitates the localization of ILK to focal adhesion plaques, coordinating cell spreading. ILK signals into various pathways and regulates anchorage-independent growth, survival pathways, cell cycle progression, invasion, migration, cell motility and tumor angiogenesis. ILK is overexpressed or constitutively activated in numerous cancer types (Hannigan *et al.,* 2005). Small interfering RNA against paxillin suppresses invasiveness of malignant melanoma cells, suggesting the ILK-paxillin pathway contributes to carcinogenesis in vivo (Hamamura *et al.*, 2005). The data show that transient introduction of hsa-miR-126 into cancer cells leads to reduced expression of ILK and paxillin and may therefore interfere with ILK and paxillin function. PLK1 (also referred to as serine-threonine protein kinase 13; STPK13) is a protein kinase that regulates mitotic spindle function to maintain chromosomal stability (Strebhardt and Ullrich, 2006). PLK1 expression is tightly regulated during the cell cycle and peaks in M phase. PLK1 is inherently oncogenic and directly inhibits the tumor suppressor function of p53 (Ando *et al.,* 2004). Overexpression of PLK1 induces a polynucleated phenotype and cellular transformation of NIH3T3 cells (Mundt *et al.*, 1997; Smith *et al.*, 1997). Likewise, PLK1 shows increased expression levels in most solid tumors, including carcinomas of the breast, colon, lung, stomach and prostate (Table 5). PLK1 overexpression is associated with disease progression and - when depleted - induces apoptosis in cancer cells (Liu and Erikson, 2003; Strebhardt and Ullrich, 2006). Currently, PLK1 is being tested as a target of various small molecule inhibitors for future therapeutic intervention (Strebhardt and Ullrich, 2006). PRKCA belongs to a family of serine-threonine kinases that are activated in response to signaling induced by receptor tyrosine kinases. Functional studies have suggested that PKCs play a role in carcinogenesis and maintenance of the malignant phenotype (Koivunen *et al.*, 2006). PRKCA is overexpressed in endometrial, prostate and high grade urinary bladder carcinomas (Koivunen *et al.*, 2006). PRKCA activity is linked to increased motility and invasion of cancer cells, a phenotype that can be reversed by PRKCA inhibition (Koivunen *et al.*, 2004).

Further growth-related genes regulated by hsa-miR-126 are the cyclins D1 (CCND1) and G1 (CCNG1), Jun, p63 (TP73L) and programmed cell death 4 (PDCD4). Cyclins are co-factors of cyclin-dependent kinases (CDKs) and function in the progression of the cell cycle. Cyclin D1 is required for the transition from G1 into S phase and is overexpressed in numerous cancer types (Donnellan and Chetty, 1998). Hsa-miR-126 negatively regulates cyclin D1 expression and therefore might interfere with abnormal cell growth that depends on high levels of cyclin D1. In contrast, cyclin G1 has growth inhibitory activity and is upregulated by hsa-miR-126 (Zhao *et al.*, 2003). Jun belongs to the basic region/leucine zipper (bZIP) class of transcription factors and is the cellular homolog of the avian oncoprotein v-Jun that induces tumor formation in birds (Maki *et al.*, 1987). p63 is a family member of the p53 tumor suppressor proteins that regulate cell cycle and apoptosis in response to DNA damage (Moll and Slade, 2004). The corresponding TP63 gene is located on chromosome 3q27-28 within a region that is frequently amplified in cancer. The majority of these tumors express a dominant negative from of p63 that acts like an oncogene in nude mice (Hibi *et al.,* 2000). PDCD4 is a tumor suppressor that is induced in response to apoptosis in normal cells. The growth inhibitory properties of PDCD4 are due to PDCD4-mediated inhibition of the c-Jun proto-oncoprotein, inhibition of cap-dependent mRNA translation and activation of the p21 Wafl/Cip1 CDK inhibitor (Yang *et al.*, 2003; Bitomsky *et al.,* 2004; Goke *et al.,* 2004). PDCD4 frequently shows reduced or lost expression in various human malignancies, such as gliomas, hepatocellular carcinomas, lung and renal cell carcinomas (Jansen *et al.,* 2004; Zhang *et al.,* 2006; Gao *et al.,* 2007). Expression of PDCD4 interferes with skin carcinogenesis in a mouse model and suppresses growth of human colon carcinoma cells (Jansen *et al.*, 2005; Yang *et al.*, 2006). Loss of PDCD4 also correlates with lung tumor progression (Chen *et al.,* 2003).

Based on the function of these targets and how they are regulated by hsa-miR-126, hsa-miR-126 appears to have anti-oncogenic activity. As outlined above, hsa-miR-126 negatively regulates bona *fide* oncogenes, such as EGFR, Jun, Met, PLK1, and stimulates the expression of known or candidate tumor suppressors, including FAS, TGFBR2/3, TRAIL and RARRES. This view is supported by our observation that hsa-miR-126 inhibits cellular proliferation of multiple cancer cell lines. However, hsa-miR-126 also regulates cancer-associated genes in a fashion, indicating that a hsa-miR-126 antagonist might be able to intercept with tumor development when appropriate. Among these targets are FGFR4, ERBB3 as well as the potential tumor suppressors ephrin B2 receptor (EphB2) and Ras association domain family protein 2 (RASSF2). Inactivation of EphB2 accelerates tumorigenesis in colorectal carcinoma (Guo *et al.*, 2006). RASSF2 interacts with K-Ras and promotes cell cycle arrest and apoptosis. Accordingly, RASSF2 is frequently downregulated in lung tumor cell lines (Vos *et al.*, 2003). ERBB3 (also known as HER-3) belongs to the protein family of EGFRs and is a homolog of the avian v-Erb oncoprotein (Hynes and Lane, 2005). In contrast to EGFR, ERBB3 transmits mitotic signals only when complexed as a heterodimer with other EGFR members such as ERBB2. Various cancer types show increased levels of ERBB3 and consequently an activation of the EGFR pathway (Table 5). Hsa-miR-126 also regulates the tumor suppressors neurofibromin 1 and 2 (NF1/NF2) which - when lost or mutated - are the cause of neurofibromatosis, one of the most commonly inherited tumor-predisposition syndromes (Rubin and Gutmann, 2005). NF1 functions as a GTPase activating protein (GAP) towards the inherently oncogenic RAS protein, inactivating RAS by catalyzing the RAS-associated GTP into GDP. Next to neurofibromatosis, loss of NF1 function occurs also in other malignancies, such as astrocytomas, gliomas and leukemia (Rubin and Gutmann, 2005).

In summary, hsa-miR-126 governs the activity of proteins that are critical regulators of cell proliferation and tumor development. These targets are frequently deregulated in human cancer. Based on this review of the genes and related pathways that are regulated by miR-126, introduction of hsa-miR-126 or inhibitory anti-hsa-miR-126 into a variety of cancer cell types would likely result in a therapeutic response.

### EXAMPLE 5:

### DELIVERY OF SYNTHETIC HSA-MIR-126 INHIBITS PROLIFERATION OF LUNG CANCER CELLS

The inventors have previously demonstrated that hsa-miR-126 is involved in the regulation of numerous cell activities that represent intervention points for cancer therapy and for therapy of other diseases and disorders (U.S. Patent Applications serial number 11/141,707 filed May 31, 2005 and serial number 11/273,640 filed November 14, 2005). For example, overexpression of hsa-miR-126 decreases the proliferation and/or viability of certain normal or cancerous cell lines.

The development of effective therapeutic regimens requires evidence that demonstrates efficacy and utility of the therapeutic in various cancer models and multiple cancer cell lines that represent the same disease. The inventors assessed the therapeutic effect of hsa-miR-126 for lung cancer by using 11 individual lung cancer cell lines. To measure cellular proliferation of lung cancer cells, the following non-small cell lung cancer (NSCLC) cells were used: cells derived from lung adenocarcinoma (A549, H1299, H522, H838, Calu-3, HCC827, HCC2935), cells derived from lung squamous cell carcinoma (H520, H226), cells derived from lung bronchioalveolar carcinoma (H1650), and cells derived from lung large cell carcinoma (H460). Synthetic hsa-miR-126 (Pre-miR™-hsa-miR-126, Ambion cat. no. AM17100) or negative control (NC) miRNA (Pre-miR™ microRNA Precursor Molecule-Negative Control #2; Ambion cat. no. AM17111) was delivered via lipid-based transfection into A549, H1299, H522, H838, Calu-3, HCC827, HCC2935, H520, H1650, and H460 cells, and via electroporation into H226 cells.

Lipid-based reverse transfections were carried out in triplicate according to a published protocol (Ovcharenko *et al.,* 2005) and the following parameters: Cells (5,000-12,000 per 96 well), 0.1-0.2 µl Lipofectamine™ 2000 (cat. no. 11668-019, Invitrogen Corp., Carlsbad, CA, USA) in 20 µl OptiMEM (Invitrogen), 30 nM final concentration of miRNA in 100 µl. Electroporation of H226 cells was carried out using the BioRad Gene Pulser Xcell™ instrument (BioRad Laboratories Inc., Hercules, CA, USA) with the following settings: 5 × 10⁶ cells with 5 µg miRNA in 200 µl OptiMEM (1.6 µM miRNA), square wave pulse at 250 V for 5 ms. Electroporated H226 cells were seeded at 7,000 cells per 96-well in a total volume of 100 µl. All cells except for Calu-3 cells were harvested 72 hours post transfection or electroporation for assessment of cellular proliferation. Calu-3 cells were harvested 10 days post transfection. Proliferation assays were performed using Alamar Blue (Invitrogen) following the manufacturer's instructions. As a control for inhibition of cellular proliferation, siRNA against the motor protein kinesin 11, also known as Eg5, was used. Eg5 is essential for cellular survival of most eukaryotic cells and a lack thereof leads to reduced cell proliferation and cell death (Weil *et al.*, 2002). siEg5 was used in lipid-based transfection following the same experimental parameters that apply to miRNA. The inventors also used a DNA topoisomerase II inhibitor, etoposide, at a final concentration of 10 µM and 50 µM as an internal standard for the potency of miRNAs. Etoposide is an FDA-approved DNA topoisomerase II inhibitor in the treatment of lung cancer. IC50 values for various lung cancer cells have been reported to range between <1-25 µM for SCLC and NSCLC cells (Ohsaki *et al.,* 1992; Tsai *et al.,* 1993). Percent (%) proliferation values from the Alamar Blue assay were normalized to values from cells treated with negative control miRNA. Percent proliferation of hsa-miR-126 treated cells relative to cells treated with negative control miRNA (100%) is shown in Table 6 and in FIG. 1.

**Table 6. Percent (%) proliferation of lung cancer cell lines treated with hsa-miR-126, Eg5-specific siRNA (siEg5), etoposide, or negative control miRNA (NC). Values are normalized to values obtained from cells transfected with negative control miRNA (100% proliferation). NC, negative control miRNA; siEg5, Eg5-specific siRNA; SD, standard deviation; n.d., not determined.**

| **Cells** | **hsa-miR-126 (30 nM)** | | **siEg5 (30nM)** | | **etoposide (10 µM)** | | **etoposide (50 µM)** | | **NC (30 nM)** | |
|---|---|---|---|---|---|---|---|---|---|---|
| | % proliferation | % SD | % proliferation | % SD | % proliferation | %SD | % proliferation | % SD | % proliferation | % SD |
| A549 | 84.30 | 6.75 | 37.84 | 1.06 | 49.13 | 2.55 | 42.18 | 3.57 | 100.00 | 19.53 |
| H1299 | 89.37 | 2.49 | 54.32 | 2.83 | 79.65 | 5.02 | 54.38 | 2.73 | 100.00 | 8.89 |
| H460 | 29.09 | 5.08 | 27.97 | 0.33 | 32.13 | 1.14 | 27.82 | 0.58 | 100.00 | 2.52 |
| H520 | 80.99 | 1.63 | 70.40 | 3.49 | 66.80 | 3.93 | 48.53 | 2.54 | 100.00 | 4.15 |
| H522 | 74.62 | 2.04 | 53.45 | 2.35 | 82.13 | 3.14 | 61.08 | 2.65 | 100.00 | 7.48 |
| H838 | 74.05 | 2.01 | 69.14 | 4.15 | 89.71 | 6.17 | 36.97 | 0.62 | 100.00 | 7.74 |
| H1650 | 80.67 | 2.77 | 87.96 | 1.73 | 90.98 | 8.44 | 60.31 | 4.59 | 100.00 | 7.21 |
| HCC827 | 84.31 | 11.96 | 91.68 | 8.89 | 98.95 | 3.00 | 82.53 | 7.75 | 100.00 | 4.32 |
| Calu-3 | 65.05 | 1.19 | 34.59 | 1.33 | 20.81 | 0.19 | 13.53 | 0.64 | 100.00 | 5.54 |
| H226 | 92.09 | 1.31 | n.d. | n.d. | 28.17 | 2.32 | 9.33 | 2.70 | 100.00 | 2.43 |
| HCC2935 | 66.89 | 5.84 | 63.61 | 6.12 | n.d. | n.d. | n.d. | n.d. | 100.00 | 13.92 |

Delivery of hsa-miR-126 inhibits cellular proliferation of lung cancer cells A549, H1299, H522, H838, Calu-3, HCC827, HCC2935, H520, H1650, H460, and H226 (Table 6 and FIG. 1). On average, hsa-miR-126 inhibits cellular proliferation by 25.33% (Table 6 and FIG. 1). Hsa-miR-126 has maximal inhibitory activity in H460 cells, reducing proliferation by 71%. The growth-inhibitory activity of hsa-miR-126 is comparable to that of etoposide at concentrations ≥10 µM. Since hsa-miR-126 induces a therapeutic response in all lung cancer cells tested, hsa-miR-126 may provide therapeutic benefit to patients with lung cancer and other malignancies.

The inventors determined sensitivity and specificity of hsa-miR-126 by administering hsa-miR-126 or negative control miRNA at increasing concentrations, ranging from 0 pM to 3,000 pM (FIG. 2). Delivery of miRNA and assessment of cellular proliferation of A549 and H460 cells were done as described above. Proliferation values from the Alamar Blue assay were normalized to values obtained from mock-transfected cells (0 pM = 100% proliferation). Increasing amounts of negative control mRNA (NC) had no effect on cellular proliferation of A549 or H460 cells (Table 7 and FIG. 2). In contrast, the growth-inhibitory phenotype of hsa-miR-126 is dose-dependent and correlates with increasing amounts of hsa-miR-126 (Table 7 and FIG. 2). Hsa-miR-126 induces a specific therapeutic response at concentrations as low as 3,000 pM.

**Table 7. Dose-dependent inhibition of cellular proliferation of lung cancer cell lines by hsa-miR-126. Values are normalized to values obtained from mock-transfected cells (0 pM miRNA). NC, negative control miRNA; % SD, standard deviation.**

| **Concentration [pM]** | **A549 Cells** | | | | **H460 Cells** | | | |
|---|---|---|---|---|---|---|---|---|
| | **hsa-miR-126** | | **NC** | | **hsa-miR-126** | | **NC** | |
| | **% proliferation** | **% SD** | **% proliferation** | **% SD** | **% proliferation** | **% SD** | **% proliferation** | **% SD** |
| 0 | 100.00 | 2.61 | 100.00 | 2.61 | 100.00 | 8.84 | 100.00 | 8.84 |
| 3 | 95.40 | 2.75 | 102.82 | 2.23 | 105.60 | 3.37 | 107.60 | 0.79 |
| 30 | 92.87 | 1.83 | 99.36 | 3.51 | 108.98 | 3.02 | 108.04 | 1.46 |
| 300 | 80.93 | 2.93 | 105.53 | 3.72 | 86.46 | 1.89 | 106.99 | 4.74 |
| 3,000 | 74.50 | 1.76 | 101.30 | 6.35 | 33.91 | 3.51 | 91.41 | 2.14 |

### EXAMPLE 6:

### HSA-MIR-126, IN COMBINATION WITH SPECIFIC HUMAN MICRO-RNAS, SYNERGISTICALLY INHIBITS PROLIFERATION OF LUNG CANCER CELL LINES

miRNAs function in multiple pathways controlling multiple cellular processes. Cancer cells frequently show aberrations in several different pathways, which determine their oncogenic properties. Therefore, administration of multiple miRNAs to cancer patients may result in a superior therapeutic benefit over administration of a single miRNA. The inventors assessed the efficacy of pair-wise miRNA combinations, administering hsa-miR-126 concurrently with either hsa-miR-34a, hsa-miR-124a, hsa-miR-147, hsa-let-7b, hsa-let-7c or hsa-let-7g (Pre-miR™ miRNA, Ambion cat. no. AM 17100). H460 lung cancer cells were transiently reverse-transfected in triplicates with each miRNA at a final concentration of 300 pM, resulting in 600 pM of oligonucleotide. For negative controls, 600 pM of Pre-miR™ microRNA Precursor Molecule-Negative Control #2 (Ambion cat. no. AM17111) were used. To correlate the effect of various combinations with the effect of the sole miRNA, each miRNA at 300 pM was also combined with 300 pM negative control miRNA. Reverse transfection was carried using the following parameters: 7,000 cells per 96 well, 0.15 µl Lipofectamine™ 2000 (Invitrogen) in 20 µl OptiMEM (Invitrogen), 100 µl total transfection volume. As an internal control for the potency of miRNA, etoposide was added at 10 µM and 50 µM to mock-transfected cells, 24 hours after transfection for the following 48 hours. Cells were harvested 72 hours after transfection and subjected to Alamar Blue assays (Invitrogen). Percent proliferation values from the Alamar Blue assay were normalized to those obtained from cells treated with 600 pM negative control miRNA. Data are expressed as % proliferation relative to negative control miRNA-treated cells (Table 8).

Transfection of 300 pM hsa-miR-126 reduces proliferation of H460 cells by 10.54% (Table 8 and FIG. 3). Additive activity of pair-wise combinations (e.g. hsa-miR-126 plus hsa-let-7b) is defined as an activity that is greater than the sole activity of each miRNA (e.g., the activity of hsa-miR-126 plus hsa-let-7b is greater than that observed for hsa-miR-126 plus NC and the activity of hsa-miR-126 plus hsa-let-7b is greater than that observed for hsa-let-7b plus NC). Synergistic activity of pair-wise combinations is defined as an activity that is greater than the sum of the sole activity of each miRNA (e.g., the activity of hsa-miR-126 plus hsa-let-7g is greater than that observed for the sum of the activity of hsa-miR-126 plus NC and the activity of hsa-let-7g plus NC). The data indicate that hsa-miR-126 combined with hsa-miR-34a, hsa-miR-124a, hsa-miR-147, hsa-let-7b, hsa-let-7c, or hsa-let-7g results in additive or synergistic activity (Table 8 and FIG. 3). Therefore, administering combinations of hsa-miR-126 with other miRNAs to cancer patients may induce a superior therapeutic response in the treatment of lung cancer. The combinatorial use of miRNAs represents a potentially useful therapy for cancer and other diseases.

**Table 8. Cellular proliferation of H460 lung cancer cells in the presence of pair-wise miR-126 miRNA combinations. Values are normalized to values obtained from cells transfected with 600 pM negative control (NC) miRNA. SD, standard deviation; S, synergistic effect; A, additive effect.**

| **miRNA [300 pM] + miRNA [300 pM]** | **% Proliferation** | **% SD** | **Effect** |
|---|---|---|---|
| NC + NC | 100.00 | 1.45 | |
| NC + miR-34a | 99.58 | 1.66 | |
| NC + miR-124a | 69.43 | 1.38 | |
| NC + miR-126 | 89.46 | 2.27 | |
| NC + miR-147 | 76.97 | 1.46 | |
| NC + let-7b | 74.92 | 3.38 | |
| NC + let-7c | 86.74 | 2.28 | |
| NC + let-7g | 91.41 | 3.26 | |
| miR-126 + miR-34a | 73.06 | 5.16 | S |
| miR-126 + miR-124a | 46.49 | 4.89 | S |
| miR-126 + miR-147 | 62.64 | 3.79 | S |
| miR-126 + let-7b | 68.76 | 5.89 | A |
| miR-126 + let-7c | 57.03 | 5.15 | S |
| miR-126 + let-7g | 61.89 | 3.27 | S |
| Etoposide (10 µM) | 20.19 | 1.89 | |
| Etoposide (50 µM) | 14.94 | 0.31 | |

### EXAMPLE 7:

### DELIVERY OF SYNTHETIC HSA-MIR-126 INHIBITS TUMOR GROWTH OF LUNG CANCER CELLS IN MICE

The inventors assessed the growth-inhibitory activity of hsa-miR-126 in human lung cancer xenografts grown in immunodeficient mice. Hsa-miR-126 was delivered into A549 and H460 lung cancer cells via electroporation using the Gene Pulser Xcell^{™} (BioRad) with the following settings: 15 x 10⁶ cells with 5 µg miRNA in 200 µl OptiMEM, square wave pulse at 150 V for 10 ms. Electroporated cells (5 × 10⁶) were mixed with BD Matrigel™, (BD Biosciences; San Jose, CA, USA; cat. no. 356237) in a 1:1 ratio and injected subcutaneously into the flank of NOD/SCID mice (Charles River Laboratories, Inc.; Wilmington, MA, USA). As a negative control, A549 and H460 cells were electroporated with negative control (NC) miRNA (Pre-miR™ microRNA Precursor Molecule-Negative Control #2; Ambion cat. no. AM17111) as described above. To assess the anti-oncogenic activity of hsa-miR-126, six animals were injected with A459 cells and six animals were injected with H460 cells. NC miRNA-treated cells were injected into the opposite flank of the same animal to control for animal-to-animal variability. Once tumors reached a measurable size (A549 at 7 days post injection; H460 at 5 days post injection), the length and width of tumors were determined every day for up to 11 days. Tumor volumes were calculated using the formula, Volume = length X width X width / 2, in which the length is greater than the width. For animals carrying A549 xenografts, tumor volumes derived from NC-treated cells and hsa-miR-126-treated cells were averaged and plotted over time (FIG. 4). The p value, indicating statistical significance, is shown for values obtained on day 18 (p = 0.0125).

Administration of hsa-miR-126 into the A549 lung cancer cells inhibited tumor growth *in vivo* (FIG. 4). Cancer cells that received negative control miRNA developed more rapidly than cells treated with hsa-miR-126. A histological analysis revealed that hsa-miR-126-treated A549 tumors displayed diminished levels of the Ki-67 antigen, suggesting that hsa-miR-126 interferes with cellular proliferation of A549 tumor cells (FIG. 7). On day 18 of the study, all animals showed smaller tumors that derived from hsa-miR-126 treated A549 cells relative to control tumors, indicating the statistical significance of tumor suppression (FIG. 5). Similarly, all H460 tumors that developed from hsa-miR-126 treated cells were smaller in size when compared to their corresponding control tumors that were treated with negative control miRNA (FIG. 6; day 7). In summary, administration of hsa-miR-126 delayed and suppressed the onset of tumor growth of human lung cancer xenografts.

These data indicate that hsa-miR-126 represents a particularly useful candidate in the treatment of lung cancer and potentially other diseases.

### REFERENCES

The following references may provide exemplary procedural or other details supplementary to those set forth herein.
U.S. Patent 4,337,063
U.S. Patent 4,404,289
U.S. Patent 4,405,711
U.S. Patent 4,659,774
U.S. Patent 4,682,195
U.S. Patent 4,683,202
U.S. Patent 4,704,362
U.S. Patent 4,816,571
U.S. Patent 4,870,287
U.S. Patent 4,959,463
U.S. Patent 5,141,813
U.S. Patent 5,143,854
U.S. Patent 5,202,231
U.S. Patent 5,214,136
U.S. Patent 5,221,619
U.S. Patent 5,223,618
U.S. Patent 5,242,974
U.S. Patent 5,264,566
U.S. Patent 5,268,486
U.S. Patent 5,288,644
U.S. Patent 5,324,633
U.S. Patent 5,378,825
U.S. Patent 5,384,261
U.S. Patent 5,399,363
U.S. Patent 5,405,783
U.S. Patent 5,412,087
U.S. Patent 5,424,186
U.S. Patent 5,428,148
U.S. Patent 5,429,807
U.S. Patent 5,432,049
U.S. Patent 5,436,327
U.S. Patent 5,445,934
U.S. Patent 5,446,137
U.S. Patent 5,466,468
U.S. Patent 5,466,786
U.S. Patent 5,468,613
U.S. Patent 5,470,710
U.S. Patent 5,470,967
U.S. Patent 5,472,672
U.S. Patent 5,480,980
U.S. Patent 5,492,806
U.S. Patent 5,503,980
U.S. Patent 5,510,270
U.S. Patent 5,525,464
U.S. Patent 5,527,681
U.S. Patent 5,529,756
U.S. Patent 5,532,128
U.S. Patent 5,543,158
U.S. Patent 5,545,531
U.S. Patent 5,574,146
U.S. Patent 5,580,726
U.S. Patent 5,580,732
U.S. Patent 5,583,013
U.S. Patent 5,593,839
U.S. Patent 5,599,672
U.S. Patent 5,599,695
U.S. Patent 5,602,240
U.S. Patent 5,602,244
U.S. Patent 5,610,287
U.S. Patent 5,610,289
U.S. Patent 5,614,617
U.S. Patent 5,623,070
U.S. Patent 5,624,711
U.S. Patent 5,631,134
U.S. Patent 5,637,683
U.S. Patent 5,639,603
U.S. Patent 5,641,515
U.S. Patent 5,645,897
U.S. Patent 5,652,099
U.S. Patent 5,654,413
U.S. Patent 5,658,734
U.S. Patent 5,661,028
U.S. Patent 5,665,547
U.S. Patent 5,667,972
U.S. Patent 5,670,663
U.S. Patent 5,672,697
U.S. Patent 5,677,195
U.S. Patent 5,681,947
U.S. Patent 5,695,940
U.S. Patent 5,700,637
U.S. Patent 5,700,922
U.S. Patent 5,705,629
U.S. Patent 5,708,153
U.S. Patent 5,708,154
U.S. Patent 5,714,606
U.S. Patent 5,728,525
U.S. Patent 5,728,525
U.S. Patent 5,739,169
U.S. Patent 5,744,305
U.S. Patent 5,744,305
U.S. Patent 5,760,395
U.S. Patent 5,763,167
U.S. Patent 5,770,358
U.S. Patent 5,777,092
U.S. Patent 5,789,162
U.S. Patent 5,792,847
U.S. Patent 5,800,992
U.S. Patent 5,801,005
U.S. Patent 5,807,522
U.S. Patent 5,824,311
U.S. Patent 5,830,645
U.S. Patent 5,830,880
U.S. Patent 5,837,196
U.S. Patent 5,846,225
U.S. Patent 5,846,945
U.S. Patent 5,847,219
U.S. Patent 5,856,174
U.S. Patent 5,858,988
U.S. Patent 5,859,221
U.S. Patent 5,871,928
U.S. Patent 5,872,232
U.S. Patent 5,876,932
U.S. Patent 5,886,165
U.S. Patent 5,919,626
U.S. Patent 5,922,591
U.S. Patent 6,004,755
U.S. Patent 6,040,193
U.S. Patent 6,040,193
U.S. Patent 6,087,102
U.S. Patent 6,251,666
U.S. Patent 6,368,799
U.S. Patent 6,383,749
U.S. Patent 6,617,112
U.S. Patent 6,638,717
U.S. Patent 6,720,138
U.S. Patent 6,723,509
U.S. Patent Serial 09/545,207
U.S. Patent Serial 10/667,126
U.S. Patent Serial 11/141,707
U.S. Patent Serial 11/273,640
U.S. Patent Serial 11/349,727
U.S. Patent Serial 60/575,743
U.S. Patent Serial 60/649,584
U.S. Patent Serial 60/650,807

Akino et al., Gastroenterology, 129(1):156-169, 2005.
Ando et al., J Biol Chem, 279(24):25549-25561, 2004.
Arap et al., Cancer Res., 55(6):1351-1354, 1995.
Austin-Ward and Villaseca, Revista Medica de Chile, 126(7):838-845, 1998.
Baba et al., Cancer Res, 60(24):6886-6889, 2000.
Bagga et al., Cell, 122(4):553-563, 2005.
Bandyopadhyay et al., Oncogene, 21(22):3541-3551, 2002.
Bartsch and Tschesche, FEBS Lett, 357(3):255-259, 1995.
Birchmeier et al., Nat Rev Mol Cell Biol, 4(12):915-925, 2003.
Biswas et al., Cancer Res, 64(14):4687-4692, 2004.
Bitomsky et al., Oncogene, 23(45):7484-7493, 2004.
Blobe et al., J Biol Chem, 276(27):24627-24637, 2001.
Boccaccio and Comoglio, Nat Rev Cancer, 6(8):637-645, 2006.
Bui et al., Br J Cancer, 77(2):319-324, 1998.
Bukowski et al., Clinical Cancer Res., 4(10):2337-2347, 1998.
Butler et al., Cancer Res, 62(14):4089-4094, 2002.
Caldas et al., Cancer Res., 54:3568-3573, 1994.
Calin and Croce, Nat Rev Cancer, 6(11):857-866, 2006.
Carbone et al., Blood, 91 (3):747-755, 1998.
Carrington and Ambros, Science, 301(5631):336-338, 2003.
Chandler et al., Int J Cancer, 81 (3):451-458, 1999.
Chen et al., J Pathol, 200(5):640-646, 2003.
Cheng et al., Nucl. Acid Res. 33(4):1290-1297, 2006.
Cho-Vega et al., Hum Pathol, 35(9):1095-1100, 2004.
Christodoulides et al., Microbiology, 144(Pt 11):3027-3037, 1998.
Cooper et al., Nature, 311(5981):29-33, 1984.
Copland et al., Oncogene, 22(39):8053-8062, 2003.
Croci et al., Cancer Res, 64(5):1730-1736, 2004.
Cummins et al., In: IRT: Nucleosides and nucleosides, La Jolla CA, 72, 1996.
Danilkovitch-Miagkova and Zbar, J Clin Invest, 109(7):863-867, 2002.
Davalos et al., Oncogene, 26(2):308-311, 2007.
Davidson et al., J. Immunother., 21(5):389-398, 1998.
Dean et al., Nature, 318(6044):385-388, 1985.
Denli et al., Trends Biochem. Sci., 28:196, 2003.
Didenko, Biotechnigues, 31(5):1106-1116, 1118, 1120-1121, 2001.
Dillman, Cancer Biother. Radiopharm., 14(1):5-10, 1999.
Donnellan and Chetty, Mol Pathol, 51(1):1-7, 1998.
Eferl et al., Cell, 112(2):181-192, 2003.
Emptage et al., Neuron, 29(1):197-208, 2001.
Endoh et al., Br J Cancer, 93(12):1395-1399, 2005.
EP 266,032
EP 373 203
EP 785 280
EP 799 897
Esquela-Kerscher and Slack, Nat Rev Cancer, 6(4):259-269, 2006.
Ezzat et al., Clin Cancer Res, 11(3):1336-1341,2005.
Fernandez et al., Clin Cancer Res, 11(15):5390-5395, 2005.
Fesik, Nat Rev Cancer, 5(11):876-885, 2005.
Fisher, J Royal Statistical Soc 85(1):87-94, 1922.
Fleischer et al., Int J Oncol, 28(1):25-32, 2006.
Florio et al., Eur J Endocrinol, 152(2):277-284, 2005.
Fodor et al., Biochemistry, 30(33):8102-8108, 1991.
Froehler et al., Nucleic Acids Res., 14(13):5399-5407, 1986.
Furutani et al., Cancer Lett, 122(1-2):209-214, 1998.
Gao et al., Oncol Rep, 17(1):123-128, 2007.
Garzon, et al., Proc. Natl. Acad. Sci. USA, 103(13):5078-5083, 2006.
Goke et al., Am J Physiol Cell Physiol, 287(6):C1541-1546, 2004.
Golay et al., Blood, 87(5):1900-1911, 1996.
Gratas et al., Cancer Res, 58(10):2057-2062, 1998.
Griffey et al., J. Mass Spectrom, 32(3):305-13, 1997.
Guo et al., Carcinogenesis, 27(3):454-464, 2006.
Hamamura et al., Proc Natl Acad Sci U S A, 102(31):11041-11046, 2005.
Hanahan and Weinberg, Cell, 100(1):57-70, 2000.
Hanibuchi et al., Int. J. Cancer, 78(4):480-485, 1998.
Hannigan et al., Nat Rev Cancer, 5(1):51-63, 2005.
Hellstrand et al., Acta Oncologica, 37(4):347-353, 1998.
Hibi et al., Proc Natl Acad Sci USA, 97(10):5462-5467, 2000.
Hishikawa et al., JBiol Chem, 274(52):37461-37466, 1999.
Houston and O'Connell, Curr Opin Pharmacol, 4(4):321-326, 2004.
Huguet et al., Cancer Res, 54(10):2615-2621, 1994.
Hui and Hashimoto, Infection Immun., 66(11):5329-5336, 1998.
Hussussian et al., Nat. Genet., 8(1):15-21, 1994.
Huusko et al., Nat Genet, 36(9):979-983, 2004.
Hynes and Lane, Nat Rev Cancer, 5(5):341-354, 2005.
Itakura and Riggs, Science, 209:1401-1405, 1980.
Jaakkola et al., Int J Cancer, 54(3):378-382, 1993.
Jansen et al., Cancer Res, 65(14):6034-6041, 2005.
Jansen et al., Mol Cancer Ther, 3(2):103-110, 2004.
Jiang et al., Cancer Res, 64(16):5787-5794, 2004.
Ju et al., Gene Ther., 7(19): 1672-1679, 2000.
Jubb et al., Clin Cancer Res, 11(14):5181-5187, 2005.
Kamb et al., Nat. Genet., 8(1):23-26, 1994.
Kamb et al., Science, 2674:436-440, 1994.
Kitada et al., Blood, 91(9):3379-3389, 1998.
Klostermeier and Millar, Biopolymers, 61 (3): 1 59-79, 2001-2002.
Kobayashi et al., Oncogene, 22(9):1294-1301, 2003.
Koivunen et al., Cancer Lett, 235(l1):1-10, 2006.
Koivunen et al., Cancer Res, 64(16):5693-5701, 2004.
Kokko et al., BMC Cancer, 6:145, 2006.
Koliopanos et al., World J Surg, 26(4):420-427, 2002.
Kornberg and Baker, In: DNA Replication, 2d Ed., Freeman, San Francisco, 1992.
Koumakpayi et al., Clin Cancer Res, 12(9):2730-2737, 2006.
Krajewska et al., Am J Pathol, 148(5):1567-1576, 1996.
Krek et al., Nature Genet 37:495-500, 2005.
Lagos-Quintana et al., Science, 294(5543):853-858, 2001.
Lahn and Sundell, Melanoma Res, 14(2):85-89, 2004.
Lambros et al., J Pathol, 205(1):29-40,2005.
Lau et al., Science, 294(5543):858-862, 2001.
Lee and Ambros, Science, 294(5543):862-864, 2001.
Lee et al., Int J Cancer, 118(10):2490-2497, 2006.
Lemoine et al., Br J Cancer, 66(6):1116-1121, 1992.
Leng et al., Chin Med Sci J, 12(2):67-70, 1997.
Lim et al., Nature, 433(7027):769-773, 2005
Lin et al., Gastroenterology, 128(1):9-23, 2005.
Liu and Erikson, Proc Natl Acad Sci USA, 100(10):5789-5794, 2003.
Lo Vasco et al., Leukemia, 18(6):1122-1126, 2004.
Lucke et al., Cancer Res, 61(2):482-485, 2001.
Maki et al., Proc Natl Acad Sci U S A, 84(9):2848-2852, 1987.
Markowitz et al., Science, 268(5215):1336-1338, 1995.
Markowitz, Biochim Biophys Acta, 1470(1):M13-20, 2000.
Marks, Semin Cancer Biol, 16(6):436-443, 2006.
Marsters et al., Recent Prog. Horm. Res., 54:225-234, 1999.
Martinez-Lorenzo et al., Int J Cancer, 75(3):473-481, 1998.
Massague et al., Cell, 103(2):295-309, 2000.
Maurer et al., Hum Pathol, 29(8):771-777, 1998.
McClatchey and Giovannini, Genes Dev, 19(19):2265-2277, 2005.
Moll and Slade, Mol Cancer Res, 2(7):371-386, 2004.
Moller et al., Int J Cancer, 57(3):371-377, 1994.
Mori et al., Cancer Res., 54(13):3396-3397, 1994.
Mundt et al., Biochem Biophys Res Commun, 239(2):377-385, 1997.
Nakada et al., Cancer Res, 64(9):3179-3185; 2004.
Nobori et al., Nature, 368(6473):753-756, 1994.
Ohsaki et al., Cancer Res. Jul 1;52(13):3534-8, 1992.
Okamoto et al., Proc. Natl. Acad. Sci. USA, 91(23):11045-11049,1994.
Olsen et al., Dev. Biol., 216:671, 1999.
Orlow et al., Cancer Res, 54(11):2848-2851, 1994.
Orlow et al., Int. J. Oncol., 15(1):17-24, 1994.
Ovcharenko etal., RNA. Jun;11 (6):985-93, 2005.
Pan et al., Neurol Res, 24(7):677-683, 2002.
PCT Appln. WO 0138580
PCT Appln. WO 0168255
PCT Appln. WO 03020898
PCT Appln. WO 03022421
PCT Appln. WO 03023058
PCT Appln. WO 03029485
PCT Appln. WO 03040410
PCT Appln. WO 03053586
PCT Appln. WO 03066906
PCT Appln. WO 03067217
PCT Appln. WO 03076928
PCT Appln. WO 03087297
PCT Appln. WO 03091426
PCT Appln. WO 03093810
PCT Appln. WO 03100012
PCT Appln. WO 03100448A1
PCT Appln. WO 04020085
PCT Appln. WO 04027093
PCT Appln. WO 09923256
PCT Appln. WO 09936760
PCT Appln. WO 93/17126
PCT Appln. WO 95/11995
PCT Appln. WO 95/21265
PCT Appln. WO 95/21944
PCT Appln. WO 95/21944
PCT Appln. WO 95/35505
PCT Appln. WO 96/31622
PCT Appln. WO 97/10365
PCT Appln. WO 97/27317
PCT Appln. WO 9743450
PCT Appln. WO 99/35505
Pietras et al., Oncogene, 17(17):2235-2249, 1998.
Pruitt et al., Nucleic Acids Res, 33(1):D501-D504, 2005.
Qin et al., Proc. Natl: Acad. Sci. USA, 95(24):14411-14416, 1998.
Rajkumar et al., J Pathol, 179(4):381-385, 1996.
Reimer et al., J Biol Chem, 274(16): 11022-11029, 1999.
Remington's Pharmaceutical Sciences" 15th Edition, pages 1035-1038 and 1570-1580, 1990.
Roussel et al., Nature, 281(5731):452-455, 1979.
Rubin and Gutmann, Nat Rev Cancer, 5(7):557-564, 2005.
Rust et al., J Clin Pathol, 58(5):520-524, 2005.
Salgia et al., Oncogene, 18(1):67-77, 1999.
Sambrook and Russell, Molecular Cloning: A Laboratory Manual 3rd Ed., Cold Spring Harbor Laboratory Press, 2001.
Sambrook et al., In: DNA microaarays: a molecular cloning manual, Cold Spring Harbor Laboratory Press, Cold Spring Harbor, NY, 2003.
Sambrook et al., In: Molecular cloning: a laboratory manual, 2nd Ed., Cold Spring Harbor Laboratory Press, Cold Spring Harbor, NY, 1989.
Sano et al., Histopathology, 46(5):532-539, 2005.
Scheit, In: Synthesis and Biological Function, Wiley-Interscience, New York, 171-172, 1980.
Seggerson et al., Dev. Biol., 243:215, 2002.
Serrano et al., Nature, 366:704-707, 1993.
Serrano et al., Science, 267(5195):249-252, 1995.
Shah et al., Oncogene, 21(54):8251-8261, 2002.
Shelly et al., J Biol Chem, 273(17):10496-10505, 1998.
Shimo et al., Cancer Lett, 174(1):57-64, 2001.
Shinoura et al., Cancer Gene Ther, 7(2):224-232, 2000.
Siegel-Lakhai et al., Oncologist, 10(8):579-589, 2005.
Sieghart et al., J Hepatol, 44(1):151-157, 2006.
Skotzko et al., Cancer Res, 55(23):5493-5498, 1995.
Smith et al., Biochem Biophys Res Commun, 234(2):397-405, 1997.
Soufla et al., Cancer Lett, 221(1):105-118,2005.
Strebhardt and Ullrich, Nat Rev Cancer, 6(4):321-330, 2006.
Sunpaweravong et al., J Cancer Res Clin Oncol, 131(2):111-119, 2005.
Thogersen et al., Cancer Res, 61(16):6227-6233, 2001.
Torring et al., Anticancer Res, 20(1A):91-95, 2000.
Toyoda et al., Biochem J, 326 (Pt 1):69-75, 1997.
Tsai et al., J Natl Cancer Inst. Jun 2;85(11):897-901, 1993.
Turley et al., Cancer Res, 67(3):1090-1098, 2007.
U.K.. Patent 1,529,202
U.K.. Patent 8 803 000
Venkatasubbarao et al., Anticancer Res, 20(1A):43-51, 2000.
Viard-Leveugle et al., J Pathol, 201(2):268-277, 2003.
Vos et al., J Biol Chem, 278(30):28045-28051, 2003.
Weichert et al., Int J Oncol, 23(3):633-639, 2003.
Weil et al., Biotechniques. Dec;33(6):1244-8, 2002.
Weiss and Bohmann, Cell Cycle, 3(2):111-113, 2004.
Woszczyk et al., Med Sci Monit, 10(1):CR33-37, 2004.
Wu et al., Eur J Cancer, 42(4):557-565, 2006a.
Wu et al., Gynecol Oncol, 102(1):15-21, 2006b.
Wu et al., Pathol Oncol Res, 10(1):26-33, 2004a.
Wuilleme-Toumi et al., Leukemia, 19(7):1248-1252, 2005.
Xu et al., Curr. Biol., 13(9):790-795, 2003.
Yang et al., Cancer Cell, 9(6):445-457, 2006a.
Yang et al., Cancer Res, 65(19):8887-8895, 2005.
Yang et al., J Androl, 22(3):471-480, 2001.
Yang et al., Mol Cell Biol, 23(1):26-37,2003.
Yang et al., Mol Cell Biol, 26(4):1297-1306, 2006b.
Yoshida et al., Ann Oncol, 15(2):252-256, 2004.
Zhang et al., Oncogene, 23(12):2241-2249, 2004.
Zhang et al., Oncogene, 25(45):6101-6112, 2006.
Zhao et al., Mol Cancer Res, 1(3):195-206, 2003.
Zhu et al., Biochem Biophys Res Commun, 273(3):1019-1024, 2000.

### SEQUENCE LISTING

<110> BADER, ANDREAS G.
   BYROM, MIKE
   JOHNSON, CHARLES D.
   BROWN, DAVID
<120> miR-126 REGULATED GENES AND PATHWAYS AS TARGETS FOR THERAPEUTIC INTERVENTION
<130> ASUR:029WO
<140> UNKNOWN
   <141> 2007-12-10
<150> 60/939,563
   <151> 2007-05-22
<150> 60/869,295
   <151> 2006-12-08
<160> 25
<170> PatentIn version 3.3
<210> 1
   <211> 21
   <212> RNA
   <213> Artificial
<220>
   <223> Synthetic primer
<400> 1
   ucguaccgug aguaauaaug c 21
<210> 2
   <211> 21
   <212> RNA
   <213> Artificial
<220>
   <223> Synthetic primer
<400> 2
   cauuauuacu uuugguacgc g 21
<210> 3
   <211> 21
   <212> RNA
   <213> Artificial
<220>
   <223> Synthetic primer
<400> 3
   cguaccguga guaauaaugc g 21
<210> 4
   <211> 21
   <212> RNA
   <213> Artificial
<220>
   <223> Synthetic primer
<400> 4
   ucguaccgug aguaauaaug c 21
<210> 5
   <211> 21
   <212> RNA
   <213> Artificial
<220>
   <223> Synthetic primer
<400> 5
   cauuauuacu uuugguacgc g 21
<210> 6
   <211> 21
   <212> RNA
   <213> Artificial
<220>
   <223> Synthetic primer
<400> 6
   ucguaccgug aguaauaaug c 21
<210> 7
   <211> 23
   <212> RNA
   <213> Artificial
<220>
   <223> Synthetic primer
<400> 7
   ucguaccgug aguaauaaug cgc 23
<210> 8
   <211> 21
   <212> RNA
   <213> Artificial
<220>
   <223> Synthetic primer
<400> 8
   cauuauuacu uuugguacgc g 21
<210> 9
   <211> 21
   <212> RNA
   <213> Artificial
<220>
   <223> Synthetic primer
<400> 9
   cauuauuacu uuugguacgc g 21
<210> 10
   <211> 21
   <212> RNA
   <213> Artificial
<220>
   <223> Synthetic primer
<400> 10
   ucguaccgug aguaauaaug c 21
<210> 11
   <211> 21
   <212> RNA
   <213> Artificial
<220>
   <223> Synthetic primer
<400> 11
   cauuauuacu uuugguacgc g 21
<210> 12
   <211> 21
   <212> RNA
   <213> Artificial
<220>
   <223> Synthetic primer
<400> 12
   ucguaccgug aguaauaaug c 21
<210> 13
   <211> 21
   <212> RNA
   <213> Artificial
<220>
   <223> Synthetic primer
<400> 13
   ucguaccgug aguaauaaug c 21
<210> 14
   <211> 21
   <212> RNA
   <213> Artificial
<220>
   <223> Synthetic primer
<400> 14
   ucguaccgug aguaauaaug c 21
<210> 15
   <211> 21
   <212> RNA
   <213> Artificial
<220>
   <223> Synthetic primer
<400> 15
   cauuauuacu uuugguacgc g 21
<210> 16
   <211> 85
   <212> RNA
   <213> Artificial
<220>
   <223> Synthetic primer
<400> 16
   cgcuggcgac gggacauuau uacuuuuggu acgcgcugug acacuucaaa cucguaccgu 60
   gaguaauaau gcgccgucca cggca 85
<210> 17
   <211> 73
   <212> RNA
   <213> Artificial
<220>
   <223> Synthetic primer
<400> 17
   ugacgggaca uuauuacuuu ugguacgcgc ugugacacuu caaacucgua ccgugaguaa 60
   uaaugcgcug uca 73
<210> 18
   <211> 101
   <212> RNA
   <213> Artificial
<220>
   <223> Synthetic primer
<400> 18
   gagccauuuu aacugcuuca caguccauua uuacuuuugg uacgcgcuag gccagacuca 60
   aacucguacc gugaguaaua augcacugug gcaguggguu u 101
<210> 19
   <211> 63
   <212> RNA
   <213> Artificial
<220>
   <223> Synthetic primer
<400> 19
   cggcccauua uuacuuuugg uacgcgcuau gccacucuca acucguaccg ugaguaauaa 60
   ugc 63
<210> 20
   <211> 84
   <212> RNA
   <213> Artificial
<220>
   <223> Synthetic primer
<400> 20
   gcuggugacg gcccauuauu acuuuuggua cgcgcuguga cacuucaaac ucguaccgug 60
   aguaauaaug cgcugugguc agca 84
<210> 21
   <211> 73
   <212> RNA
   <213> Artificial
<220>
   <223> Synthetic primer
<400> 21
   ugacagcaca uuauuacuuu ugguacgcgc ugugacacuu caaacucgua ccgugaguaa 60
   uaaugcgcgg uca 73
<210> 22
   <211> 73
   <212> RNA
   <213> Artificial
<220>
   <223> Synthetic primer
<400> 22
   ugacagcaca uuauuacuuu ugguacgcgc ugugacacuu caaacucgua ccgugaguaa 60
   uaaugcgugg uca 73
<210> 23
   <211> 63
   <212> RNA
   <213> Artificial
<220>
   <223> Synthetic primer
<400> 23
   cggcccauua uuacuuuugg uacgcgcuau gccacucuca acucguaccg ugaguaauaa 60
   ugc 63
<210> 24
   <211> 69
   <212> RNA
   <213> Artificial
<220>
   <223> Synthetic primer
<400> 24
   ggcugugcau uauuacuuuu gguacgcgcu gugucacauc aaacucguac cgugaguaau 60
   aaugcgcag 69
<210> 25
   <211> 17
   <212> RNA
   <213> Artificial
<220>
   <223> Synthetic primer
<400> 25
   yayyrukasu wwurrur 17

## Claims

1. An isolated nucleic acid comprising a miR-126 nucleic acid sequence for use in combination with an isolated nucleic acid comprising a miR-34a, miR-124a, or miR-147 nucleic acid sequence in the treatment of cancer.

2. The nucleic acid for use according to claim 1, **characterized in that** the subject has, is suspected of having, or at risk of developing a cancerous disease or condition, wherein the cancerous condition is lung cancer.

3. The nucleic acid for use according to claims 1 or 2, **characterized in that** the isolated miR-126, miR-34a, miR-124a, or miR147 nucleic acid is a recombinant nucleic acid, preferably RNA or DNA, preferably wherein the recombinant nucleic acid comprises a miR-126, miR-34a, miR-124a, or miR-147 expression cassette, particularly preferred wherein the expression cassette is comprised in a viral vector, or plasmid DNA vector, even more preferred wherein the viral vector is for administration at a dose of 1x10⁵ to 1x10¹⁴ viral particles per dose or the plasmid DNA vector is administered at a dose of 100 mg per patient to 4000 mg per patient.

4. The nucleic acid for use according to claims 1 or 2, **characterized in that** the isolated miR-126, miR-34a, miR-124a, or miR147 nucleic acid is a synthetic nucleic acid, preferably for administration at a dose of 0.01 mg/kg of body weight to 10 mg/kg of body weight.

5. The nucleic acid for use according to claims 1 to 4, **characterized in that** the miR-126 is a hsa-miR-126 or hsa-miR-126*.

6. The nucleic acid for use according to claims 1 to 5, **characterized in that** the nucleic acid is for enteral or parenteral administration, preferably wherein enteral administration is orally or wherein parenteral administration is intravascular, intracranial, intrapleural, intratumoral, intraperitoneal, intramuscular, intralymphatic, intraglandular, subcutaneous, topical, intrabronchial, intratracheal, intranasal, inhaled, or instilled.

7. The nucleic acid for use according to claims 1 to 6, **characterized in that** the nucleic acid is comprised in a pharmaceutical formulation, preferably in a lipid composition or a nanoparticle composition or wherein the pharmaceutical formulation consists of biocompatible and/or biodegradable molecules.

8. The nucleic acid for use according to claim 1 to 7, further comprising administering the miRNAs in a single composition.

9. An isolated nucleic acid comprising a miR-126 nucleic acid sequence for use in combination with an isolated nucleic acid comprising miR-34a, miR-124a, or miR-147 nucleic acid sequence for treating a patient diagnosed with or suspected of having or suspected of developing cancer comprising the steps of:
(a) administering to the patient an amount of an isolated nucleic acid comprising a miR-126 nucleic acid sequence in combination with an isolated nucleic acid comprising miR-34a, miR-124a, or miR-147 nucleic acid sequence in an amount sufficient to treat or sensitize a patient to a second therapy; and
(b) administering a second therapy, wherein the patient is sensitized to the second therapy.

## Patentansprüche

1. Isolierte Nukleinsäure, die eine miR-126 Nukleinsäuresequenz umfasst, zur Verwendung in Kombination mit einer isolierten Nukleinsäure, die eine miR-34a, miR-124a oder miR-147 Nukleinsäuresequenz umfasst, bei der Behandlung von Krebs.

2. Nukleinsäure zur Verwendung nach Anspruch 1, **dadurch gekennzeichnet, dass** das Subjekt an einer Krebserkrankung oder einem Krebsleiden leidet, der Verdacht darauf besteht oder das Risiko besteht, dass das Subjekt diese(s) entwickelt, wobei das Krebsleiden Lungenkrebs ist.

3. Nukleinsäure zur Verwendung nach Anspruch 1 oder 2, **dadurch gekennzeichnet, dass** die isolierte miR-126, miR-34a, miR-124a oder miR147 Nukleinsäure eine rekombinante Nukleinsäure ist, vorzugsweise RNA oder DNA, wobei vorzugsweise die rekombinante Nukleinsäure eine miR-126, miR-34a, miR-124a oder miR-147 Expressionskassette umfasst, wobei bevorzugt die Expressionskassette besonders in einem viralen Vektor oder Plasmid-DNA-Vektor enthalten ist, wobei noch bevorzugter der virale Vektor für die Verabreichung mit einer Dosis von 1x10⁵ bis 1x10¹⁴ viralen Partikeln pro Dosis geeignet ist oder der Plasmid-DNA-Vektor mit einer Dosis von 100 mg pro Patient bis 4000 mg pro Patient verabreicht wird.

4. Nukleinsäure zur Verwendung nach Anspruch 1 oder 2, **dadurch gekennzeichnet, dass** die isolierte miR-126, miR-34a, miR-124a oder miR147 Nukleinsäure eine synthetische Nukleinsäure ist, vorzugsweise für die Verabreichung mit einer Dosis von 0,01 mg/kg Körpergewicht bis 10 mg/kg Körpergewicht.

5. Nukleinsäure zur Verwendung nach Anspruch 1 bis 4, **dadurch gekennzeichnet, dass** die miR-126 eine hsa-miR-126 oder hsa-miR-126* ist.

6. Nukleinsäure zur Verwendung nach Anspruch 1 bis 5, **dadurch gekennzeichnet, dass** die Nukleinsäure zur enteralen oder parenteralen Verabreichung geeignet ist, wobei vorzugsweise die enterale Verabreichung oral ist, oder wobei die parenterale Verabreichung intravaskulär, intrakranial, intrapleural, intratumoral, intraperitoneal, intramuskulär, intralymphatisch, intraglandulär, subkutan, topisch, intrabronchial, intratrache* al, intranasal, inhaliert oder instilliert ist.

7. Nukleinsäure zur Verwendung nach Anspruch 1 bis 6, **dadurch gekennzeichnet, dass** die Nukleinsäure in einer pharmazeutischen Formulierung enthalten ist, vorzugsweise in einer Lipid-Zusammensetzung oder einer Nanopartikel-Zusammensetzung, oder wobei die pharmazeutische Formulierung aus biokompatiblen und/oder biologisch abbaubaren Molekülen besteht.

8. Nukleinsäure zur Verwendung nach Anspruch 1 bis 7, die ferner die Verabreichung der miRNAs in einer einzelnen Zusammensetzung umfasst.

9. Isolierte Nukleinsäure, die eine miR-126 Nukleinsäuresequenz umfasst, zur Verwendung in Kombination mit einer isolierten Nukleinsäure, die eine miR-34a, miR-124a oder miR-147 Nukleinsäuresequenz umfasst, zur Behandlung eines Patienten, bei dem Krebs diagnostiziert wurde, der Verdacht darauf besteht oder der Verdacht der Entwicklung besteht, welche folgende Schritte umfasst:
(a) das Verabreichen einer Menge einer isolierten Nukleinsäure, die eine miR-126 Nukleinsäuresequenz umfasst, in Kombination mit einer isolierten Nukleinsäure, die eine miR-34a, miR-124a oder miR-147 Nukleinsäuresequenz umfasst, an den Patienten in einer Menge, die ausreichend ist, einen Patienten für eine zweite Therapie zu behandeln oder zu sensibilisieren; und
(b) das Verabreichen einer zweiten Therapie, wobei der Patient für die zweite Therapie sensibilisiert ist.

## Revendications

1. Acide nucléique isolé comprenant une séquence d'acide nucléique miR-126 pour une utilisation en association avec un acide nucléique isolé comprenant une séquence d'acide nucléique miR-34a, miR-124a ou miR-147 dans le traitement d'un cancer.

2. Acide nucléique pour une utilisation suivant la revendication 1, **caractérisé en ce que** le sujet a, est supposé avoir, ou présente un risque de développer, une maladie ou affection cancéreuse, ladite affection cancéreuse étant un cancer du poumon.

3. Acide nucléique pour une utilisation suivant la revendication 1 ou 2, **caractérisé en ce que** l'acide nucléique miR-126, miR-34a, miR-124a ou miR147 isolé est un acide nucléique recombinant, de préférence un ARN ou ADN, avantageusement dans lequel l'acide nucléique recombinant comprend une cassette d'expression miR-126, miR-34a, miR-124a ou miR-147, de manière particulièrement appréciée, dans lequel la cassette d'expression est présente dans un vecteur viral ou un vecteur consistant en un ADN de plasmide, de manière encore plus appréciée dans lequel le vecteur viral est destiné à l'administration à une dose de 1x10⁵ à 1x10¹⁴ particules virales par dose ou bien le vecteur consistant en un ADN de plasmide est administré à une dose de 100 mg par patient à 4000 mg par patient.

4. Acide nucléique pour une utilisation suivant la revendication 1 ou 2, **caractérisé en ce que** l'acide nucléique miR-126, miR-34a, miR-124a ou miR147 isolé est un acide nucléique synthétique, de préférence pour l'administration à une dose de 0,01 mg/kg de poids corporel à 10 mg/kg de poids corporel.

5. Acide nucléique pour une utilisation suivant les revendications 1 à 4, **caractérisé en ce que** le miR-126 est un hsa-miR-126 ou hsa-miR-126*.

6. Acide nucléique pour une utilisation suivant les revendications 1 à 5, **caractérisé en ce que** l'acide nucléique est destiné à l'administration entérale ou parentérale, ladite administration entérale étant de préférence orale ou bien ladite administration parentérale étant intravasculaire, intracrânienne, intrapleurale, intratumorale, intrapéritonéale, intramusculaire, intralym-phatique, intraglandulaire, souscutanée, topique, intra-bronchique, intratrachéale, intranasale, par inhalation ou bien par instillation.

7. Acide nucléique pour une utilisation suivant les revendications 1 à 6, **caractérisé en ce que** l'acide nucléique est présent dans une formulation pharmaceutique, de préférence dans une composition lipidique ou une composition en nanoparticules, ou bien dans une formulation pharmaceutique consistant en molécules biocompatibles et/ou biodégradables.

8. Acide nucléique pour une utilisation suivant les revendications 1 à 7, comprenant en outre l'administration des ARNmi dans une composition unique.

9. Acide nucléique isolé comprenant une séquence d'acide nucléique miR-126 pour une utilisation en association avec un acide nucléique isolé comprenant une séquence d'acide nucléique miR-34a, miR-124a ou miR-147 pour le traitement d'un patient chez lequel a été diagnostiqué, ou supposé avoir ou bien supposé développer un cancer, comprenant les étapes consistant à
(a) administrer au patient une quantité d'un acide nucléique isolé comprenant une séquence d'acide nucléique miR-126 en association avec un acide nucléique isolé comprenant une séquence d'acide nucléique miR-34a, miR-124a ou miR-147 en une quantité suffisante pour traiter ou sensibiliser un patient à une thérapie secondaire ; et
(b) administrer une seconde thérapie, dans laquelle le patient est sensibilisé à la seconde thérapie.
